(19) Europäisches Patentamt
European Patent Office
Office européen des brevets

(11) Veröffentlichungsnummer: **0 094 349 B1**

(12) # EUROPÄISCHE PATENTSCHRIFT

(45) Veröffentlichungstag der Patentschrift: **06.04.94**

(51) Int. Cl.5: **C07D 215/26**, C07D 215/28, C07D 401/12, C07D 405/12, C07D 413/12, A01N 43/42

(21) Anmeldenummer: **83810186.3**

(22) Anmeldetag: **02.05.83**

Die Akte enthält technische Angaben, die nach dem Eingang der Anmeldung eingereicht wurden und die nicht in dieser Patentschrift enthalten sind.

(54) **Verwendung von Chinolinderivaten zum Schützen von Kulturpflanzen.**

(30) Priorität: **07.05.82 CH 2841/82**

(43) Veröffentlichungstag der Anmeldung:
**16.11.83 Patentblatt 83/46**

(45) Bekanntmachung des Hinweises auf die Patenterteilung:
**06.04.94 Patentblatt 94/14**

(84) Benannte Vertragsstaaten:
**AT BE CH DE FR IT LI NL SE**

(56) Entgegenhaltungen:
**DE-A- 2 546 845**
**US-A- 3 351 525**

**CHEMICAL ABSTRACTS, Band 79, Nr. 9, 3. September 1973, Columbus, Ohio, USA; M. LEVI et al. "Derivatives of 8-hydroxyquinoline III", Seite 350, Spalte 2, Abstract Nr. 53154r**

(73) Patentinhaber: **CIBA-GEIGY AG**
**Klybeckstrasse 141**
**CH-4002 Basel(CH)**

(72) Erfinder: **Hubele, Adolf, Dr.**
**Obere Egg 9**
**CH-4312 Magden(CH)**

EP 0 094 349 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von Chinolinderivaten zum Schützen von Kulturhirse, Reis, Mais und Getreidearten gegen schädigende Wirkungen von Agrarchemikalien, Mittel, welche diese Chinolinderivate enthalten, neue Chinolinderivate und die Herstellung der Chinolinderivate. Die Chinolinderivate lassen sich auch zur Regulierung des Pflanzenwachstums einsetzen.

Bei Einsatz von Agrarchemikalien, wie Pflanzenschutzmitteln und insbesondere Herbiziden, können in Abhängigkeit von Faktoren wie beispielsweise Dosis der Agrarchemikalie und Applikationsart, Art der Kulturpflanze, Bodenbeschaffenheit und klimatischen Bedingungen, wie beispielsweise Belichtungsdauer, Temperatur und Niederschlagsmengen, die Kulturpflanzen in gewissem Masse geschädigt werden. So ist beispielsweise bekannt, dass Herbizide aus den verchiedensten Stoffklassen, wie Triazine, Harnstoffderivate, Carbamate, Thiolcarbamate, Halogenacetanilide, Halogenphenoxyessigsäuren und anderen Klassen bei der Anwendung in wirksamer Dosis die Kulturpflanzen, welche gegen die nachteilige Wirkung von unerwünschtem Pflanzenwuchs geschützt werden sollen, in gewissem Masse schädigen können. Um diesem Problem zu begegnen, sind schon verschiedene Stoffe vorgeschlagen worden, welche befähigt sind, die schädigende Wirkung eines Herbizids auf die Kulturpflanze spezifisch zu antagonisieren, d.h., die Kulturpflanze zu schützen, ohne zugleich die Herbizidwirkung gegen zu bekämpfende Unkräuter merklich zu beeinflussen. Dabei hat sich jedoch gezeigt, dass die vorgeschlagenen Gegenmittel häufig nur ein enges Einsatzgebiet haben, d.h., ein bestimmtes Gegenmittel eignet sich oftmals nur zur Anwendung bei einzelnen Kulturpflanzenarten und/oder zum Schutz der Kulturpflanzen gegen einzelne herbizide Substanzen oder Stoffklassen.

So beschreibt die GB-A-1,277,557 die Behandlung von Samen bzw. Sprösslingen von Weizen und Sorghum mit gewissen Oxamsäureestern und Amiden zum Schutz vor dem Angriff durch "ALACHLOR" (N-Methoxymethyl-N-chloracetyl-2,6-diäthylanilin). In DE-A-1,952,910 und DE-A-2,245,471, sowie in FR-B-2,021,611 werden Gegenmittel zur Behandlung von Getreide-, Mais- und Reissamen zum Schutz gegen die schädigende Einwirkung von herbizid wirksamen Thiolcarbamaten vorgeschlagen. Gemäss DE-C-1,567,075 und der US-A-3,131,509 werden Hydroxyaminoacetanilide und Hydantoine für den Schutz von Getreidesamen gegenüber Carbamaten verwendet.

Die direkte pre- oder postemergente Behandlung gewisser Nutzpflanzen mit Gegenmitteln als Antagonisten bestimmter Herbizidklassen auf einer Anbaufläche ist in DE-A-2,141,586 und DE-A-2,218,097, sowie in US-A-3,867,444 beschrieben.

Ferner können Maispflanzen gemäss DE-A-2,402,983 wirksam vor Schädigung durch Chloracetanilide geschützt werden, indem man dem Boden als Gegenmittel ein N-disubstituiertes Dichloracetamid zuführt.

Gemäss EP-A-11 047 können auch Alkoximinobenzylcyanide, deren Alkoxygruppe u.a. durch eine acetalisierte Carbonylgruppe substituiert ist, als Mittel zum Schutz von Kulturpflanzen vor der schädigenden Wirkung von Herbiziden verschiedener Stoffklassen verwendet werden.

Substituierte 2-(Chinolyl-oxy)- und 2-(Isochinolyl-oxy)-carbonsäurederivate und ihre Verwendung als Herbizid sind aus DE-A-2,546,845 bekannt. Aehnliche Verbindungen offenbart Chem. Abstr. 79 (1973), 53154r und schreibt ihnen eine gewisse Aktivität gegen gram-positive und gramnegative Bakterien zu. Speziell 2-(Chinolyl-oxy)-essigsäurederivate sind auch aus US-A-3,351,525 bekannt. Sie dienen zur Bekämpfung von Coccidien in Tieren und können u.a. auch deren Wachstum fördern.

Es wurde nun gefunden, dass sich überraschenderweise eine Gruppe von Chinolinderivaten hervorragend dazu eignet, Kulturhirse, Reis, Mais und Getreidearten gegen schädigende Wirkungen von Agrarchemikalien, wie beispielsweise Pflanzenschutzmitteln, insbesondere Herbiziden, zu schützen. Diese Chinolinderivate werden daher im folgenden auch als "Gegenmittel", "Antidot" oder "Safener" bezeichnet. Sie besitzen zusätzlich auch eine pflanzenwuchsregulierende Wirkung und eignen sich besonders zur Wuchsregulierung bei Dicotylen, vor allem zur Ertragssteigerung bei Kulturpflanzen, insbesondere bei Soja. Ferner können Wurzelwachstum und Keimung gefördert werden.

Gegenstand vorliegender Erfindung ist somit die Verwendung von Chinolinderivaten der Formel I

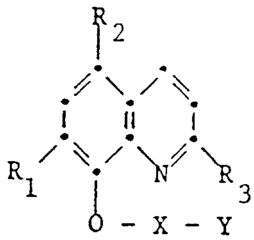

(I),

worin

$R_1$ Wasserstoff, Chlor, Jod oder Brom, $R_2$ Wasserstoff, Chlor, Jod, Brom, Nitro, Methyl oder Aethyl, und $R_3$ Wasserstoff oder Methyl bedeuten, X für eine gegebenenfalls durch eine oder zwei Methylgruppen substituierte Methylenbrücke und Y für einen Oxazolin-2-yl-Rest, $-COOR_4$, $-COSR_5$ oder $-CONR_6R_7$ steht, worin

$R_4$
- Wasserstoff, ein Alkalimetallkation oder ein quaternäres Ammoniumkation ausgewählt aus der Gruppe Ammoniumkation, Trimethyl-, Triäthyl- und Tri-(2-hydrozyäthylen)-ammoniumkation;
- $C_1$-$C_{18}$-Alkyl;
- $C_1$-$C_{10}$-Alkyl, welches durch 1 oder 2 Hydroxygruppen substituiert ist;
- $C_1$-$C_4$-Alkyl, welches durch 1 bis 3 Chlor- oder Bromatome, eine Nitrogruppe, eine Cyanogruppe, eine $C_1$-$C_4$-Alkoxygruppe, eine $C_2$-$C_8$-Alkoxygruppe, welche durch 1 oder 2 Sauerstoffatome unterbrochen ist, eine Hydroxy-$C_1$-$C_4$-Alkoxygruppe, eine Hydroxy-$C_2$-$C_6$-Alkorygruppe, welche durch 1 oder 2 Sauerstoffatome unterbrochen ist, eine $C_1$-$C_4$-Alkoxycarbonylgruppe, eine Di-($C_1$-$C_4$-Alkyl)-aminogruppe, einen Phenylrest, welcher unsubstituiert oder durch Chlor oder Methoxy substituiert ist, einen Cyclohexylrest, einen Furan-, Tetrahydrofuran-, Tetrahydropyran-, Pyridino-, Piperidino- oder Morpholinorest substituiert ist;
- $C_3$-$C_{10}$-Alkenyl;
- $C_3$-$C_4$-Alkinyl, welches unsubstituiert oder durch eine Hydroxygruppe substituiert ist;
- Cyclohexyl, welches unsubstituiert oder durch 1 oder 2 Methylgruppen substituiert ist;
- Phenyl, welches unsubstituiert oder durch 1 bis 2 Substituenten aus der Gruppe Chlor, Nitro, $C_1$-$C_4$-Alkyl oder Methoxy substituiert ist;
- Naphthyl; oder
- Pyridin,

$R_5$
- $C_1$-$C_{12}$-Alkyl;
- $C_1$-$C_4$-Alkyl, welches durch eine $C_1$-$C_4$-Alkoxycarbonylgruppe substituiert ist;
- Phenyl, welches unsubstituiert oder durch ein Chloratom substituiert ist,

$R_6$
- Wasserstoff;
- $C_1$-$C_{18}$-Alkyl;
- $C_2$-$C_8$-Alkyl, welches durch eine Aminogruppe substituiert ist;
- $C_2$-$C_6$-Alkyl, welches durch eine Hydroxygruppe substituiert ist;
- $C_2$-$C_4$-Alkyl, welches durch ein Chlor- oder Bromatom, eine $C_1$-$C_4$-Alkylaminogruppe, eine Di-($C_1$-$C_4$-Alkyl)-aminogruppe, eine $C_1$-$C_4$-Hydroxyalkylaminogruppe, eine Di-($C_1$-$C_3$-Hydroxyalkyl)-aminogruppe, eine $C_1$-$C_4$-Alkoxygruppe oder eine $C_1$-$C_4$-Alkoxycarbonylgruppe substituiert ist;
- $C_1$-$C_4$-Alkyl, welches durch eine Cyanogruppe oder einen Phenylrest, welcher unsubstituiert oder durch Chlor substituiert ist, einen Furanyl-, Tetrahydrofuranyl, Piperdino- oder Morpholinorest substituiert ist;
- $C_1$-$C_3$-Alkoxy;
- $C_3$-$C_4$-Alkenyl;
- Cyclohexyl, welches unsubstituiert oder durch eine Methylgruppe substituiert ist;
- Phenyl, welches unsubstituiert oder durch 1 oder 2 Substituenten aus der Gruppe Chlor, Nitro, Cyano, Methyl, Aethyl, Methoxy und Trifluormethyl substituiert ist, und

$R_7$
- Wasserstoff;
- $C_1$-$C_6$-Alkyl;
- $C_1$-$C_4$-Alkyl, welches durch eine Hydroxygruppe oder eine Cyanogruppe substituiert ist;
- $C_2$-$C_4$-Alkoxyalkyl;
- $C_3$-$C_4$-Alkenyl;

- Cyclohexyl; oder
- Phenyl, oder

$R_6$ und $R_7$ gemeinsam mit dem Stickstoffatom, an das sie gebunden sind, einen unsubstituierten oder durch 1 oder 2 Methylgruppen substituierten Piperidino-, Tetrahydropyrimidino-, Morpholino- oder Imidazolylrest bedeuten, oder X und Y zusammen einen Tetrahydrofuran-2-on-3-yl-Ring bilden; zum Schützen von Kulturhirse, Reis, Mais und Getreidearten gegen kulturpflanzenschädigende Wirkungen von Herbiziden.

Wenn in den Verbindungen der Formel I X für $-CH(CH_3)-$ steht, oder X und Y zusammen einen Tetrahydrofuran-2-on-3-yl-Ring bilden, existieren optisch isomere Verbindungen. Im Rahmen der vorliegenden Erfindung sind unter den entsprechenden Verbindungen der Formel I sowohl die optisch reinen Isomere wie auch die Isomerengemische zu verstehen.

Besonders geeignet zur erfindungsgemässen Verwendung sind solche Verbindungen der Formel I, worin Y für $-COOR_4$ oder $-COSR_5$ oder $-CONR_6R_7$ steht oder X und Y zusammen einen Tetrahydrofuran-2-on-3-yl-Ring bilden.

Die Chinolinderivate der Formel I besitzen in hervorragendem Masse die Eigenschaft, Kulturhirse, Reis, Mais und Getreidearten gegen schädigende Wirkungen von Agrarchemikalien zu schützen. Als Agrarchemikalien kommen beispielsweise Defoliationsmittel, Desiccationsmittel, Mittel zum Schutz gegen Frostschäden und Pflanzenschutzmittel, wie beispielsweise Insektizide, Fungizide, Bakterizide, Nematozide und insbesondere Herbizide in Betracht. Die Agrarchemikalien können verschiedenen Stoffklassen angehören. Herbizide können beispielsweise zu einer der folgenden Stoffklassen gehören:
Triazine und Triazinone; Harnstoffe wie beispielsweise-1-(Benzthiazol-2-yl)-1,3-dimethylharnstoff ("Methabenzthiazuron") oder insbesondere Phenylharnstoffe, vor allem 3-(4-Isopropylphenyl)-1,1-dimethylharnstoff ("Isoproturon"), oder Sulfonylharnstoffe; Carbamate und Thiocarbamate; Halogenacetanilide, insbesondere Chloracetanilide; Chloracetamide; Halogenphenoxyessigsäureester; Diphenyläther, wie beispielsweise substituierte Phenoxyphenoxyessigsäureester und -amide und substituierte Phenoxyphenoxypropionsäureester und -amide; substituierte Pyridyloxyphenoxyessigsäureester und -amide und substituierte Pyridyloxyphenoxypropionsäureester und -amide, insbesondere 2-[4-(3,5-Dichlorpyridyl-2-oxy)-phenoxy]-propionsäure-2-propinylester und 2-[4-(5-Trifluormethylpyridyl-2-oxy)-phenoxy]-propionsäure-n-butylester; Benzoesäurederivate; Nitroaniline; Oxadiazolone; Phosphate; und Pyrazole.

Im einzelnen kommen beispielsweise folgende Substanzen in Betracht: Triazine und Triazinone: 2,4-Bis-(isopropylamino)-6-methylthio-1,3,5-triazin ("Prometryn"), 2,4-bis(äthylamino)-6-methylthio-1,3,5-triazin ("Simetryn"), 2-(1',2'-Dimethylpropylamino)-4-äthylamino-6-methylthio-1,3,5-triazin ("Dimethametryn"), 4-Amino-6-tert.butyl-4,5-dihydro-3-methylthio-1,2,4-triazin-5-on ("Metribuzin"), 2-Chlor-4-äthylamino-6-isopropylamino-1,3,5-triazin ("Atrazin"), 2-Chlor-4,6-bis-(äthylamino)-1,3,5-triazin ("Simazin"), 2-tert.Butylamino-4-chlor-6-äthylamino-1,3,5-triazin ("Terbuthylazin"), 2-tert.Butylamino-4-äthylamino-6-methoxy-1,3,5-triazin ("Terbumeton"), 2-tert.Butylamino-4-äthylamino-6-methylthio-1,3,5-triazin ("Terbutryn"), 2-Aethylamino-4-isopropylamino-6-methylthio-1,3,5-triazin ("Ametryn");
Harnstoffe: 1-(Benzothiazol-2-yl)-1,3-dimethylharnstoff; Phenylharnstoffe wie beispielsweise 3-(3-Chlor-p-tolyl)-1,1-dimethylharnstoff ("Chlortoluron"), 1,1-Dimethyl-3-($\alpha\alpha\alpha$-trifluor-m-tolyl)-harnstoff ("Fluometuron"), 3-(4-Brom-3-chlorphenyl)-1-methoxy-1-methylharnstoff ("Chlorbromuron"), 3-(4-Bromphenyl)-1-methoxy-1-methylharnstoff ("Metobromuron"), 3-(3,4-Dichlorphenyl)-1-methoxy-1-methylharnstoff ("Linuron"), 3-(4-Chlorphenyl)-1-methoxy-1-methylharnstoff ("Monolinuron"), 3-(3,4-Dichlorphenyl)-1,1-dimethylharnstoff ("Diuron"), 3-(4-Chlorphenyl)-1,1-dimethylharnstoff ("Monuron"), 3- (3-Chlor-4-methoxyphenyl)-1,1-dimethylharnstoff ("Metoxuron"); Sulfonylharnstoffe wie beispielsweise N-(2-Chlorphenylsulfonyl)-N'-(4-methoxy-6-methyl-1,3,5-triazin-2-yl)-harnstoff, N-(2-Methoxycarbonylphenylsulfonyl)-N'-(4,6-dimethylpyrimidin-2-yl)-harnstoff, N-(2,5-Dichlorphenylsulfonyl)-N'-(4,6-dimethoxypyrimidin-2-yl)-harnstoff, N-[2-(2-butenyloxy)-phenylsulfonyl]-N'-(4-methoxy-6-methyl-1,3,5-triazin-2-yl)-harnstoff sowie die in den europäischen Patentpublikationen 44808 und 44809 genannten Sulfonylharnstoffe;
Carbamate und Thiocarbamate: N-(3',4'-Dichlorphenyl)-propionanilid ("Propanil"), S-4-Chlorbenzyl-diäthylthiocarbamat ("Benthiocarb"), S-Aethyl-N,N-hexamethylen-thiocarbamat ("Molinate"), S-Aethyl-di-propylthiocarbamat ("EPTC"), N,N-di-sec.Butyl-S-benzyl-thiocarbamat, S-(2,3-Dichlorallyl)-di-isopropyl-thiocarbamat ("Di-allate"), 1-(Propylthiocarbonyl)-decahydro-chinaldin, S-Aethyl-di-isobutyl-thiocarbamat ("Butylate");
Chloracetanilide: 2-Chlor-2',6'-diäthyl-N-(2''-n-propoxyäthyl)-acet-anilid ("Propalochlor"), 2-Chlor-6'-äthyl-N-(2''-methoxy-1''-methyläthyl)-acet-o-toluidid ("Metolachlor"), 2-Chlor-2',6'-diäthyl-N-(butoxymethyl)acetanilid ("Butachlor"), 2-Chlor-6'-äthyl-N-(äthoxymethyl)acet-o-toluidid ("Acetochlor"), 2-Chlor-6'-äthyl-N-(2''-propoxy-1''-methyläthyl)acet-o-toluidid, 2-Chlor-2',6'-dimethyl-N-(2''-methoxy-1''-methyläthyl)acetanilid, 2-Chlor-2',6'-dimethyl-N-(2''-methoxyäthyl)acetanilid ("Dimethachlor"), 2-Chlor-2',6'-diäthyl-N-(pyrazol-1-ylmethyl)-acetanilid, 2-Chlor-6'-äthyl-N-(pyrazol-1-ylmethyl)acet-o-toluidid, 2-Chlor-6'-äthyl-N-(3,5-dimethyl-pyrazol-1-ylmethyl)acet-o-toluidid, 2-Chlor-6'-äthyl-N-(2''-butoxy-1''-methyläthyl)acet-o-toluidid ("Metazolachlor"), 2-Chlor-6'-äthyl-N-(2''-butoxyl-1''-(methyläthyl) acet-o-toluidid und 2-Chlor-2'-trimethylsilyl-N-(butoxymethyl)-acet-anilid;

Chloracetamide: N-[1-Isopropyl-2-methylpropen-1-yl-(1)]-N-(2'-methoxyäthyl)-chloracetamid;

Diphenyläther und Nitrodiphenyläther: 2,4-Dichlorphenyl-4'-nitrophenyläther ("Nitrofen"), 2-Chlor-1-(3'-äthoxy-4'-nitrophenoxy)-4-trifluormethyl-benzol ("Oxyfluorfen"), 2',4'-Dichlorphenyl-3-methoxy4-nitrophenyläther ("Chlormethoxynil"), 2-[4'-(2'',4''-Dichlorphenoxy)-phenoxy)propionsäure-methylester, N-(2'-Phenoxyäthyl)-2-[5'-(2''-chlor-4''-trifluormethylphenoxy)-phenoxy]-propionsäureamid, 2-[2-Nitro-5-(2-chlor-4-trifluormethylphenoxy)-phenoxy]-propionsäure-2-methoxyäthyl ester, 2-Chlor-4-trifluormethylphenyl-3'-oxazolin-2'-yl-4'-nitrophenyläther;

Benzoesäurederivate: Methyl-5-(2',4'-dichlorphenoxy)-2-nitrobenzoat ("Bifenox"), 5-(2'-Chlor-4'-trifluormethylphenoxy)-2-nitrobenzoesäure ("Acifluorfen"), 2,6-Dichlorbenzonitril ("Dichlobenil");

Nitroaniline: 2,6-Dinitro-N,N-dipropyl-4-trifluormethylanilin ("Trifluralin"), N-(1'-Aethylpropyl)-2,6-dinitro-3,4-xylidin ("Pendimethalin") ;

Oxadiazolone: 5-tert.-Butyl-3-(2',4'-dichlor-5'-isopropoxyphenyl)-1,3,4-oxadiazol-2-on ("Oxadiazon");

Phosphate: S-2-Methylpiperidino-carbonylmethyl-O,O-dipropyl-phosphorodithioat ("Piperophos") ;

Pyrazole: 1,3-Dimethyl-4-(2',4'-dichlorbenzoyl)-5-(4'-tolylsulfonyloxy)-pyrazol ;

2-[1-(Äthoxyamino)-butyl]-5-[2-(äthylthio)-propyl]-3-hydroxy-2-cyclohexen-1-on und das Natriumsalz von 2-[1-(N-Allyloxyamino)-butyliden]-5,5-dimethyl-4-methoxycarbonyl-cyclohexan-1,3-dion.

Besonders erwähnenswert ist die Schutzwirkung von Verbindungen der Formel I bei Getreide gegen schädigende Wirkungen von Herbiziden, wie Diphenyläthern und substituierten Pyridyloxyphenoxypropionsäureestern, insbesondere 2-[4-(3,5-Dichlorpyridyl-2-oxy)-phenoxy]-propionsäure-2-propinylester.

Gegenstand der vorliegenden Anmeldung sind auch die neuen Verbindungen der Formel I, worin

$R_1$ Wasserstoff, Chlor, Jod oder Brom, $R_2$ Wasserstoff, Chlor, Jod, Brom, Nitro, Methyl oder Aethyl, und $R_3$ Wasserstoff oder Methyl bedeuten, X für eine gegebenenfalls durch eine oder zwei Methylgruppen substituierte Methylenbrücke und Y für einen Oxazolin-2-yl-Rest, $-COOR_4$, $-COSR_5$ oder $-CONR_6R_7$ steht, worin

$R_4$
- ein quaternäres Ammoniumkation, ausgewählt aus der Gruppe Trimethyl-, Triäthyl- und Tri-(2-hydroxyäthylen)-ammoniumkation;
-Alkyl mit mehr als 5 C-Atomen aber höchstens 18 C-Atomen;
- $C_1$-$C_{10}$-Alkyl, welches durch 1 oder 2 Hydroxygruppen substituiert ist;
- $C_1$-$C_4$-Alkyl, welches durch 1 bis 3 Chlor- oder Bromatome, eine Nitrogruppe, eine Cyanogruppe, eine $C_1$-$C_4$-Alkoxygruppe, eine $C_2$-$C_8$-Alkoxygruppe, welche durch 1 oder 2 Sauerstoffatome unterbrochen ist, eine Hydroxy-$C_1$-$C_4$-Alkoxygruppe, eine Hydroxy-$C_2$-$C_6$-Alkoxygruppe, welche durch 1 oder 2 Sauerstoffatome unterbrochen ist, eine $C_1$-$C_4$-Alkoxycarbonylgruppe, eine Di-($C_1$-$C_4$-Alkyl)-aminogruppe, einen Phenylrest, welcher unsubstituiert oder durch Chlor oder Methoxy substituiert ist, einen Cyclohexylrest, einen Furan-, Tetrahydrofuran-, Tetrahydropyran-, Pyridino-, Piperidino- oder Morpholinorest substituiert ist;
- $C_3$-$C_{10}$-Alkenyl;
- $C_3$-$C_4$-Alkinyl, welches unsubstituiert oder durch eine Hydroxygruppe substituiert ist;
- Cyclohexyl, welches unsubstituiert oder durch 1 oder 2 Methylgruppen substituiert ist;
- Phenyl, welches unsubstituiert oder durch 1 bis 2 Substituenten aus der Gruppe Chlor, Nitro, $C_1$-$C_4$-Alkyl oder Methoxy substituiert ist;
- Naphthyl; oder
- Pyridin,

$R_5$
- $C_1$-$C_{12}$-Alkyl;
- $C_1$-$C_4$-Alkyl, welches durch eine $C_1$-$C_4$-Alkoxycarbonylgruppe substituiert ist;
- Phenyl, welches unsubstituiert oder durch ein Chloratom substituiert ist,

$R_6$
- Alkyl mit mehr als 6 C-Atomen aber höchstens 18 C-Atomen;
- $C_2$-$C_8$-Alkyl, welches durch eine Aminogruppe substituiert ist;
- $C_2$-$C_6$-Alkyl, welches durch eine Hydroxygruppe substituiert ist;
- $C_2$-$C_4$-Alkyl, welches durch ein Chlor- oder Bromatom, eine
- $C_1$-$C_4$-Hydroxyalkylaminogruppe, eine Di-($C_1$-$C_3$-Hydroxyalkyl)-amino gruppe oder eine $C_1$-$C_4$-Alkoxycarbonylgruppe substituiert ist;
- $C_1$-$C_4$-Alkyl, welches durch eine Cyanogruppe oder einen Phenylrest, welcher unsubstituiert oder durch Chlor substituiert ist, einen Furanyl-, Tetrahydrofuranyl-, Piperidino- oder Morpholinorest substituiert ist;
- $C_1$-$C_3$-Alkoxy;
- Cyclohexyl, welches durch eine Methylgruppe substituiert ist;

- Phenyl, welches durch 1 oder 2 Substituenten aus der Gruppe Nitro, Cyano, Methyl, Aethyl, Methoxy und Trifluormethyl substituiert ist, und

$R_7$ - Wasserstoff;
- $C_1$-$C_6$-Alkyl;
- $C_1$-$C_4$-Alkyl, welches durch eine Hydroxygruppe oder eine Cyanogruppe substituiert ist;
- $C_2$-$C_4$-Alkoxyalkyl;
- $C_3$-$C_4$-Alkenyl;
- Cyclohexyl; oder
- Phenyl, oder

$R_6$ und $R_7$ gemeinsam mit dem Stickstoffatom, an das sie gebunden sind, einen durch 1 oder 2 Methylgruppen substituierten Piperidino-, Tetrahydropyrimidino-, Morpholino- oder Imidazolylrest bedeuten, oder X und Y zusammen einen Tetrahydrofuran-2-on-3-yl-Rest bilden.

Bevorzugte Gruppen von Verbindungen der Formel I stellen jene dar, worin Y für $-COOR_4$ oder $-COSR_5$ oder $-CONR_6R_7$ steht, oder worin X und Y zusammen einen unsubstituierten Tetrahydrofuran-2-on-3-yl-Ring darstellen.

Ein geeignetes Verfahren zum Schützen von Kulturhirse, Reis, Mais und Getreidearten unter Verwendung dieser Verbindungen der Formel I besteht darin, dass man die Kulturpflanzen, Teile dieser Pflanzen oder für den Anbau dieser Kulturpflanzen bestimmte Böden vor oder nach dem Einbringen des pflanzlichen Materials in den Boden mit einer Verbindung der Formel I oder einem Mittel, welches eine solche Verbindung enthält, behandelt. Die Behandlung kann vor, gleichzeitig mit oder nach dem Einsatz der Agrarchemikalie erfolgen. Als Pflanzenteile kommen insbesondere diejenigen in Betracht, die zur Neubildung einer Pflanze befähigt sind, wie beispielsweise Samen, Früchte, Stengelteile und Zweige (Stecklinge) sowie auch Wurzeln, Knollen und Rhizome. Gegenstand der vorliegenden Anmeldung sind somit auch Mittel zum Schützen von Kulturhirse, Reis, Mais und Getreidearten gegen kulturpflanzenschädigende Wirkungen von Herbiziden, wobei diese Mittel als aktive Komponente eine Verbindung der Formel I enthalten.

Die Verbindungen der Formel I können auch zur selektiven Bekämpfung von Unkräutern in Beständen von Kulturhirse, Reis, Mais und Getreidearten verwendet werden, wobei die Bestände, Teile der betreffenden Kulturpflanzen oder Anbauflächen dieser Kulturpflanzen mit einem Herbizid und einer Verbindung der Formel I oder einem Mittel, welches diese Kombination enthält, behandelt. Gegenstand der vorliegenden Erfindung sind somit auch Mittel, die neben der Verbindung der Formel I auch ein kulturpflanzenschädigendes Herbizid enthalten.

Vorzugsweise handelt es sich bei diesem Herbizid um einen Diphenyläther oder einen substituierten Pyridyloxyphenoxypropionsäureester, insbesondere 2-[4-(3,5-Dichlorpyridyl-2-oxy)-phenoxy]-propionsäure-2-propinylester.

Bei den zu bekämpfenden Unkräutern kann es sich sowohl um monokotyle wie um dikotyle Unkräuter handeln.

Als Anbauflächen gelten die bereits mit den genannten Kulturpflanzen bewachsenen oder die ausgesäten Bodenareale, wie auch die zur Bebauung mit den Kulturpflanzen bestimmten Böden.

Die zu applizierende Aufwandmenge Antidot im Verhältnis zur Agrarchemikalie richtet sich weitgehend nach der Anwendungsart. Bei einer Feldbehandlung, welche entweder unter Verwendung einer Tankmischung oder durch getrennte Applikation von Agrarchemikalie und Antidot durchgeführt wird, liegt in der Regel ein Verhältnis von Antidot zu Agrarchemikalie von 1:100 bis 10:1, bevorzugt 1:5 bis 8:1, und insbesondere 1:1, vor.

Dagegen werden bei der Samenbeizung und ähnlichen Einsatzmethoden weit geringere Mengen Antidot im Verhältnis zur Aufwandmenge an Agrarchemikalie/ha Anbaufläche benötigt. Bei der Samenbeizung werden in der Regel 0,1 bis 10 g Antidot/kg Samen, bevorzugt 1 bis 2 g, appliziert. Wird das Gegenmittel kurz vor der Aussaat unter Samenquellung appliziert, so werden zweckmässigerweise Antidot-Lösungen verwendet, welche den Wirkstoff in einer Konzentration von 1 bis 10 000 ppm, bevorzugt 100 bis 1000 ppm, enthalten.

Die vorliegende Erfindung betrifft auch Mittel, welche Verbindungen der Formel I, ein Trägermaterial und/oder inerte Zuschlagstoffe enthalten.

Zur Applikation als Antidot oder Wuchsregulator werden die Verbindungen der Formel I oder Kombinationen von Verbindungen der Formel I mit zu antagonisierenden Agrarchemikalien zweckmässigerweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln,

Verstäuben, Verstreuen, Bestreichen oder Giessen werden gleich wie die Art der Mittel den angestrebten Zielen und den gegebenen Verhältnissen entsprechend gewählt.

Die Formulierungen, d.h., die den Wirkstoff der Formel I oder eine Kombination von Wirkstoff der Formel I mit zu antagonisierender Agrarchemikalie und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon,Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffs der Formel I und gegebenenfalls auch der zu antagonisierenden Agrarchemikalie nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls suhstituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierre Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ringwood New Jersey, 1980

Sisely and Wood, "Encyclopedia of Surface Active Agents", Chemical Publishing Co., Inc. New York, 1980

Die agrochemischen Zubereitungen enthalten in der Regel 0,1 bis 99 %, insbesondere 0,1 bis 95 %, Wirkstoff der Formel I, 99,9 bis 1 % insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel I oder sie enthaltende Mittel zum Schützen von Kulturpflanzen gegen schädigende Wirkungen von Agrarchemikalien kommen verschiedene Methoden und Techniken in Betracht, wie beispielsweise die folgenden:

i) Samenbeizung

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei etwa 10 bis 500 g Wirkstoff der Formel I (40 g bis 2 kg Spritzpulver) pro 100 kg Saatgut.

b) Beizung der Samen mit einem Emulsionskonzentrat des Wirkstoffs der Formel I nach der Methode a) (Nassbeizung).

c) Beizung durch Tauchen des Saatguts in eine Brühe mit 50-3200 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 10 g bis 500 g, vorzugsweise 50 bis 250 g AS pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Gegenmittel und Herbizid (gegenseitiges Mengenverhältnis zwischen 10:1 und 1:10) wird verwendet, wobei die Aufwandmenge an Herbizid 0,1 bis 10 kg pro Hektar beträgt. Solche Tankmischung wird vorzugsweise vor oder unmittelbar nach der Aussaat appliziert oder 5 bis 10 cm tief in den noch nicht gesäten Boden eingearbeitet.

iii) Applikation in die Saatfurche

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Die Herstellung von Verbindungen der Formel I erfolgt, indem man

a) eine Verbindung der Formel II

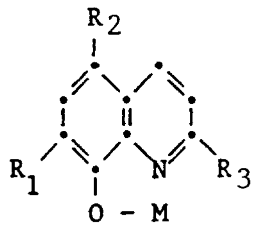

(II),

worin $R_1$, $R_2$ und $R_3$ die angegebenen Bedeutungen haben und M für Wasserstoff, ein Alkali- oder Erdalkalimetallatom steht, mit einer Verbindung der Formel III

Z - X - Y (III)

worin X und Y die angegebenen Bedeutungen haben und Z für einen abspaltbaren Rest steht, umsetzt, oder

b) zur Herstellung von Verbindungen der Formel I, in denen Y für $-COOR_4$ steht, ein Säurehalogenid der Formel IV

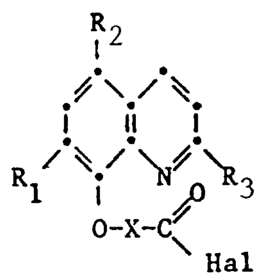

(IV),

worin $R_1$, $R_2$, $R_3$ und X die angegebenen Bedeutungen haben und Hal für ein Halogenatom steht, mit einer Verbindung der Formel V

$M' - O - R_4$ (V),

worin $R_4$ die angegebene Bedeutung hat und M' für Wasserstoff, ein Alkali- oder Erdalkalimetallatom steht, umsetzt, oder

c) zur Herstellung von Verbindungen der Formel I, in denen Y für $-COSR_5$ steht, ein Säurehalogenid der Formel VI

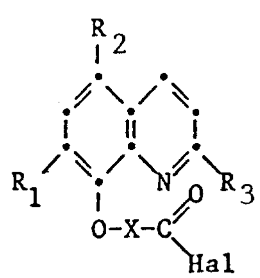

(VI),

worin $R_1$, $R_2$, $R_3$ und X die angegebenen Bedeutungen haben und Hal für ein Halogenatom steht, mit einer Verbindung der Formel VII

$M'' - S - R_5$ (VII),

worin $R_5$ die angegebene Bedeutung hat und M'' für Wasserstoff, ein Alkali- oder Erdalkalimetallatom steht, umsetzt, oder

d) zur Herstellung von Verbindungen der Formel I, in denen Y für $-CONR_6R_7$ steht, ein Säurehalogenid der Formel VIII

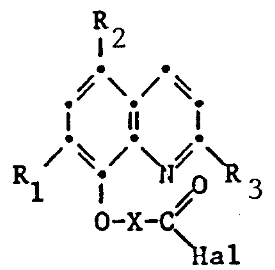

(VIII),

worin $R_1$, $R_2$, $R_3$ und X die angegebenen Bedeutungen haben und Hal für ein Halogenatom steht, mit einer Verbindung der Formel IX

$HNR_6R_7$ (IX),

worin $R_9$ und $R_{10}$ die angegebenen Bedeutungen haben, umsetzt, oder

e) zur Herstellung von Verbindungen der Formel I, in denen Y für $-COOR_4$ steht, eine Verbindung der Formel X

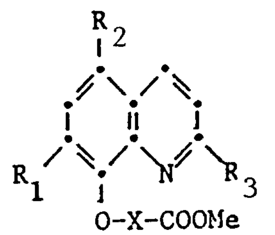

(X),

worin $R_1$, $R_2$, $R_3$ und X die angegebenen Bedeutungen haben und Me für ein Alkalimetall-, Erdalkalimetall-, Blei- oder oder Silberatom steht, mit einer Verbindung der Formel XI

Hal - $R_4$ (XI),

worin $R_7$ die angegebene Bedeutung hat und Hal für ein Halogenatom steht, umsetzt, oder

f) zur Herstellung von Verbindungen der Formel I, in denen Y für $-COSR_5$ steht, eine Verbindung der Formel XII

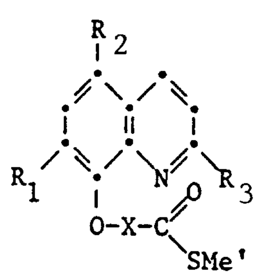

(XII),

worin $R_1$, $R_2$, $R_3$ und X die angegebenen Bedeutungen haben und Me' für ein Alkalimetall-, Erdalkalimetall-, Blei- oder Silberatom steht, mit einer Verbindung der Formel XIII

Hal - $R_5$ (XIII),

worin $R_5$ die angegebene Bedeutung hat und Hal für ein Halogenatom steht, umsetzt, oder

g) zur Herstellung von Verbindungen der Formel I, in denen Y für $-COOR_4$ steht, eine Verbindung der Formel XIV

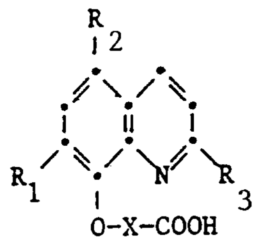

(XIV),

worin $R_1$, $R_2$, $R_3$ und X die angegebenen Bedeutungen haben, mit einer Verbindung der Formel XV

HO - $R_4$ (XV),

worin $R_7$ die angegebene Bedeutung hat, umsetzt, oder
h) zur Herstellung von Verbindungen der Formel I, in denen Y für $-COSR_5$ steht, eine Verbindung der Formel XVI

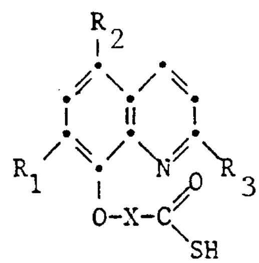

(XVI),

worin $R_1$, $R_2$, $R_3$ und X die angegebenen Bedeutungen haben, mit einer Verbindung der Formel XVII

HO - $R_5$ (XVII),

worin $R_5$ die angegebene Bedeutung hat, umsetzt, oder
i) zur Herstellung von Verbindungen der Formel I, in denen Y für $-COOR_4$ steht, eine Verbindung der Formel Ia

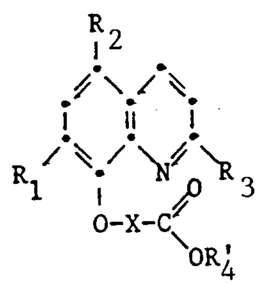

(Ia),

worin $R_1$, $R_2$, $R_3$ und X die angegebenen Bedeutungen haben und $R_4'$ die für $R_4$ angegebene Bedeutung hat, mit einer Verbindung der Formel XVIII

HO - $R_4''$ (XVIII),

worin $R_4''$ die für $R_4$ angegebene Bedeutung hat und nicht mit $R_4'$ identisch ist, umsetzt, oder
k) zur Herstellung von Verbindungen der Formel I, in denen Y für $-COSR_5$ steht, eine Verbindung der Formel Ib

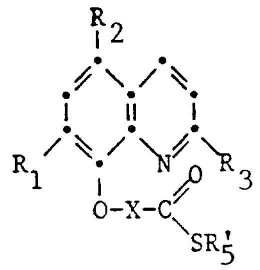

(Ib),

worin $R_1$, $R_2$, $R_3$ und X die angegebenen Bedeutungen haben und $R_5'$ die für $R_5$ angegebene Bedeutung hat, mit einer Verbindung der Formel XIX

$HSR_5''$ (XIX),

worin $R_5''$ die für $R_5$ in Formel I angegebene Bedeutung hat und nicht mit $R_5'$ identisch ist, umsetzt, oder
l) zur Herstellung von Verbindungen der Formel I, in denen Y für $-CONR_6R_7$ steht, eine Verbindung der Formel XX

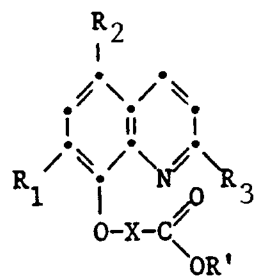

(XX),

worin $R_1$, $R_2$, $R_3$ und X die angegebenen Bedeutungen haben und R' für einen aliphatischen, acyclischen Kohlenwasserstoffrest steht, mit einer Verbindung der Formel XXI

$HNR_6R_7$ (XXI),

worin $R_6$ und $R_7$ die angegebenen Bedeutungen haben, umsetzt, oder
m) zur Herstellung von Verbindungen der Formel I, in denen X für $-CH_2CH_2-$ steht, eine Verbindung der Formel XXII

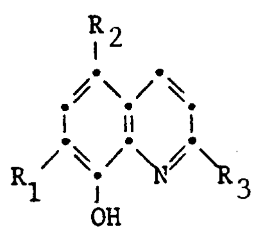

(XXII),

worin $R_1$, $R_2$ und $R_3$ die angegebenen Bedeutungen haben, mit einer Verbindung der Formel XXIII

$CH_2 = CH - Y$ (XXIII),

worin Y die angegebene Bedeutung hat, umsetzt, oder
n) zur Herstellung von Verbindungen der Formel I, in denen Y für einen gegebenenfalls substituierten Oxazolin-2-yl-Rest steht, eine Verbindung der Formel XXIV

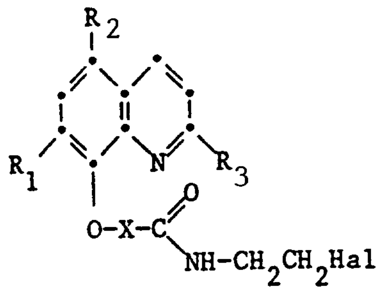

(XXIV),

worin $R_1$, $R_2$, $R_3$ und X die angegebenen Bedeutungen haben und Hal für ein Halogenatom, insbesondere ein Chlor- oder Bromatom, steht, in Gegenwart eines säurebindenden Mittels cyclisiert.

Bei der Verfahrensvariante a) kommt in der Verbindung der Formel III als abspaltbarer Rest Z insbesondere ein Halogenatom oder eine Methylsulfonyloxy-, Phenylsulfonyloxy- oder para-Tolylsulfonyloxygruppe in Betracht. Halogen steht hier für Fluor, Chlor, Brom und Jod, bevorzugt für Chlor und Brom.

Wenn in der Verbindung der Formel II M für Wasserstoff und in der Verbindung der Formel III Z für ein Halogenatom steht, so lässt sich die Umsetzung bevorzugt in Gegenwart eines üblichen Protonenakzeptors durchführen. Ferner wirkt, wenn in der Verbindung der Formel III Z für ein Halogenatom steht, die Zugabe einer geringen Menge Alkalijodid katalytisch.

Bei den Verfahrensvarianten b), c) und d) steht in den Verbindungen der Formeln IV, VI und VIII Hal für ein Halogenatom, wobei als Halogenatom Fluor, Chlor, Brom und Jod, vorzugsweise Chlor und Brom, in Betracht kommen.

Wenn bei den Verfahrensvarianten b) und c) in den Verbindungen der Formeln V oder VII M' oder M" für Wasserstoff stehen, wird die Reaktion wie bei der Verfahrensvariante d) vorzugsweise in Gegenwart eines säurebindenden Mittels durchgeführt.

Als Säurehalogenide der Verbindungen IV, VI und VIII kommen vorzugsweise Halogenwasserstoffsäuren, insbesondere Chlor- und Bromwasserstoffsäure, in Betracht.

Bei den Verfahrensvarianten g) und h) lässt sich das bei der Umsetzung entstehende Wasser beispielsweise mittels eines Wasserabscheiders aus dem Reaktionsgemisch entfernen. Durch Zugabe von Säure wird eine katalytische Wirkung erzielt.

Die Umesterung gemäss den Verfahrensvarianten i) und k) kann durch Zusatz von Säure oder Base katalytisch beeinflusst werden. Vorteilhafterweise wird die Umsetzung mit einem Ueberschuss an Verbindungen der Formeln XIII oder XIX durchgeführt.

Bei der Verfahrensvariante 1) steht in den Verbindungen der Formel XX R' vorzugsweise für einen Alkylrest mit 1 bis 6 Kohlenstoffatomen, insbesondere Methyl oder Aethyl.

Die Umsetzungen gemäss Verfahrensvarianten a) bis n) werden zweckmässigerweise in Gegenwart von gegenüber den Reaktionsteilnehmern inerten Lösungsmitteln durchgeführt. Als inerte Lösungsmittel kommen beispielsweise Kohlenwasserstoffe wie Benzol, Toluol, Xylol, Petroläther oder Cyclohexan, Aether wie beispielsweise Diäthyläther, Tetrahydrofuran, Dioxan, Dimethoxyäthan oder Diäthylenglykol-dimethyläther, Säureamide wie beispielsweise Dimethylformamid, 2-Pyrrolidinon oder Hexamethyl-phosphorsäuretriamid oder Sulfoxide wie beispielsweise Dimethylsulfoxid in Betracht.

Als säurebindende Mittel können beispielsweise Alkalimetall- und Erdalkalimetallhydroxide oder -alkoholate, Alkalicarbonate oder tertiäre organische Basen eingesetzt werden.

Die Reaktionstemperaturen liegen im allgemeinen in einem Bereich von 0 bis 200 °C, insbesondere im Bereich von 50 bis 150 °C.

Die in den Verfahrensvarianten a) bis n) verwendeten Ausgangsprodukte sind bekannt oder können analog bekannten Verfahren hergestellt werden.

Aus der Literatur ist eine Anzahl von Chinolinderivaten und ihre Verwendung in verschiedenen Anwendungsbereichen bekannt. So beschreiben beispielsweise die US-A-4.176.185, die GB-A-760.319, GB-A-989.578, GB-A-1.003.477 und GB-A-1.003.478, die CH-A-408.007, die DE-A-25 46 845, Areschka, A. et al., Eur.J.Med.Chem.-Chimica Therapeutica, Sept.-Okt. 1975-10, No. 5, 463-469, Major R.T. et al., J. Med. Pharm. Chem. 4, 317-326, 1961, und Thompson, H.E., Botan. Gaz. 107, 476-507, 1946, den Einsatz von Chinolinderivaten auf therapeutischem Gebiet, als Ausgangsprodukt zur Herstellung therapeutischer Wirkstoffe, als Mittel zur Wachstumsförderung bei Tieren, als Pflanzenwuchshemmer oder als Herbizide.

Einen Bestandteil der vorliegenden Erfindung bilden neue Chinolinderivate der Formel I. Besonders erwähnenswert sind neue Verbindungen der Formel I, die zu einer der vorgängig aufgeführten Untergruppen o), p), q), r), s), t), u), v), w), x), y), z), $z^1$) , $z^2$), $z^3$) und $z^4$) gehören.

Beispiel 1: 23,2 g 8-Hydroxychinolin werden in der Wärme in 400 ml Butanon-2 gelöst und portionenweise mit 30 g Kaliumcarbonat versetzt. Das Gemisch wird eine Stunde unter Rückfluss erhitzt. Anschliessend werden zunächst 2 g Kaliumjodid und dann unter Rühren und Kochen 40 g 2-Brompropionsäuremethylester in 100 ml Butanon-2 innerhalb einer Stunde zugetropft. Dann wird das Gemisch noch 10 Stunden unter Rückfluss erhitzt. Nach dem Abkühlen auf Raumtemperatur wird das Gemisch auf 1 Liter Wasser gegossen und dreimal mit je 200 ml Aethylacetat extrahiert. Die vereinigten Extrakte werden einmal mit 50 ml Wasser gewaschen, über Natriumsulfat getrocknet und filtriert. Das Lösungsmittel wird verdampft und der ölige Rückstand durch Anreiben mit Petroläther zur Kristallisation gebracht. Nach dem Umkristallisieren aus Hexan erhält man 2-(8-Chinolinoxy)-propionsäuremethylester (Verbindung Nr. 3) in Form beigefarbener Kristalle. Smp. 70 bis 72° C.

Analog einer der vorstehend beschriebenen Methoden lassen sich auch die folgenden, in der Tabelle 1 zusammen mit der Verbindung des vorstehenden Beispiels aufgeführten Verbindungen der Formel I herstellen:

Tabelle 1

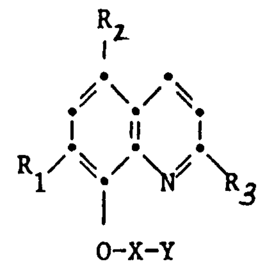

| Verbindung No. | $R_1$ | $R_2$ | $R_3$ | X | Y | Physikal. Konstante |
|---|---|---|---|---|---|---|
| 1 | Cl | Cl | H | $-CH(CH_3)-$ | $-COOCH_3$ | Smp. 65-66° C |
| 2 | H | H | H | $-CH(CH_3)-$ | $-COO(CH_2)_{13}-CH_3$ | |
| 3 | H | H | H | $-CH(CH_3)-$ | $-COOCH_3$ | Smp. 70-72° C |
| 4 | H | H | H | $-CH_2-$ | $-COOH \cdot H_2O$ | Smp.184-185°C |

Tabelle 1 (Fortsetzung)

| Verbindung No. | $R_1$ | $R_2$ | $R_3$ | X | Y | Physikal. Konstante |
|---|---|---|---|---|---|---|
| 5 | H | H | H | $-CH-$<br>$CH_3$ | $-COOH$ | |
| 6 | H | H | H | $-CH_2-$ | $-COOCH_2CH_2OCH_3$ | Smp. 80-82°C |
| 7 | H | H | H | $-CH-$<br>$CH_3$ | $-COOCH_2CH_2OCH_3$ | |
| 8 | H | H | H | $-CH_2-$ | $-COOCH_3$ | Smp. 46.5-67.0°C |
| 9 | H | H | H | $-CH-$<br>$CH_3$ | $-COOCH_2CH_2OC_2H_5$ | |
| 10 | H | H | H | $-CH-$<br>$CH_3$ | 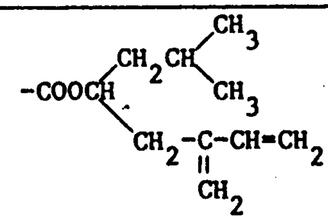 | |

Tabelle 1 (Fortsetzung)

| Verbindung No. | $R_1$ | $R_2$ | $R_3$ | X | Y | Physikal. Konstante |
|---|---|---|---|---|---|---|
| 11 | H | H | H | $-CH_2-$ | $-COOC_2H_5 \cdot H_2O$ | Smp. 56-59°C |
| 12 | H | H | H | -CH-<br>I<br>$CH_3$ | 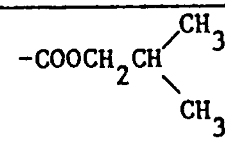 | |
| 13 | H | H | H | -CH-<br>I<br>$CH_3$ | $-CONH(CH_2)_3OC_2H_5$ | Smp. 54-56°C |
| 14 | H | H | H | -CH-<br>I<br>$CH_3$ | 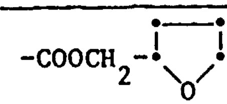 | |
| 15 | H | H | H | -CH-<br>I<br>$CH_3$ | $-COOC_2H_5$ | |
| 16 | H | H | H | -CH-<br>I<br>$CH_3$ | 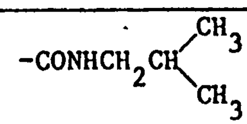 | |

Tabelle 1 (Fortsetzung)

| Verbindung No. | $R_1$ | $R_2$ | $R_3$ | X | Y | Physikal. Konstante |
|---|---|---|---|---|---|---|
| 17 | H | H | H | -CH-<br>\|<br>$CH_3$ | $-CONHC_2H_5$ | Smp. 86-88°C |
| 18 | H | H | H | -CH-<br>\|<br>$CH_3$ | 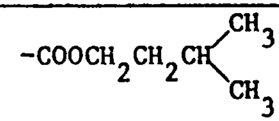 | |
| 19 | H | H | H | -CH-<br>\|<br>$CH_3$ | 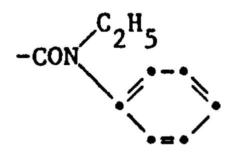 | |
| 20 | H | H | H | $-CH_2-$ | $-COOC_3H_7n$ | Smp. 28-31°C |
| 21 | H | H | H | -CH-<br>\|<br>$CH_3$ | 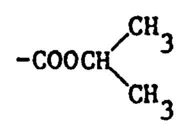 | |
| 22 | H | H | H | -CH-<br>\|<br>$CH_3$ | 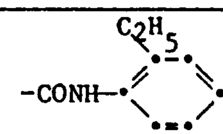 | |

Tabelle 1 (Fortsetzung)

| Verbindung No. | $R_1$ | $R_2$ | $R_3$ | X | Y |
|---|---|---|---|---|---|
| 23 | H | H | H | -CH-<br>$CH_3$ | 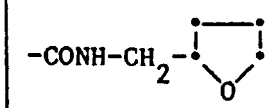 |
| 24 | H | H | H | -CH-<br>$CH_3$ | 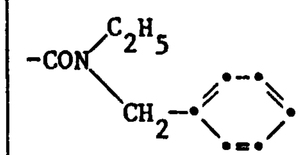 |
| 25 | H | H | H | -CH-<br>$CH_3$ | 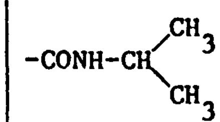 |
| 26 | H | H | H | -CH-<br>$CH_3$ | $-COOCH_2CH(C_2H_5)_2$ |
| 27 | H | H | H | -CH-<br>$CH_3$ | 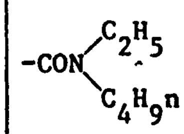 |

Tabelle 1 (Fortsetzung)

| Verbindung No. | $R_1$ | $R_2$ | $R_3$ | X | Y | Physikal. Konstante |
|---|---|---|---|---|---|---|
| 28 | H | H | H | $-CH_2-$ | $-COOC_3H_7$iso | $n_D^{23}$ = 1,5696 |
| 29 | H | H | H | $-CH(CH_3)-$ | $-COOCH_2CH_2OH$ | |
| 30 | H | H | H | $-CH(CH_3)-$ | $-COSCH(CH_3)_2$ | |
| 31 | H | H | H | $-CH(CH_3)-$ | $-CONHCH_2CH(C_2H_5)(CH_2)_3CH_3$ | |
| 32 | H | H | H | $-CH_2-$ | $-CONHCH_3$ • $H_2O$ | Smp. 74-81°C |
| 33 | H | H | H | $-CH(CH_3)-$ | $-COSC_2H_5$ | |

Tabelle 1 (Fortsetzung)

| Verbindung No. | $R_1$ | $R_2$ | $R_3$ | X | Y | Physikal. Konstante |
|---|---|---|---|---|---|---|
| 34 | H | H | H | $-CH-$<br>$CH_3$ | 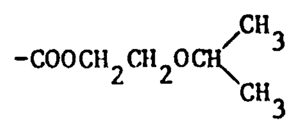 | |
| 35 | H | H | H | $-CH-$<br>$CH_3$ | 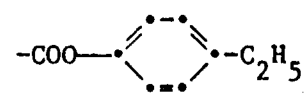 | |
| 36 | H | H | H | $-CH_2-$ | 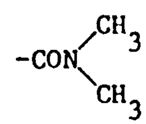 | Smp. 142-145°C |
| 37 | H | H | H | $-CH_2-$ | $-CONHC_2H_5$ | $n_D^{22,5} = 1,6002$ |
| 38 | H | H | H | $-CH-$<br>$CH_3$ | $-COOCH_2CH{=}CH_2$ | |

Tabelle 1 (Fortsetzung)

| Verbindung No. | $R_1$ | $R_2$ | $R_3$ | X | Y |
|---|---|---|---|---|---|
| 39 | H | $NO_2$ | H | $-CH-$<br>$CH_3$ | $-COOH$ |
| 40 | H | H | H | $-CH-$<br>$CH_3$ | $-CONHCH_2-CH=CH_2$ |
| 41 | H | H | H | $-CH-$<br>$CH_3$ | $-COO-$ 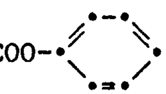 $-CH_3$ |
| 42 | H | H | H | $-CH-$<br>$CH_3$ | $-CONHCH_2CH_2Br$ |
| 43 | H | H | H | $-CH-$<br>$CH_3$ | $-CONHCH_2CH_2-$ 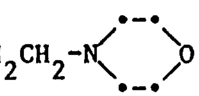 |

Tabelle 1 (Fortsetzung)

| Verbindung No. | $R_1$ | $R_2$ | $R_3$ | X | Y |
|---|---|---|---|---|---|
| 44 | H | H | H | -CH-<br>$CH_3$ | $CH_3$<br>$-COOCH_2-C=CH_2$ |
| 45 | H | H | H | -CH-<br>$CH_3$ | 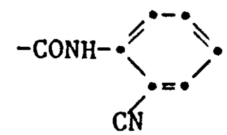 |
| 46 | H | H | H | $-CH_2-$ | 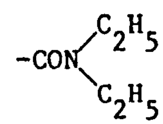 |
| 47 | H | H | H | -CH-<br>$CH_3$ | 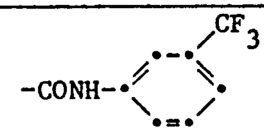 |
| 48 | H | $NO_2$ | H | -CH-<br>$CH_3$ | $-COOCH_2CH_2OCH_3$ |

Tabelle 1 (Fortsetzung)

| Verbindung No. | $R_1$ | $R_2$ | $R_3$ | X | Y |
|---|---|---|---|---|---|
| 49 | H | H | H | $-CH(CH_3)-$ | $-CONHCH(C_2H_5)CH_2OH$ |
| 50 | H | H | H | $-CH(CH_3)-$ | $-COOCH_2CH_2OCH_3$ |
| 51 | H | H | H | $-CH(CH_3)-$ | $-CONH-C(C_2H_5)_2-CH_2OH$ |
| 52 | H | H | H | $-CH_2-$ | $-CONH_2$ |

Tabelle 1 (Fortsetzung)

| Verbindung No. | $R_1$ | $R_2$ | $R_3$ | X | Y | Physikal. Konstante |
|---|---|---|---|---|---|---|
| 53 | H | H | H | $-CH(CH_3)-$ | 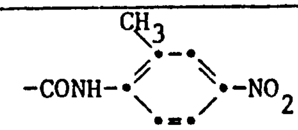 | |
| 54 | H | H | H | $-CH(CH_3)-$ | $-CONHCH_2CH_2OCH_3$ | |
| 55 | H | H | H | $-CH(CH_3)-$ | $-CONH(CH_2)_3OH$ | Smp. 120-122°C |
| 56 | H | H | H | $-CH_2-$ | $-COOCH_2CH_2OC_2H_5$ | $n_D^{24}$ = 1,5673 |

Tabelle 1 (Fortsetzung)

| Verbindung No. | $R_1$ | $R_2$ | $R_3$ | X | Y |
|---|---|---|---|---|---|
| 57 | H | H | H | $-CH-$<br>$CH_3$ | $-CONH_2$ |
| 58 | H | H | H | $-CH-$<br>$CH_3$ | 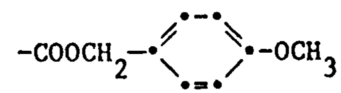 |
| 59 | H | H | H | $-CH-$<br>$CH_3$ | 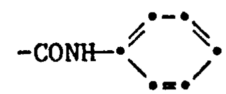 |
| 60 | H | $NO_2$ | H | $-CH-$<br>$CH_3$ | $-COOCH_3$ |

Tabelle 1 (Fortsetzung)

| Verbindung No. | $R_1$ | $R_2$ | $R_3$ | X | Y | Physikal. Konstante |
|---|---|---|---|---|---|---|
| 61 | H | H | H | -CH-<br>$CH_3$ | -CONH— 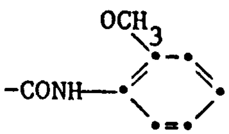 | |
| 62 | H | H | H | -CH-<br>$CH_3$ | $-CONH(CH_2)_3OCH_3$ | |
| 63 | H | H | H | -CH-<br>$CH_3$ | $-CONHCH_2$— 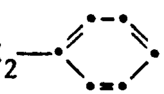 | Smp. 88-90°C |
| 64 | H | $NO_2$ | H | -CH-<br>$CH_3$ | $-COOC_2H_5$ | |
| 65 | H | H | H | -CH-<br>$CH_3$ | $-CONHCH_3$ | |

Tabelle 1 (Fortsetzung)

| Verbindung No. | $R_1$ | $R_2$ | $R_3$ | X | Y | Physikal. Konstante |
|---|---|---|---|---|---|---|
| 66 | H | H | H | 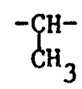 | 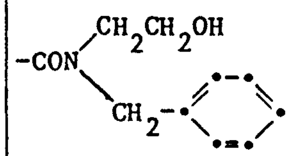 | |
| 67 | H | H | H | $-CH_2-$ | $-CONH(CH_2)_3CH_3$ | Smp. 66-68°C |
| 68 | H | H | H | 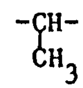 | 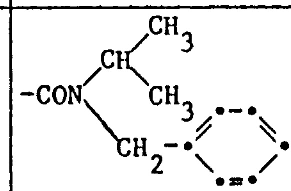 | |
| 69 | H | H | H | 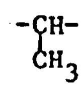 | 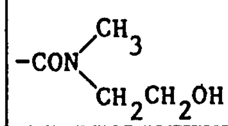 | $n_D^{22}$ = 1,6054 |
| 70 | H | H | H | 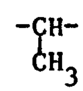 | 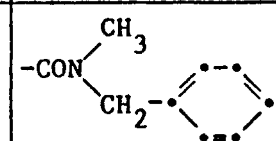 | |
| 71 | H | $NO_2$ | H | 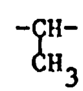 | $-COOC_3H_7n$ | |

Tabelle 1 (Fortsetzung)

| Verbindung No. | $R_1$ | $R_2$ | $R_3$ | X | Y |
|---|---|---|---|---|---|
| 72 | H | H | H | $-CH-$<br>$CH_3$ | $CH_2CH_2OH$<br>$-CONH(CH_2)_3-N$<br>$CH_2CH_2OH$ |
| 73 | H | $NO_2$ | H | $-CH-$<br>$CH_3$ | $-COOC_3H_7$ iso |
| 74 | H | H | H | $-CH-$<br>$CH_3$ | OH<br>$-CON(CH_2CHCH_3)_2$ |
| 75 | H | H | H | $-CH-$<br>$CH_3$ | $-CON(CH_2CH_2OCH_3)_2$ |
| 76 | H | H | H | $-CH-$<br>$CH_3$ | $-CONH(CH_2)_3NHCH_3$ |
| 77 | H | H | H | $-CH-$<br>$CH_3$ | $-COOCH_2CH_2Br$ |
| 78 | H | $NO_2$ | H | $-CH-$<br>$CH_3$ | $-CONHCH_3$ |
| 79 | H | H | H | $-CH-$<br>$CH_3$ | $-COO(CH_2)_4OH$ |

Tabelle 1 (Fortsetzung)

| Verbindung No. | $R_1$ | $R_2$ | $R_3$ | X | Y | Physikal. Konstante |
|---|---|---|---|---|---|---|
| 80 | H | H | H | $-CH_2-$ | 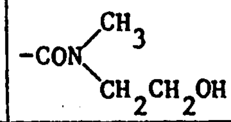 | Smp. 146-149°C |
| 81 | H | H | H | 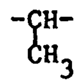 | 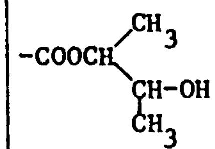 | |
| 82 | H | H | H | 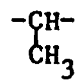 | 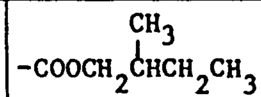 | |
| 83 | H | H | H | 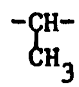 | $-COOCH_2-C{\equiv}C-CH_2OH$ | |
| 84 | H | H | H | $-CH_2-$ | 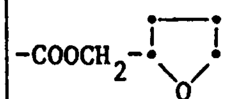 | zähe Masse |
| 85 | H | H | H | 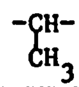 | $-COOC_4H_9n$ | |
| 86 | H | H | H | 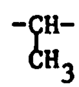 | 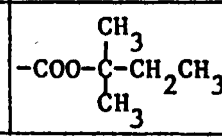 | |

Tabelle 1 (Fortsetzung)

| Verbindung No. | $R_1$ | $R_2$ | $R_3$ | X | Y | Physikal. Konstante |
|---|---|---|---|---|---|---|
| 87 | H | H | H | 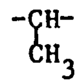 | 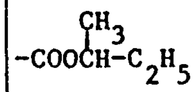 | |
| 88 | H | $NO_2$ | H | 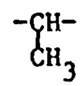 | 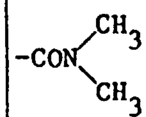 | |
| 89 | H | H | H | 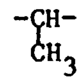 | $-CONH(CH_2)_3CH_3 \cdot H_2O$ | Smp. 73-76°C |
| 90 | H | H | H | 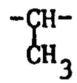 | 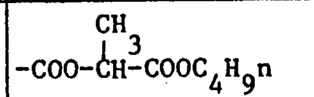 | |
| 91 | H | H | H | 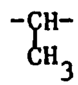 | 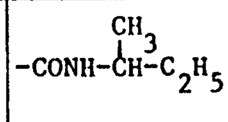 | |
| 92 | H | $NO_2$ | H | 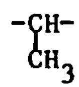 | $-CONH_2$ | |
| 93 | H | H | H | 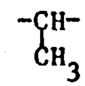 | 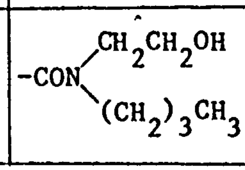 | |

Tabelle 1 (Fortsetzung)

| Verbindung No. | $R_1$ | $R_2$ | $R_3$ | X | Y |
|---|---|---|---|---|---|
| 94 | H | $NO_2$ | H | $-CH-$<br>$CH_3$ | $-COOCH_2CH_2OC_2H_5$ |
| 95 | H | H | H | $-CH-$<br>$CH_3$ | $-COS(CH_2)_3CH_3$ |
| 96 | H | H | H | $-CH-$<br>$CH_3$ | 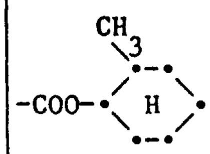 |
| 97 | H | H | H | $-CH-$<br>$CH_3$ | $CH_3$<br>$-CON$<br>$(CH_2)_3CH_3$ |
| 98 | Br | Cl | H | $-CH_2-$ | $-COOCH_3$ |
| 99 | H | H | H | $-CH-$<br>$CH_3$ | $-COOCH_2CH_2OC_4H_9n$ |
| 100 | Br | Cl | H | $-CH_2-$ | $-COOC_2H_5$ |
| 101 | H | H | H | $-CH-$<br>$CH_3$ | $-COOCH_2\hat{C}H_2Cl$ |

Tabelle 1 (Fortsetzung)

| Verbindung No. | $R_1$ | $R_2$ | $R_3$ | X | Y |
|---|---|---|---|---|---|
| 102 | H | H | H | $-\underset{CH_3}{CH}-$ | 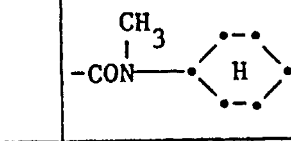 |
| 103 | H | H | H | $-\underset{CH_3}{CH}-$ | 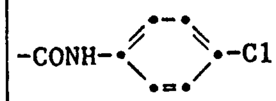 |
| 104 | Br | Cl | H | $-CH_2-$ | $-COOC_3H_7n$ |
| 105 | H | H | H | $-\underset{CH_3}{CH}-$ | 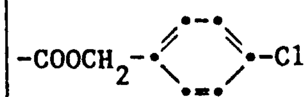 |
| 106 | Br | Cl | H | $-CH_2-$ | $-CONHCH_3$ |
| 107 | H | H | H | $-\underset{CH_3}{CH}-$ | 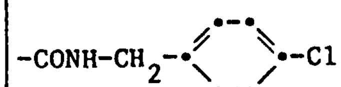 |
| 108 | H | H | H | $-\underset{CH_3}{CH}-$ | 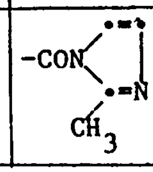 |

Tabelle 1 (Fortsetzung)

| Verbindung No. | $R_1$ | $R_2$ | $R_3$ | X | Y |
|---|---|---|---|---|---|
| 109 | H | H | H | $-\underset{CH_3}{CH}-$ | 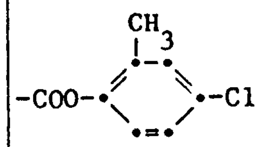 |
| 110 | H | H | H | $-\underset{CH_3}{CH}-$ | $-COOCH_2\overset{CH_3}{CH}CH_2CH_2CH_3$ |
| 111 | H | H | H | $-\underset{CH_3}{CH}-$ | 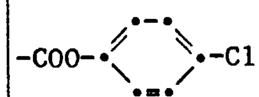 |
| 112 | H | H | H | $-\underset{CH_3}{CH}-$ | $-COO(CH_2)_3Cl$ |
| 113 | H | H | H | $-\underset{CH_3}{CH}-$ | $-CONH(CH_2)_3Cl$ |
| 114 | H | H | H | $-\underset{CH_3}{CH}-$ | $-COOCH_2-\overset{NO_2}{CH}-CH_3$ |

Tabelle 1 (Fortsetzung)

| Verbindung No. | $R_1$ | $R_2$ | $R_3$ | X | Y |
|---|---|---|---|---|---|
| 115 | H | H | H | $-CH-$<br>$CH_3$ | 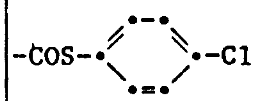 |
| 116 | Br | Cl | H | $-CH_2-$ | 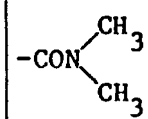 |
| 117 | H | H | H | $-CH-$<br>$CH_3$ | $-COOCH_2-CH=CH-CH_3$ |
| 118 | H | H | H | $-CH-$<br>$CH_3$ | 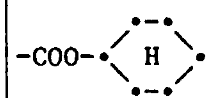 |
| 119 | H | H | H | $-CH-$<br>$CH_3$ | 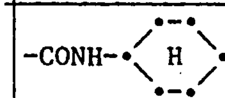 |
| 120 | H | H | H | $-CH-$<br>$CH_3$ | 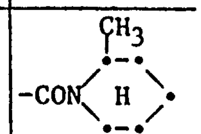 |
| 121 | H | H | H | $-CH-$<br>$CH_3$ | $-COO(CH_2)_{10}OH$ |

Tabelle 1 (Fortsetzung)

| Verbindung No. | $R_1$ | $R_2$ | $R_3$ | X | Y |
|---|---|---|---|---|---|
| 122 | H | H | H | $-CH(CH_3)-$ | $-COO(CH_2)_9CH_3$ |
| 123 | H | H | H | $-CH(CH_3)-$ | $-CONH(CH_2)_9CH_3$ |
| 124 | H | H | H | $-CH(CH_3)-$ | $-CON(CH_2CH_2OH)(CH_2CH_2OH)$ |
| 125 | H | H | H | $-CH(CH_3)-$ | $-CON(C_2H_5)(C_2H_5)$ |
| 126 | Br | Cl | H | $-CH_2-$ | $-CONH_2$ |
| 127 | H | H | H | $-CH(CH_3)-$ | $-COOCH_2CH_2N(C_2H_5)(C_2H_5)$ |
| 128 | H | H | H | $-CH(CH_3)-$ | $-CONHCH_2CH_2N(C_2H_5)(C_2H_5)$ |
| 129 | H | H | H | $-CH(CH_3)-$ | $-COOCH_2CH_2OCH_2CH_2OC_2H_5$ |

Tabelle 1 (Fortsetzung)

| Verbindung No. | $R_1$ | $R_2$ | $R_3$ | X | Y | Physikal. Konstante |
|---|---|---|---|---|---|---|
| 130 | H | H | H | $-CH-$<br>$CH_3$ | 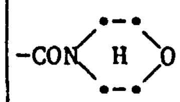 | Smp. 120-121°C |
| 131 | H | H | H | $-CH-$<br>$CH_3$ | 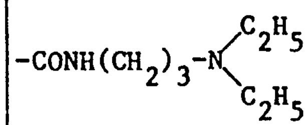 | |
| 132 | H | H | H | $-CH-$<br>$CH_3$ | $-COOCH_2CH_2OCH_2CH_2OC_4H_9n$ | |
| 133 | H | H | H | $-CH-$<br>$CH_3$ | $-COOCH_2CH_2OCH_2CH_2OCH_3$ | |
| 134 | H | H | H | $-CH-$<br>$CH_3$ | $-COOCH_2CH_2OCH_2CH_2OH$ | |
| 135 | H | H | H | $-CH-$<br>$CH_3$ | 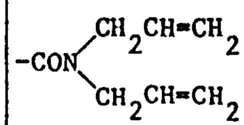 | |
| 136 | H | H | H | $-CH-$<br>$CH_3$ | 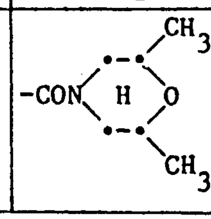 | |

Tabelle 1 (Fortsetzung)

| Verbindung No. | $R_1$ | $R_2$ | $R_3$ | X | Y |
|---|---|---|---|---|---|
| 137 | H | H | H | $-\underset{CH_3}{CH}-$ | $-CONH(CH_2)_6NH_2$ |
| 138 | H | H | H | $-\underset{CH_3}{CH}-$ | $-COOCH_2\underset{Br}{CH}-CH_2Br$ |
| 139 | H | H | H | $-\underset{CH_3}{CH}-$ | $C_4H_9n$<br>$-CON$<br>$C_4H_9n$ |
| 140 | H | H | H | $-\underset{CH_3}{CH}-$ | -COO- 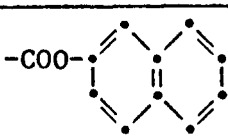 |
| 141 | H | H | H | $-\underset{CH_3}{CH}-$ | $-CONH(CH_2)_8NH_2$ |
| 142 | H | H | H | $-\underset{CH_3}{CH}-$ | $-COOCH_2CH_2N(CH_2CH_2CH_2CH_3)_2$ |
| 143 | H | H | H | $-\underset{CH_3}{CH}-$ | $-CONHCH_2CH_2N(C_4H_9n)_2$ |
| 144 | H | H | H | $-\underset{CH_3}{CH}-$ | $-CONH(CH_2)_3NH_2$ |

Tabelle 1 (Fortsetzung)

| Verbindung No. | $R_1$ | $R_2$ | $R_3$ | X | Y |
|---|---|---|---|---|---|
| 145 | H | H | H | $-\underset{CH_3}{\underset{\vert}{CH}}-$ | 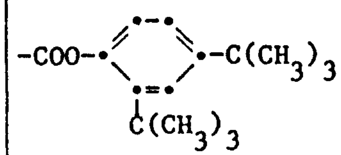 |
| 146 | H | H | H | $-\underset{CH_3}{\underset{\vert}{CH}}-$ | $-COO(CH_2)_{17}CH_3$ |
| 147 | H | H | H | $-\underset{CH_3}{\underset{\vert}{CH}}-$ | $-COOCH(CH_2Cl)_2$ |
| 148 | H | H | H | $-\underset{CH_3}{\underset{\vert}{CH}}-$ | 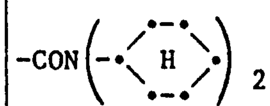 |
| 149 | H | H | H | $-\underset{CH_3}{\underset{\vert}{CH}}-$ | 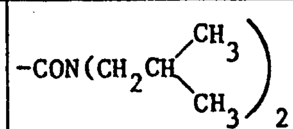 |
| 150 | H | H | H | $-\underset{CH_3}{\underset{\vert}{CH}}-$ | $-COOCH_2CH_2NO_2$ |

Tabelle 1 (Fortsetzung)

| Verbindung No. | $R_1$ | $R_2$ | $R_3$ | X | Y | Physikal. Konstante |
|---|---|---|---|---|---|---|
| 151 | H | H | H | 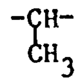 | 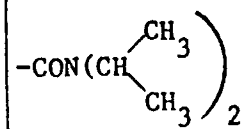 | |
| 152 | H | H | H | 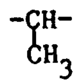 | 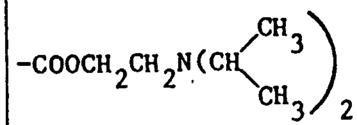 | |
| 153 | H | H | H | 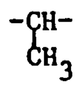 | 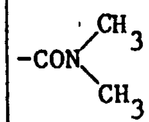 | Smp. 105-111°C |
| 154 | H | H | H | 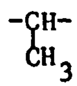 | 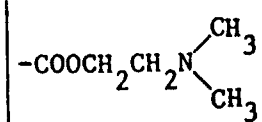 | |
| 155 | H | H | H | 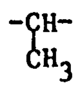 | 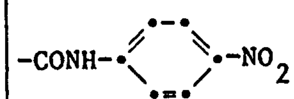 | |
| 156 | H | H | H | 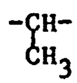 | 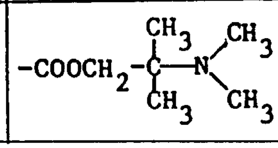 | |

Tabelle 1 (Fortsetzung)

| Verbindung No. | $R_1$ | $R_2$ | $R_3$ | X | Y |
|---|---|---|---|---|---|
| 157 | H | H | H | 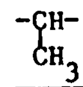 | 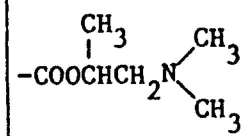 |
| 158 | H | H | H | 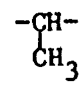 | 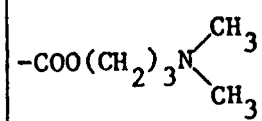 |
| 159 | H | H | H | 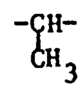 | 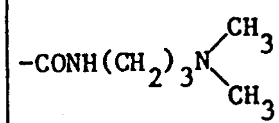 |
| 160 | H | H | H | 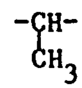 | 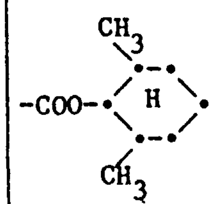 |

Tabelle 1 (Fortsetzung)

| Verbindung No. | $R_1$ | $R_2$ | $R_3$ | X | Y |
|---|---|---|---|---|---|
| 161 | H | H | H | 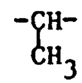 | 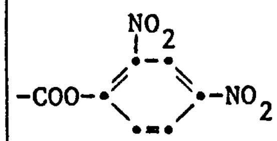 |
| 162 | H | H | H | 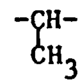 | $-CON(C_5H_{11}n)_2$ |
| 163 | H | H | H | 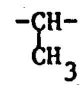 | 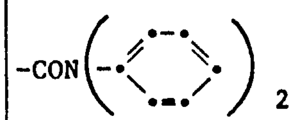 |
| 164 | H | H | H | 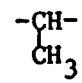 | $-CON(C_3H_7n)_2$ |
| 165 | H | H | H | 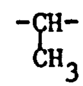 | $-COO(CH_2)_{11}CH_3$ |
| 166 | H | H | H | 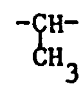 | $-CONH(CH_2)_{11}CH_3$ |

Tabelle 1 (Fortsetzung)

| Verbindung No. | $R_1$ | $R_2$ | $R_3$ | X | Y |
|---|---|---|---|---|---|
| 167 | H | H | H | $-CH-$<br>$CH_3$ | $-COOCH_2-$ 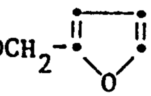 |
| 168 | H | H | H | $-CH-$<br>$CH_3$ | $-COS(CH_2)_{11}CH_3$ |
| 169 | H | H | H | $-CH-$<br>$CH_3$ | $-CONH-CH_2-$ 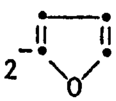 |
| 170 | H | H | H | $-CH-$<br>$CH_3$ | $-COOCH_2CH-CH_2OH$<br>$OH$ |
| 171 | Br | Cl | H | $-CH-$<br>$CH_3$ | $-COOCH_3$ |
| 172 | H | H | H | $-CH-$<br>$CH_3$ | $-COO(CH_2)_6CH_3$ |
| 173 | H | H | H | $-CH-$<br>$CH_3$ | $-COO(CH_2)_{15}CH_3$ |
| 174 | H | H | H | $-CH-$<br>$CH_3$ | $-COO(CH_2)_6OH$ |

Tabelle 1 (Fortsetzung)

| Verbindung No. | $R_1$ | $R_2$ | $R_3$ | X | Y |
|---|---|---|---|---|---|
| 175 | Br | Cl | H | $-\underset{CH_3}{CH}-$ | $-COOC_2H_5$ |
| 176 | H | H | H | $-\underset{CH_3}{CH}-$ | $-COO\overset{CH_3}{CH}(CH_2)_2\overset{CH_3}{CH}OH$ |
| 177 | H | H | H | $-\underset{CH_3}{CH}-$ | $-COO(CH_2)_5CH_3$ |
| 178 | H | H | H | $-\underset{CH_3}{CH}-$ | $-CONH(CH_2)_5CH_3$ |
| 179 | H | H | H | $-\underset{CH_3}{CH}-$ | $-COO-$ 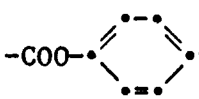 $-OCH_3$ |
| 180 | H | H | H | $-\underset{CH_3}{CH}-$ | $-CONHCH_2CH_2NHCH_2CH_2OH$ |
| 181 | Br | Cl | H | $-\underset{CH_3}{CH}-$ | $-COOC_3H_7iso$ |

Tabelle 1 (Fortsetzung)

| Verbindung No. | $R_1$ | $R_2$ | $R_3$ | X | Y |
|---|---|---|---|---|---|
| 182 | H | H | H | -CH-<br>$CH_3$ | $-COOCH_2CH_2$ 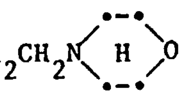 |
| 183 | Br | Cl | H | -CH-<br>$CH_3$ | $-CONHCH_3$ |
| 184 | Br | Cl | H | -CH-<br>$CH_3$ | $-CONH_2$ |
| 185 | Br | Cl | H | -CH-<br>$CH_3$ | 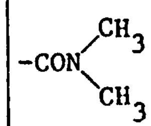 |
| 186 | H | H | H | -CH-<br>$CH_3$ | $-COOCH_2CH_2$ 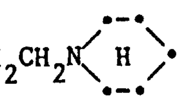 |
| 187 | H | H | H | -CH-<br>$CH_3$ | $-COOCH_2CH_2-$ 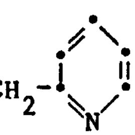 |

Tabelle 1 (Fortsetzung)

| Verbindung No. | $R_1$ | $R_2$ | $R_3$ | X | Y |
|---|---|---|---|---|---|
| 188 | H | H | H | $-CH(CH_3)-$ | $-COOCH_2CN$ |
| 189 | H | H | H | $-CH(CH_3)-$ | $-CONH(CH_2)_3COOCH_3$ |
| 190 | H | H | H | $-CH(CH_3)-$ | $-COO-C(CH_3)_2-COOC_2H_5$ |
| 191 | Cl | Cl | H | $-CH_2-$ | $-COOH$ |
| 192 | H | H | H | $-CH(CH_3)-$ | $-COOCH_2-$ 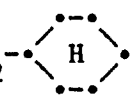 |
| 193 | H | H | H | $-CH(CH_3)-$ | $-COOCH_2-$ 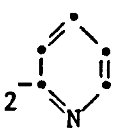 |
| 194 | H | H | H | $-CH(CH_3)-$ | $-COOCH_2\hat{C}H_2CN$ |

Tabelle 1 (Fortsetzung)

| Verbindung No. | $R_1$ | $R_2$ | $R_3$ | X | Y |
|---|---|---|---|---|---|
| 195 | Cl | Cl | H | $-CH_2-$ | $-COOCH_2CH_2OCH_3$ |
| 196 | H | H | H | $-CH-$<br>$CH_3$ | $-COOCH_2-$ 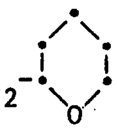 |
| 197 | H | H | H | $-CH-$<br>$CH_3$ | $-COO-$ 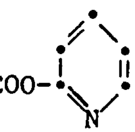 |
| 198 | H | H | H | $-CH-$<br>$CH_3$ | $-CON$ 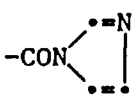 |
| 199 | H | H | H | $-CH-$<br>$CH_3$ | 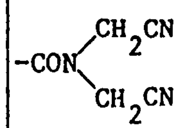 |
| 200 | H | H | H | $-CH-$<br>$CH_3$ | $-CONH(CH_2)_{17}CH_3$ |
| 201 | Cl | Cl | H | $-CH_2-$ | $-COOCH_3$ |

Tabelle 1 (Fortsetzung)

| Verbindung No. | $R_1$ | $R_2$ | $R_3$ | X | Y |
|---|---|---|---|---|---|
| 202 | H | H | H | $-CH(CH_3)-$ | $-COO(CH_2)_7CH_3$ |
| 203 | H | H | H | $-CH(CH_3)-$ | -COO- 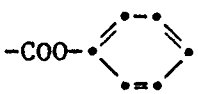 |
| 204 | H | H | H | $-CH(CH_3)-$ | $-COOCH_2CH_2-$ 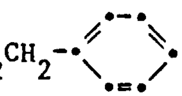 |
| 205 | Cl | Cl | H | $-CH_2-$ | $-COOC_2H_5$ |
| 206 | H | H | H | $-CH(CH_3)-$ | $-CONH(CH_2)_7CH_3$ |
| 207 | H | H | H | $-CH(CH_3)-$ | 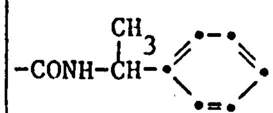 |
| 208 | H | H | H | $-CH(CH_3)-$ | $-COO(CH_2)_4CH_3$ |

Tabelle 1 (Fortsetzung)

| Verbindung No. | $R_1$ | $R_2$ | $R_3$ | X | Y |
|---|---|---|---|---|---|
| 209 | H | H | H | $-CH-$<br>$CH_3$ | $-CONHCH_2CH_2-$ 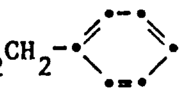 |
| 210 | H | H | H | $-CH-$<br>$CH_3$ | $-COOCH_2CH_2CH_2-$ 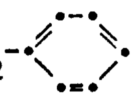 |
| 211 | Cl | Cl | H | $-CH_2-$ | $COOC_3H_7$iso |
| 212 | H | H | H | $-CH-$<br>$CH_3$ | -CON 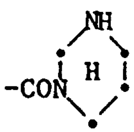 |
| 213 | Cl | Cl | H | $-CH_2-$ | $-CONH_2$ |
| 214 | H | H | H | $-CH-$<br>$CH_3$ | -CON 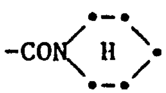 |
| 215 | Cl | Cl | H | $-CH-$<br>$CH_3$ | -COOH . |

Tabelle 1 (Fortsetzung)

| Verbindung No. | $R_1$ | $R_2$ | $R_3$ | X | Y |
|---|---|---|---|---|---|
| 216 | H | H | H | $-CH-$<br>$CH_3$ | $-CONHCH_2CH_2-$ 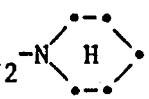 |
| 217 | H | H | H | $-CH-$<br>$CH_3$ | $-COOCH_2-C\equiv CH$ |
| 218 | H | H | H | $-CH-$<br>$CH_3$ | $-COOCH_2CH_2(OCH_2CH_2)_3OH$ |
| 219 | Cl | Cl | H | $-CH-$<br>$CH_3$ | $-COOCH_2CH_2OCH_3$ |
| 220 | Cl | Cl | H | $-CH-$<br>$CH_3$ | $-COOC_2H_5$ |
| 221 | H | H | H | $-CH-$<br>$CH_3$ | $-COSCH_2COOCH_3$ |
| 222 | Cl | Cl | H | $-CH-$<br>$CH_3$ | $-COOC_3H_7n$ |

Tabelle 1 (Fortsetzung)

| Verbindung No. | $R_1$ | $R_2$ | $R_3$ | X | Y |
|---|---|---|---|---|---|
| 223 | H | H | H | 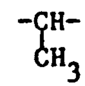 | 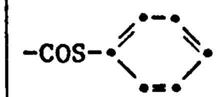 |
| 224 | Cl | Cl | H | 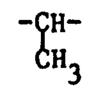 | $-COOC_3H_7$iso |
| 225 | H | H | H | 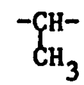 | 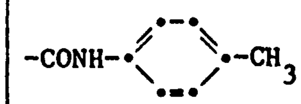 |
| 226 | H | H | H | 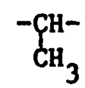 | $-COOCH_2CCl_3$ |
| 227 | H | H | H | 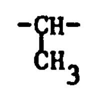 | 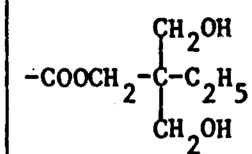 |
| 228 | H | H | H | 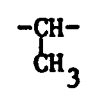 | 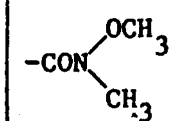 |
| 229 | Cl | Cl | H | 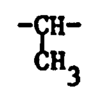 | 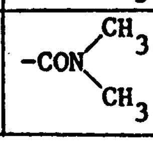 |

Tabelle 1 (Fortsetzung)

| Verbindung No. | $R_1$ | $R_2$ | $R_3$ | X | Y |
|---|---|---|---|---|---|
| 230 | H | J | H | $-\underset{CH_3}{CH}-$ | $-COOH$ |
| 231 | H | Br | $CH_3$ | $-\underset{CH_3}{CH}-$ | $-COOH$ |
| 232 | Cl | Cl | H | $-\underset{CH_3}{CH}-$ | $-CONHC_2H_5$ |
| 233 | H | J | H | $-\underset{CH_3}{CH}-$ | $-COOCH_2CH_2OCH_3$ |
| 234 | H | Br | $CH_3$ | $-\underset{CH_3}{CH}-$ | $-COOCH_2CH_2OCH_3$ |
| 235 | Cl | Cl | H | $-\underset{CH_3}{CH}-$ | $-CONH_2$ |
| 236 | H | J | H | $-\underset{CH_3}{CH}-$ | $-COOCH_3$ |
| 237 | H | Br | $CH_3$ | $-\underset{CH_3}{CH}-$ | $-COOCH_3$ |
| 238 | H | J | H | $-\underset{CH_3}{CH}$ | $-COOC_2H_5$ |

Tabelle 1 (Fortsetzung)

| Verbindung No. | $R_1$ | $R_2$ | $R_3$ | X | Y |
|---|---|---|---|---|---|
| 239 | H | J | H | $-CH(CH_3)-$ | $-CONHCH_3$ |
| 240 | H | Br | $CH_3$ | $-CH(CH_3)-$ | $-CON(CH_3)_2$ |
| 241 | H | Br | H | $-CH(CH_3)-$ | $-COOH$ |
| 242 | H | Br | H | $-CH(CH_3)-$ | $-COOCH_2CH_2OCH_3$ |
| 243 | H | J | H | $-CH(CH_3)-$ | $-CON(CH_3)_2$ |
| 244 | H | Br | H | $-CH(CH_3)-$ | $-COOCH_3$ |
| 245 | H | Cl | $CH_3$ | $-CH_2-$ | $-COOCH_3$ |
| 246 | H | Br | H | $-CH(CH_3)-$ | $-CONH_2$ |
| 247 | H | Cl | $CH_3$ | $-CH_2-$ | $-COOC_2H_5$ |

<u>Tabelle 1</u> (Fortsetzung)

| Verbindung No. | $R_1$ | $R_2$ | $R_3$ | X | Y |
|---|---|---|---|---|---|
| 248 | H | Br | H | $-\underset{CH_3}{\underset{\vert}{CH}}-$ | $-CONHC_2H_5$ |
| 249 | H | Cl | $CH_3$ | $-CH_2-$ | $-CONHCH_3$ |
| 250 | H | Cl | $CH_3$ | $-CH_2-$ | $-CON\begin{smallmatrix}CH_3\\CH_3\end{smallmatrix}$ |
| 251 | H | Cl | $CH_3$ | $-\underset{CH_3}{\underset{\vert}{CH}}-$ | $-COOH$ |
| 252 | H | Cl | $CH_3$ | $-\underset{CH_3}{\underset{\vert}{CH}}-$ | $-COOCH_3$ |

Tabelle 1 (Fortsetzung)

| Verbindung No. | $R_1$ | $R_2$ | $R_3$ | X | Y |
|---|---|---|---|---|---|
| 253 | H | Cl | $CH_3$ | 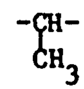 | $-COOC_2H_5$ |
| 254 | H | Cl | $CH_3$ | 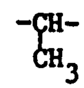 | $-COOC_3H_7$iso |
| 255 | H | Cl | $CH_3$ | 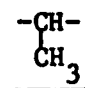 | $-CONHCH_3$ |
| 256 | H | Cl | $CH_3$ | 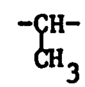 | 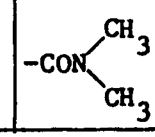 |

Tabelle 1 (Fortsetzung)

| Verbindung No. | $R_1$ | $R_2$ | $R_3$ | X | Y | Physikal. Konstante |
|---|---|---|---|---|---|---|
| 257 | H | Cl | H | $-\underset{\mid \atop CH_3}{CH}-$ | $-COOH$ | |
| 258 | H | Cl | H | $-\underset{\mid \atop CH_3}{CH}-$ | $-COOCH_2CH_2OCH_3$ | |

Tabelle 1 (Fortsetzung)

| Verbindung No. | $R_1$ | $R_2$ | $R_3$ | X | Y | Physikal. Konstante |
|---|---|---|---|---|---|---|
| 259 | H | Cl | H | $-CH(CH_3)-$ | $-COOCH_3$ | |
| 260 | H | Cl | H | $-CH(CH_3)-$ | $-COOC_2H_5$ | |
| 261 | H | Cl | H | $-CH_2-$ | $-COOH$ | Smp. 232-233°C |
| 262 | H | Cl | H | $-CH(CH_3)-$ | $-COOC_3H_7n$ | |
| 263 | H | Cl | H | $-CH_2-$ | $-COOCH_2CH_2OCH_3$ | Smp. 97-98°C |
| 264 | H | Cl | H | $-CH(CH_3)-$ | $-CONHCH_3$ | |
| 265 | H | Cl | H | $-CH_2-$ | $-COOCH_3$ | Smp. 104-105,5°C |

Tabelle 1 (Fortsetzung)

| Verbindung No. | $R_1$ | $R_2$ | $R_3$ | X | Y | Physikal. Konstante |
|---|---|---|---|---|---|---|
| 266 | H | Cl | H | 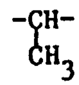 | 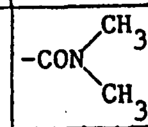 | |
| 267 | H | Cl | H | $-CH_2-$ | $-COOC_2H_5$ | Smp. 116-117°C |
| 268 | H | Cl | H | 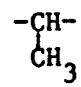 | $-CONHC_2H_5$ | |
| 269 | H | Cl | H | $-CH_2-$ | $-COOC_3H_7n$ | Smp. 108-109°C |
| 270 | H | Cl | H | 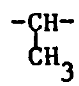 | 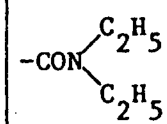 | |
| 271 | H | Cl | H | $-CH_2-$ | $-CONHCH_3$ | |
| 272 | H | Cl | H | 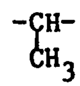 | $-CONH_2$ | |
| 273 | H | Cl | H | $-CH_2-$ | 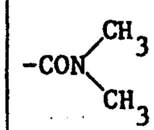 | Smp. 135-136°C |
| 274 | H | Cl | H | 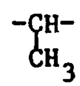 | $-COOCH_2CH_2OC_2H_5$ | |

Tabelle 1 (Fortsetzung)

| Verbindung No. | $R_1$ | $R_2$ | $R_3$ | X | Y | Physikal. Konstante |
|---|---|---|---|---|---|---|
| 275 | H | Cl | H | $-CH_2-$ | $-CONH_2$ | |
| 276 | H | Cl | H | 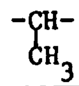 | $-CONH(CH_2)_3CH_3$ | |
| 277 | H | Cl | H | 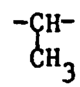 | 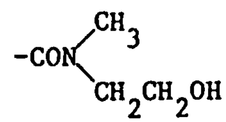 | |
| 278 | H | H | $CH_3$ | $-CH_2-$ | $-COOCH_3$ | Smp. 58-66°C |
| 279 | H | H | $CH_3$ | 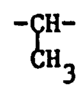 | $-COOH$ | |
| 280 | H | Cl | H | 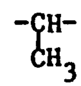 | 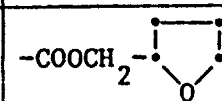 | |
| 281 | H | H | $CH_3$ | $-CH_2-$ | 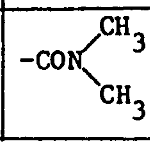 | |

Tabelle 1 (Fortsetzung)

| Verbindung No. | $R_1$ | $R_2$ | $R_3$ | X | Y |
|---|---|---|---|---|---|
| 282 | H | H | $CH_3$ | 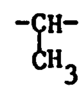 | $-COOCH_3$ |
| 283 | H | H | $CH_3$ | 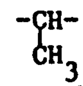 | $-COOC_3H_7$iso |
| 284 | H | H | $CH_3$ | 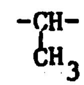 | 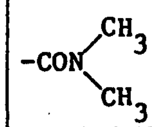 |
| 285 | H | H | $CH_3$ | 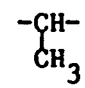 | $-CONHCH_3$ |
| 286 | H | H | $CH_3$ | 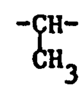 | 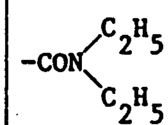 |
| 287 | H | H | $CH_3$ | 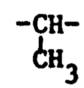 | $-CONH_2$ |

Tabelle 1 (Fortsetzung)

| Verbindung No. | $R_1$ | $R_2$ | $R_3$ | X | Y |
|---|---|---|---|---|---|
| 288 | H | H | H | $-CH_2-$ | 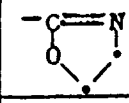 |
| 289 | H | Cl | H | $-CH-$<br>$CH_3$ | " |
| 290 | Br | Cl | H | $-CH_2-$ | " |
| 291 | H | H | $CH_3$ | $-CH_2-$ | " |
| 292 | Cl | Cl | $CH_3$ | $-CH_2-$ | " |
| 293 | Br | Cl | H | $-CH-$<br>$CH_3$ | " |
| 294 | H | Cl | H | $-CH_2-$ | " |
| 295 | H | H | H | $-CH-$<br>$CH_3$ | " |

Tabelle 1 (Fortsetzung)

| Verbindung No. | $R_1$ | $R_2$ | $R_3$ | X | Y |
|---|---|---|---|---|---|
| 296 | H | H | $CH_3$ | $-CH-$<br>$CH_3$ | 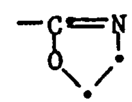 |
| 297 | H | Br | H | $-CH_2-$ | " |
| 298 | Cl | Cl | $CH_3$ | $-CH-$<br>$CH_3$ | " |
| 299 | H | $C_2H_5$ | H | $-CH_2-$ | " |
| 300 | H | Br | H | $-CH-$<br>$CH_3$ | " |
| 301 | Cl | Cl | H | $-CH_2-$ | " |
| 302 | J | Cl | H | $-CH_2-$ | " |

Tabelle 1 (Fortsetzung)

| Verbindung No. | $R_1$ | $R_2$ | $R_3$ | X | Y |
|---|---|---|---|---|---|
| 303 | H | $C_2H_5$ | H | -CH-<br>$CH_3$ | " |
| 304 | Cl | Cl | H | -CH-<br>$CH_3$ | " |
| 305 | J | Cl | H | -CH-<br>$CH_3$ | " |

Tabelle 1 (Fortsetzung)

| Verbindung No. | $R_1$ | $R_2$ | $R_3$ | X + Y | Physikalische Konstante °C |
|---|---|---|---|---|---|
| 306 | H | H | H | 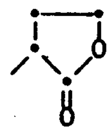 | |
| 307 | H | Cl | H | " | 140-141.5°C |
| 308 | Cl | Cl | H | " | |
| 309 | H | H | $CH_3$ | " | |
| 310 | H | Br | H | " | |
| 311 | Br | Cl | H | " | |
| 312 | H | Br | $CH_3$ | " | |
| 313 | H | Cl | $CH_3$ | " | |
| 314 | H | J | H | " | |
| 315 | J | Cl | H | " | |
| 316 | Cl | Cl | $CH_3$ | " | |
| 317 | H | $NO_2$ | H | " | |
| 318 | H | $C_2H_5$ | H | " | |
| 319 | Cl | $CH_3$ | H | " | |

Tabelle 1 (Fortsetzung)

| Verbindung No. | $R_1$ | $R_2$ | $R_3$ | X | Y | Physikal. Konstante |
|---|---|---|---|---|---|---|
| 320 | H | H | $CH_3$ | $-CH_2-$ | $-COOC_2H_5$ | $n_D^{22,5} = 1{,}5762$ |
| 321 | H | Cl | H | $-CH_2-$ | $-COOC_4H_9$tert. | Smp. 63-69°C |
| 322 | H | H | H | $-CH_2-$ | $-COOC_4H_9$tert. | Smp. 68-70°C |
| 323 | H | Cl | H | $-CH_2-$ | $-COOCH_2-C{\equiv}CH$ | Smp. 115-116°C |
| 324 | H | Cl | H | $-CH_2-$ | $-COOC_3H_7$iso | Smp. 147-148°C |
| 325 | H | Cl | H | $-CH_2-$ | $-COOCH_2CH_2OC_2H_5$ | Smp. 102-104°C |
| 326 | H | Cl | H | $-CH_2-$ | $-COOCH_2-$ 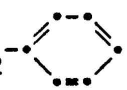 | Smp. 110-112°C |
| 327 | H | Cl | H | $-CH_2-$ | $-COOCH_2-CH{=}CH_2$ | Smp. 98-99°C |
| 328 | H | Cl | H | $-CH_2-$ | $-COO(CH_2)_{11}CH_3$ | Smp. 76-77°C |
| 329 | H | Cl | H | $-CH_2-$ | $-COOC_4H_9$sek. | Smp. 110-111°C |
| 330 | H | H | H | $-CH_2-$ | $-COO(CH_2)_7CH_3$ | $n_D^{24} = 1{,}5419$ |

**Tabelle 1** (Fortsetzung)

| Verbindung No. | $R_1$ | $R_2$ | $R_3$ | X | Y | Physikal. Konstante |
|---|---|---|---|---|---|---|
| 331 | H | Cl | H | $-CH_2-$ | $-COOC_4H_9n$ | Smp. 90,5-92°C |
| 332 | H | H | H | $CH_3$<br>-C-<br>$CH_3$ | $-COOC_2H_5$ | $n_D^{24}$ = 1,5600 |
| 333 | H | H | H | $-CH_2-$ | $-COO(CH_2)_{11}CH_3$ | $n_D^{23}$ = 1,5232 |
| 334 | H | H | H | $-CH_2-$ | $-COOCH_2-CH=CH_2$ | $n_D^{23}$ = 1,5885 |
| 335 | H | Cl | H | $CH_3$<br>-C-<br>$CH_3$ | $-COOC_2H_5$ | Smp. 57-58°C |
| 336 | H | Cl | H | $-CH_2-$ | $-COO(CH_2)_7CH_3$ | Smp. 87-88°C |
| 337 | H | H | H | $-CH_2-$ | $-COOC_4H_9n$ | $n_D^{22}$ = 1,5642 |
| 338 | H | H | H | $-CH_2-$ | $-COOC_4H_9$sek. | Oel (rot) |

Tabelle 1 (Fortsetzung)

| Verbindung No. | $R_1$ | $R_2$ | $R_3$ | X | Y | Physikal. Konstante |
|---|---|---|---|---|---|---|
| 339 | H | Cl | H | $-CH_2-$ | $-COOCH_2CH_2Cl$ | Smp. 125-126°C |
| 340 | H | H | H | $-CH_2-$ | $-COOCH_2-$ 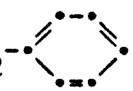 | $n_D^{23,5}$ = 1,6099 |
| 341 | H | Cl | H | $-CH_2-$ | $-COOCH_2-$ 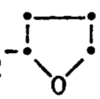 | Smp. 101-103°C |
| 342 | H | Cl | H | $-CH_2-$ | $-COS(CH_2)_7CH_3$ | Smp. 53-54°C |
| 343 | H | H | H | $-CH_2-$ | $-COOCH_2CH_2Cl$ | Smp. 109-110°C |
| 344 | J | Cl | H | $-CH_2-$ | $-COOC_4H_9$tert. | Smp. 81-97°C |
| 345 | J | Cl | H | $-CH_2-$ | $-COOC_2H_5$ | Smp. 92-94°C |
| 346 | J | Cl | H | $-CH_2-$ | $-COO(CH_2)_{11}CH_3$ | Smp. 51-53°C |
| 347 | J | Cl | H | $-CH_2-$ | $-COOCH_3$ | Smp. 121-126°C |
| 348 | J | Cl | H | $-CH_2-$ | $-COOCH_2CH_2Cl$ | Smp. 44-45°C |

Tabelle 1 (Fortsetzung)

| Verbindung No. | $R_1$ | $R_2$ | $R_3$ | X | Y | Physikal. Konstante |
|---|---|---|---|---|---|---|
| 349 | J | Cl | H | $-CH_2-$ | $-COOCH_2-$ 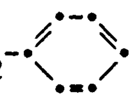 | Smp. 112-113°C |
| 350 | J | Cl | H | $-CH_2$ | $-COOC_3H_7n$ | Smp. 71-73°C |
| 351 | H | H | $CH_3$ | $CH_3$<br>$-C-$<br>$CH_3$ | $-COOC_2H_5$ | Smp. 47-53°C |
| 352 | H | H | H | $-CH_2-$ | $-COOC_4H_9iso$ | $n_D^{22} = 1,5632$ |
| 353 | H | H | H | $-CH_2-$ | $-COOCHCH_2CH_2CH_3$<br>$CH_3$ | $n_D^{22} = 1,5391$ |
| 354 | H | H | H | $-CH_2-$ | $-COOCH(CH_2)_5CH_3$<br>$CH_3$ | $n_D^{22} = 1,5342$ |
| 355 | H | H | H | $-CH_2-$ | $-CONH(CH_2)_{11}CH_3$ | Smp. 56-61°C |

Tabelle 1 (Fortsetzung)

| Verbindung No. | $R_1$ | $R_2$ | $R_3$ | X | Y | Physikal. Konstante |
|---|---|---|---|---|---|---|
| 356 | H | H | H | $-CH_2-$ | 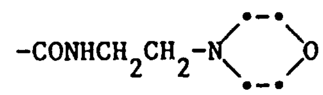 | Smp. 94-99°C |
| 357 | H | H | H | $-CH_2-$ | $-CONHCH_2CH_2CH_2OH$ | Smp. 138-139°C |
| 358 | H | H | H | $-CH_2-$ | 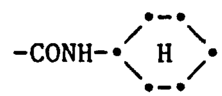 | Smp. 104-106°C |
| 359 | H | H | H | $-CH_2-$ | 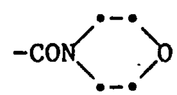 | Smp. 99-103°C |
| 360 | H | H | H | $-CH_2-$ | 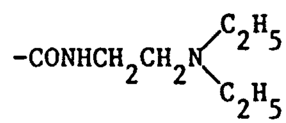 | $n_D^{23}$ = 1,5686 |
| 361 | H | H | H | $-CH_2-$ | 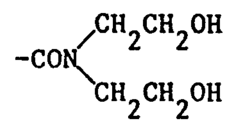 | Smp. 144-146°C |
| 362 | H | H | H | $-CH_2-$ | 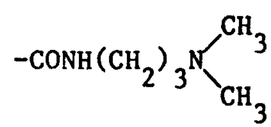 | $n_D^{23}$ = 1,5766 |

Tabelle 1 (Fortsetzung)

| Verbindung No. | $R_1$ | $R_2$ | $R_3$ | X | Y | Physikal. Konstante |
|---|---|---|---|---|---|---|
| 363 | H | H | H | $-CH_2-$ | 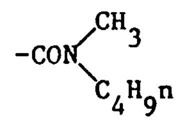 | $n_D^{22} = 1,5840$ |
| 364 | H | H | H | $-CH_2-$ | 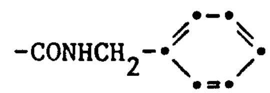 • $H_2O$ | Smp. 70,5-73,5°C |
| 365 | H | H | H | $-CH_2-$ | 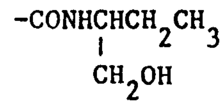 | Smp. 150-151°C |
| 366 | H | H | H | $-CH_2-$ | 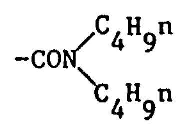 • $2H_2O$ | Smp. 105-106°C |
| 367 | H | H | H | $-CH_2-$ | 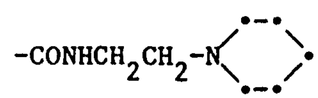 | $n_D^{26} = 1,5821$ |
| 368 | H | H | H | $-CH_2-$ | 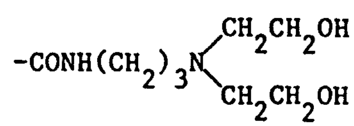 | Smp. 109-110°C |

Tabelle 1 (Fortsetzung)

| Verbindung No. | $R_1$ | $R_2$ | $R_3$ | X | Y | Physikal. Konstante |
|---|---|---|---|---|---|---|
| 369 | H | H | H | $-CH_2-$ | $-CONHCH_2-CH=CH_2 \cdot H_2O$ | Smp. 71-75°C |
| 370 | H | H | H | $-CH_2-$ | $-CONHCH_2-$ 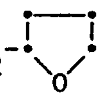 • $H_2O$ | Smp. 57-58°C |
| 371 | H | H | H | $-CH_2-$ | $-CONH(CH_2)_3OC_2H_5$ | Smp. 51-61°C |
| 372 | H | H | H | $-CH_2-$ | $-CONHCH_2CH_2NHCH_2CH_2OH$ | Smp. 70-91°C |
| 373 | H | Cl | H | $-CH_2-$ | $-CONH(CH_2)_3OC_2H_5$ | Smp. 85-88°C |
| 374 | H | Cl | H | $-CH_2-$ | 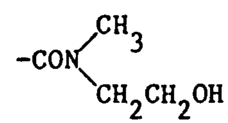 | Smp. 187-189°C |
| 375 | H | Cl | H | $-CH_2-$ | 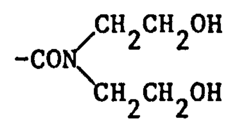 | Smp. 177-179°C |
| 376 | H | Cl | H | $-CH_2-$ | 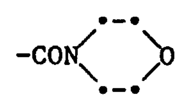 | Smp. 148-150°C |

Tabelle 1 (Fortsetzung)

| Verbindung No. | $R_1$ | $R_2$ | $R_3$ | X | Y | Physikal. Konstante |
|---|---|---|---|---|---|---|
| 377 | H | Cl | H | $-CH_2-$ | $-CONHCH_2CH_2CH_2OH$ | Smp. 157-160°C |
| 378 | H | Cl | H | $-CH_2-$ | $-CONHC_4H_9n \cdot H_2O$ | Smp. 87-90°C |
| 379 | H | Cl | H | $-CH_2-$ | $-CONHC_2H_5$ | Smp. 94-98°C |
| 380 | H | Cl | H | $-CH_2-$ | $-CONHCH_2-$ 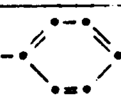 $\cdot \frac{1}{2}H_2O$ | Smp. 146-149°C |
| 381 | H | H | $CH_3$ | $-CH_2-$ | $-CONH_2$ | Smp. 193-196°C |
| 382 | H | H | H | $-CH_2-$ | $-CONHNH_2 \cdot H_2O$ | Smp. 121-124°C |
| 383 | H | H | H | $-CH_2-$ | $-COONa \cdot H_2O$ | Smp. 140-142°C |
| 384 | H | H | H | $-CH_2-$ | $-COOK \cdot H_2O$ | Smp. > 200°C |
| 385 | H | H | H | $-CH_2-$ | $-COO^{\ominus}\ \overset{\oplus}{H}N(CH_3)_3$ | Smp. 176-178 °C |

Tabelle 1 (Fortsetzung)

| Verbindung No. | $R_1$ | $R_2$ | $R_3$ | X | Y | Physikal. Konstante |
|---|---|---|---|---|---|---|
| 386 | H | H | H | $-CH_2-$ | $-COO^{\ominus}\ \overset{\oplus}{H}N(CH_2CH_2OH)_3$ | Smp. 97-98°C |
| 387 | H | Cl | H | $-CH_2-$ | $-COOK \cdot H_2O$ | Smp. > 260°C |
| 388 | H | Cl | H | $-CH_2-$ | $-COONa \cdot H_2O$ | Smp. > 260°C |
| 389 | H | H | H | $-CH_2-$ | $-COO^{\ominus}\ \overset{\oplus}{H}N(C_2H_5)_3$ | Smp. 255-257°C (Z) |
| 390 | H | Cl | H | $-CH_2-$ | $-COO^{\ominus}\ ^{\oplus}NH_4$ | Smp. 227-228°C (Z) |
| 391 | H | Cl | H | $-CH_2-$ | $-COO^{\ominus}\ \overset{\oplus}{H}N(CH_2CH_2OH)_3$ | Smp. 132-156°C (Z) |

Formulierungsbeispiele für flüssige Wirkstoffe der Formel I (% = Gewichtsprozent)

2. Emulsions-Konzentrate

| | a) | b) | c) |
|---|---|---|---|
| Wirkstoff aus Tabelle 1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

3. Lösungen

| | a) | b) | c) | d) |
|---|---|---|---|---|
| Wirkstoff aus Tabelle 1 | 80 % | 10 % | 5 % | 95 % |
| Aethylenglykol-monomethyl-äther | 20 % | - | - | - |
| Polyäthylenglykol M G 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190 °C) | - | - | 94 % | - |

Die Lösungen sind zur Anwendung in Form kleinster Tropfen geeignet.

4. Granulate

| | a) | b) |
|---|---|---|
| Wirkstoff aus Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

5. Stäubemittel

| | a) | b) |
|---|---|---|
| Wirkstoff aus Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

6. Spritzpulver

| | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

7. Emulsions-Konzentrat

| | |
|---|---|
| Wirkstoff aus Tabelle 1 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

8. Stäubemittel

| | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

9. Extruder Granulat

| | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

10. Umhüllungs-Granulat

| | |
|---|---|
| Wirkstoff aus Tabelle 1 | 3 % |
| Polyäthylenglykol (M G 200 ) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

11. Suspensions-Konzentrat

| | |
|---|---|
| Wirkstoff aus Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel 12: Versuch mit Antidot und Herbizid an Weizen

Weizensamen werden in Plastiktöpfe, die 0,5 l Gartenerde enthalten, im Gewächshaus ausgesät. Nach da Auflaufen der Pflanzen bis zum 2- bis 3-Blattstadium wird die als Antidot zu prüfende Substanz zusammen mit dem Herbizid 2-[4-(3,5-Dichlorpyridyl-2-oxy)-phenoxy]-propionsäure-2-propinylester als Tankmischung appliziert.

20 Tage nach der Applikation wird die Schutzwirkung des Antidots in Prozent bonitiert. Als Referenzen dienen dabei die mit dem Herbizid allein behandelten Pflanzen sowie die vollständig unbehandelte Kontrolle. Die Ergebnisse zeigen die folgenden Tabellen:

Tabelle 2

Aufwandmengen:

Herbizid 0,5 kg/ha

Antidot 0,5 kg/ha

| Verbindung Nr. | relative Schutzwirkung in Prozent |
|---|---|
| 1 | 50 |
| 3 | 50 |

Tabelle 3

Aufwandmengen:

Herbizid 0,75 kg/ha

Antidot 1,5 kg/ha

| Verbindung Nr. | relative Schutzwirkung in Prozent | Verbindung Nr. | relative Schutzwirkung in Prozent |
|---|---|---|---|
| 1 | 50 | 36 | 25 |
| 3 | 50 | 37 | 63 |
| 4 | 63 | 55 | 50 |
| 6 | 38 | 56 | 63 |
| 8 | 38 | 63 | 25 |
| 11 | 63 | 67 | 50 |
| 13 | 75 | 69 | 12,5 |
| 20 | 63 | 80 | 50 |
| 28 | 63 | 84 | 63 |

Fortsetzung Tabelle 3

| Verbindung Nr. | relative Schutzwirkung in Prozent | Verbindung Nr. | relative Schutzwirkung in Prozent |
|---|---|---|---|
| 89 | 50 | 339 | 63 |
| 130 | 75 | 340 | 63 |
| 153 | 12,5 | 341 | 63 |
| 261 | 50 | 342 | 50 |
| 263 | 63 | 343 | 63 |
| 265 | 50 | 345 | 75 |
| 267 | 63 | 346 | 75 |
| 269 | 50 | 347 | 63 |
| 273 | 63 | 348 | 63 |
| 278 | 12,5 | 349 | 63 |
| 307 | 25 | 350 | 63 |
| 320 | 12,5 | 355 | 38 |
| 321 | 75 | 356 | 12,5 |
| 322 | 63 | 357 | 25 |
| 323 | 75 | 358 | 12,5 |
| 324 | 65 | 359 | 25 |
| 325 | 25 | 360 | 38 |
| 326 | 38 | 361 | 50 |
| 327 | 25 | 362 | 25 |
| 328 | 25 | 363 | 38 |
| 329 | 12,5 | 364 | 63 |
| 330 | 50 | 365 | 12,5 |
| 331 | 50 | 366 | 50 |
| 332 | 63 | 367 | 12,5 |
| 333 | 63 | 368 | 12,5 |
| 334 | 63 | 369 | 25 |
| 335 | 63 | 371 | 50 |
| 336 | 63 | 372 | 63 |
| 337 | 63 | 373 | 50 |
| 338 | 75 | 374 | 65 |

Fortsetzung Tabelle 3

| Verbindung Nr. | relative Schutzwirkung in Prozent | Verbindung Nr. | relative Schutzwirkung in Prozent |
|---|---|---|---|
| 375 | 50 | 384 | 63 |
| 376 | 50 | 385 | 63 |
| 377 | 63 | 386 | 63 |
| 378 | 75 | 387 | 63 |
| 379 | 25 | 388 | 50 |
| 380 | 63 | 389 | 63 |
| 382 | 25 | 390 | 75 |
| 383 | 63 | 391 | 75 |

Beispiel 13: Versuch mit Antidot und Herbizid an Gerste

Gerstensamen werden in Plastiktöpfe, die 0,5 l Gartenerde enthalten, im Gewächshaus ausgesät. Nach dem Auflaufen der Pflanzen bis zum 2- bis 3-Blattstadium wird die als Antidot zu prüfende Substanz zusammen mit dem Herbizid 2-[4-(3,5-Dichlorpyridyl-2-oxy)-phenoxy]-propionsäure-2-propinylester als Tankmischung appliziert.

20 Tage nach der Applikation wird die Schutzwirkung des Antidots in Prozent bonitiert. Als Referenzen dienen dabei die mit dem Herbizid allein behandelten Pflanzen sowie die vollständig unbehandelte Kontrolle. Die Ergebnisse zeigen die folgenden Tabellen:

**Tabelle 4**

Aufwandmengen:

Herbizid 0,25 kg/ha

Antidot 0,25 kg/ha

| Verbindung Nr. | relative Schutzwirkung in Prozent |
|---|---|
| 3 | 38 |

**Tabelle 5**

Aufwandmengen:

Herbizid 0,5 kg/ha

Antidot 0,5 kg/ha

| Verbindung Nr. | relative Schutzwirkung in Prozent |
|---|---|
| 8 | 88 |

**Tabelle 6**

Aufwandmengen:

Herbizid 0,5 kg/ha

Antidot 1,5 kg/ha

| Verbindung Nr. | relative Schutzwirkung in Prozent | Verbindung Nr. | relative Schutzwirkung in Prozent |
|---|---|---|---|
| 4 | 12,5 | 130 | 38 |
| 6 | 75 | 260 | 50 |
| 13 | 12,5 | 262 | 63 |
| 20 | 50 | 264 | 25 |
| 28 | 88 | 266 | 75 |
| 56 | 50 | 268 | 38 |
| 84 | 25 | 320 | 63 |
| 89 | 12,5 | 321 | 63 |

Fortsetzung Tabelle 6

| Verbindung Nr. | relative Schutzwirkung in Prozent | Verbindung Nr. | relative Schutzwirkung in Prozent |
|---|---|---|---|
| 323 | 75 | 343 | 38 |
| 324 | 63 | 347 | 12,5 |
| 325 | 75 | 348 | 12,5 |
| 326 | 75 | 350 | 38 |
| 327 | 75 | 374 | 75 |
| 328 | 63 | 375 | 75 |
| 329 | 50 | 376 | 63 |
| 330 | 38 | 377 | 63 |
| 331 | 63 | 379 | 38 |
| 332 | 25 | 380 | 25 |
| 333 | 25 | 383 | 63 |
| 334 | 63 | 384 | 12,5 |
| 336 | 63 | 386 | 38 |
| 337 | 38 | 387 | 50 |
| 338 | 50 | 388 | 50 |
| 339 | 50 | 389 | 25 |
| 340 | 50 | 390 | 50 |
| 341 | 63 | 391 | 50 |
| 342 | 50 | | |

Beispiel 14: Samenquellung Reis, Herbizid im Vorlaufverfahren

Reissamen werden 48 Stunden mit Lösungen der als Antidot zu prüfenden Substanz in einer Konzentration von 100 ppm getränkt. Man lässt die Samen dann etwa zwei Stunden trocknen, bis sie nicht mehr kleben. Plastikbehälter (Länge x Breite x Höhe = 25 x 17 x 12 cm) werden bis 2 cm unter dem Rand mit sandigem Lehm gefüllt. Die verquollenen Samen werden auf die Bodenoberfläche des Behälters gesät und nur ganz schwach mit Erde bedeckt. Die Erde wird in einem feuchten (nicht sumpfigen) Zustand gehalten. Nun wird das Herbizid 2-Chlor-2',6'-di-äthyl-N-[2"-(n-propoxy)-äthyl]-acetanilid in verdünnter Lösung auf die Bodenoberfläche versprüht. Der Wasserstand wird entsprechend dem Wachstum der Pflanzen sukzessive erhöht. 18 Tage nach der Herbizidapplikation wird die Schutzwirkung des Antidots in Prozent bonitiert. Als Referenz dienen dabei die mit dem Herbizid allein behandelten Pflanzen sowie die vollständig unbehandelte Kontrolle. Die Ergebnisse zeigt die folgende Tabelle:

Tabelle 7

| Antidot Verbindung Nr. | Antidot ppm | Herbizid kg AS/ha | Relative Schutzwirkung in % |
|---|---|---|---|
| 8 | 100 | 0,25 | 50 |
| 366 | 100 | 0,25 | 38 |
| 321 | 100 | 0,25 | 25 |
| 343 | 100 | 0,25 | 63 |
| 384 | 100 | 0,25 | 63 |

Beispiel 15: Saatbeizung Reis, Herbizid im Vorlaufverfahren

Reissamen werden mit der als Antidot zu prüfenden Substanz in einen Glasbehälter gegeben und durch Schütteln und Rotation gut durchgemischt. Behälter (Länge x Breite x Höhe = 47 x 29 x 24 cm) werden mit sandiger Lehmerde gefüllt und die gebeizten Samen eingesät. Nach dem Bedecken des Samens mit Erde wird das Herbizid 2-Chlor-6'-äthyl-N-(2''-methoxy-1''-methyläthyl)-acet-o-toluidid in einer verdünnten Lösung auf die Bodenoberfläche versprüht. 20 Tage nach der Aussaat, wenn die Pflanzen das 3-Blattstadium erreicht haben, wird die Bodenoberfläche mit Wasser 4 cm hoch überschichtet. 30 Tage nach der Herbizidapplikation wird die Schutzwirkung des Antidots in Prozent bonitiert. Als Referenz dienen dabei die mit Herbizid allein behandelten Pflanzen sowie die vollständig unbehandelte Kontrolle. Die Verbindungen der Formel I zeigen auch in diesem Test eine gute Wirkung.

Beispiel 16: Saatbeizung Reis, Herbizid im Vorlaufverfahren

Reissamen der Sorte IR-36 werden mit der als Antidot zu prüfenden Substanz in einen Glasbehälter gegeben und durch Schütteln und Rotation gut durchgemischt. Plastikbehälter (Länge x Breite x Höhe = 47 x 29 x 24 cm) werden mit sandiger Lehmerde gefüllt und die gebeizten Samen eingesät. Nach dem Bedecken des Samens mit Erde wird das Herbizid 2-Chlor-6'-äthyl-N-(2''-methoxy-1''-methyläthyl)-acet-o-toluidid auf die Bodenoberfläche versprüht. 18 Tage nach der Aussaat wird die Schutzwirkung des Antidots in Prozent bonitiert. Als Referenzen dienen dabei die mit Herbizid allein behandelten Pflanzen sowie die vollständig unbehandelte Kontrolle. Auch in diesem Test zeigen die Verbindungen der Formel I eine gute Wirkung.

Beispiel 17: Tankmischung im Vorauflaufverfahren in Sorghum

Töpfe (oberer Durchmesser 6 cm) werden mit sandiger Lehmerde gefüllt und Sorghumsamen der Sorte 6522 eingesät. Nach dem Bedecken der Samen mit Erde wird die als Antidot zu prüfende Substanz zusammen mit dem Herbizid 2-Chlor-6'-äthyl-N-(2''-methoxy-1''-methyläthyl)-acet-o-toluidid in verdünnter Lösung als Tankmischung auf die Bodenoberfläche versprüht. 21 Tage nach der Herbizidapplikation wird die Schutzwirkung des Antidots in Prozent bonitiert. Als Referenzen dienen dabei die mit Herbizid allein behandelten Pflanzen sowie die vollständig unbehandelte Kontrolle. Die Ergebnisse zeigt die folgende Tabelle:

Tabelle 8

Aufwandmengen:
Herbizid 1,5 kg/ha
Antidot 1,5 kg/ha

| Verbindung Nr. | relative Schutzwirkung in Prozent |
|---|---|
| 364 | 50 |
| 367 | 50 |
| 371 | 38 |

Beispiel 18: Saatbeizung Reis, Herbizid im Vorauflaufverfahren

Reissamen werden mit der als Antidot zu prüfenden Substanz in einen Glasbehälter gegeben und durch Schütteln und Rotation gut durchgemischt. Behälter (Länge x Breite x Höhe = 47 x 29 x 24 cm) werden mit sandiger Lehmerde gefüllt und die gebeizten Samen eingesät. Nach dem Bedecken des Samens mit Erde wird das Herbizid 2-[4-(3,5-Dichlorpyridyl-2-oxy)-phenoxy]-propionsäure-2-propinylester in einer verdünnten Lösung auf die Bodenoberfläche versprüht. 20 Tage nach der Aussaat, wenn die Pflanzen das 3-Blattstadium erreicht haben, wird die Bodenoberfläche mit Wasser 4 cm hoch überschichtet. 30 Tage nach der Herbizidapplikation wird die Schutzwirkung des Antidots in Prozent bonitiert. Als Referenz dienen dabei die mit Herbizid allein behandelten Pflanzen sowie die vollständig unbehandelte Kontrolle. Die Ergebnisse zeigt die folgende Tabelle:

Tabelle 9

| Antidot Verbindung Nr. | Antidot g AS/kg Samen | Herbizid kg AS/ha | Raltive Schutzwirkung in % |
|---|---|---|---|
| 3 | 0,6 | 0,25 | 50 |
| | 0,4 | 0,25 | 63 |
| | 0,2 | 0,25 | 63 |

Beispiel 19: Saatbeizung Reis, Herbizid im Nachauflaufverfahren

Reissamen werden mit der als Antidot zu prüfenden Substanz in einen Glasbehälter gegeben und durch Schütteln und Rotation gut durchgemischt. Plastikbehälter (Länge x Breite x Höhe = 25 x 17 x 12 cm) werden mit sandiger Lehmerde gefüllt und die gebeizten Samen eingesät. Nach dem Bedecken des Samens mit Erde wird das Herbizid 2-[4-(3,5-Dichlorpyridyl-2-oxy)-phenoxy]-propionsäure-2-propinylester im Nachauflaufverfahren appliziert. 21 Tage nach der Applikation wird die Schutzwirkung des Antidots in Prozent bonitiert. Als Referenz dienen dabei die mit Herbizid allein behandelten Pflanzen sowie die vollständig unbehandelte Kontrolle. Die Ergebnisse zeigt die folgende Tabelle:

Tabelle 10

| Antidot Verbindung Nr. | Antidot g AS/kg Samen | Herbizid kg AS/ha | Relative Schutzwirkung in % |
|---|---|---|---|
| 3 | 1<br>0,8<br>0,6 | 0,25<br>0,25<br>0,25 | 25<br>25<br>38 |

Beispiel 20: Saatbeizung Reis, Herbizid im Vorauflaufverfahren

Reissamen der Sorte IR-36 werden mit der als Antidot zu prüfenden Substanz in einen Glasbehälter gegeben und durch Schütteln und Rotation gut durchgemischt. Plastikbehälter (Länge x Breite x Höhe = 47 x 29 x 24 cm) werden mit sandiger Lehmerde gefüllt und die gebeizten Samen eingesät. Nach dem Bedecken des Samens mit Erde wird das Herbizid 2-[4-(3,5-Dichlorpyridyl-2-oxy)-phenoxy]-propionsäure-2-propinylester auf die Bodenoberfläche versprüht. 18 Tage nach der Aussaat wird die Schutzwirkung des Antidots in Prozent bonitiert. Als Referenzen dienen dabei die mit Herbizid allein behandelten Pflanzen sowie die vollständig unbehandelte Kontrolle. Die Ergebnisse zeigt die folgende Tabelle:

Tabelle 11

| Antidot Verbindung Nr. | Antidot g AS/kg Samen | Herbizid kg AS/ha | Relative Schutzwirkung in % |
|---|---|---|---|
| 3 | 0,6<br>0,4<br>0,2 | 0,25<br>0,25<br>0,25 | 50<br>63<br>63 |

Beispiel 21: Saatbeizung Weizen, Herbizid im Nachauflaufverfahren

Weizensamen werden mit der als Antidot zu prüfenden Substanz in einen Glasbehälter gegeben und durch Schütteln und Rotation gut durchgemischt. Plastikbehälter (Länge x Breite x Höhe = 25 x 17 x 12 cm) werden mit sandiger Lehmerde gefüllt und die gebeizten Samen eingesät. Nach dem Bedecken des Samens mit Erde wird das Herbizid N-[(2-(2-Chloräthoxy)-phenyl)-sulfonyl]-N'-(4-methoxy-6-methyl-1,3,5-triazin-2-yl)-harnstoff im Nachauflaufverfahren appliziert. 21 Tage nach der Applikation wird die Schutzwirkung des Antidots in Prozent bonitiert. Als Referenz dienen dabei die mit Herbizid allein behandelten Pflanzen sowie die vollständig unbehandelte Kontrolle. Die Ergebnisse zeigt die folgende Tabelle:

Tabelle 12

| Antidot Verbindung Nr. | Antidot g AS/kg Samen | Herbizid kg AS/ha | Relative Schutzwirkung in % |
|---|---|---|---|
| 8 | 0,25<br>0,125 | 1,0<br>1,0 | 25<br>25 |
| | 0,25<br>0,125 | 0,5<br>0,5 | 12,5<br>12,5 |

Beispiel 22: Saatbeizung in Weizen, Herbizid im Vorauflaufverfahren

Weizensamen werden mit der als Antidot zu prüfenden Substanz in einen Glasbehälter gegeben und durch Schütteln und Rotation gut durchgemischt. Plastikbehälter (Länge x Breite x Höhe = 25 x 17 x 12 cm) werden mit sandiger Lehmerde gefüllt und die gebeizten Samen eingesät. Nach dem Bedecken des

Samens mit Erde wird das Herbizid N-[(2-(2-Chloräthoxy)-phenyl)-sulfonyl]-N'-(4-methoxy-6-methyl-1,3,5-triazin-2-yl)-harnstoff auf die Bodenoberfläche gesprüht. 21 Tage nach der Herbizidapplikation wird die Schutzwirkung des Antidots in Prozent bonitiert. Als Referenz dienen dabei die mit Herbizid allein behandelten Pflanzen sowie die vollständig unbehandelte Kontrolle. Die Ergebnisse zeigt die nachfolgende Tabelle:

Tabelle 13

| Antidot Verbindung Nr. | Antidot g AS/kg Samen | Herbizid kg AS/ha | Relative Schutzwirkung in % |
|---|---|---|---|
| 8 | 0,25<br>0,125 | 1,0<br>1,0 | 25<br>25 |
| 8 | 0,25<br>0,125 | 0,5<br>0,5 | 25<br>25 |
| 8 | 0,25<br>0,125 | 0,25<br>0,25 | 12,5<br>12,5 |

Beispiel 23: Ertragssteigerung durch Wachstumsregulierung bei Soja

In Kunststoffbehältern mit einem Erde-Torf-Sandgemisch im Verhältnis 6:3:1 werden Sojabohnen dei Sorte "Hark" angesät und in einer Klimakammer unter otpimalen Bedingungen bezüglich Temperatur, Beleuchtung, Düngung und Bewässerung gehalten. Die Pflanzen entwickeln sich so in ca. 5 Wochen zum 5- bis 6-Trifolia-Blattstadium. In diesem Entwicklungsstadium werden die Pflanzen mit der wässrigen Brühe eines Wirkstoffes der Formel I bis zur guten Benetzung besprüht. Die Wirkstoffkonzentration beträgt 500 ppm Aktivsubstanz. Die Auswertung erfolgt 5 Wochen nach Applikation des Wirkstoffs. Im Vergleich zu unbehandelten Kontrollpflanzen weisen die mit erfindungsgemässen Wirkstoffen der Formel I behandelten Pflanzen eine merkliche Erhöhung der Anzahl und des Gewichts der geernteten Schoten auf. Als besonders wirksam erweisen sich die Verbindungen Nr. 56, 269 und 340.

Beispiel 24: Förderung des Wurzelwachstums bei Weizen und Soja

Verbindungen der Formel I werden als wässrige Dispersion, erhalten aus 25%igem Spritzpulver, angewendet. Der Versuch wird mit Samen durchgeführt, die in mit Erde gefüllte Plastikzylinder von 5 x 30 cm eingesät werden (10 Samen pro Zylinder), wobei a) die Samen vor der Einsaat in Aufwandmengen von 4-130 mg pro kg Saatgut behandelt werden oder b) unbehandelte Samen eingesät und der Boden mit der Wirkstoffdispersion in Aufwandmengen von 0,3 bis 3 kg pro Hektar besprüht wird. Die Zylinder werden in einer Klimakammer unter kontrollierten Bedingungen gehalten. Nach 10 Tagen werden Keimlinge durch vorsichtiges Waschen mit Wasser von der Erde befreit und Länge und Trockengewicht der Wurzeln gemessen. Von den Verbindungen der Formel I zeigen in diesem Test insbesondere die Verbindungen Nr. 326 und 331 eine gute Wirkung.

**Patentansprüche**
**Patentansprüche für folgende Vertragsstaaten : BE, CH, DE, FR, IT, LI, NL, SE**

**1.** Verwendung von Chinolinderivaten der Formel I

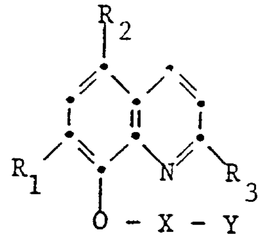

(I),

worin

$R_1$ Wasserstoff, Chlor, Jod oder Brom, $R_2$ Wasserstoff, Chlor, Jod, Brom, Nitro, Methyl oder Aethyl, und $R_3$ Wasserstoff oder Methyl bedeuten, X für eine gegebenenfalls durch eine oder zwei Methylgruppen substituierte Methylenbrücke und Y für einen Oxazolin-2-yl-Rest, $-COOR_4$, $-COSR_5$ oder $-CONR_6R_7$ steht, worin

$R_4$ - Wasserstoff, ein Alkalimetallkation oder ein quaternäres Ammoniumkation ausgewählt aus der Gruppe Ammoniumkation, Trimethyl-, Triäthyl- und Tri-(2-hydroryäthylen)-ammoniumkation;
- $C_1$-$C_{18}$-Alkyl;
- $C_1$-$C_{10}$-Alkyl, welches durch 1 oder 2 Hydroxygruppen substituiert ist,
- $C_1$-$C_4$-Alkyl, welches durch 1 bis 3 Chlor- oder Bromatome, eine Nitrogruppe, eine Cyanogruppe, eine $C_1$-$C_4$-Alkoxygruppe, eine $C_2$-$C_8$-Alkoxygruppe, welche durch 1 oder 2 Sauerstoffatome unterbrochen ist, eine Hydroxy-$C_1$-$C_4$-Alkoxygruppe, eine Hydroxy-$C_2$-$C_6$-Alkoxygruppe, welche durch 1 oder 2 Sauerstoffatome unterbrochen ist, eine $C_1$-$C_4$-Alkoxycarbonylgruppe, eine Di-($C_1$-$C_4$-Alkyl)-aminogruppe, einen Phenylrest, welcher unsubstituiert oder durch Chlor oder Methoxy substituiert ist, einen Cyclohexylrest, einen Furan-, Tetrahydrofuran, Tetrahydropyran-, Pyridino-, Piperidino- oder Morpholinorest substituiert ist;
- $C_3$-$C_{10}$-Alkenyl;
- $C_3$-$C_4$-Alkenyl, welches unsubstituiert oder durch eine Hydroxygruppe substituiert ist;
- Cyclohexyl, welches unsubstituiert oder durch 1 oder 2 Methylgruppen substituiert ist;
- Phenyl, welches unsubstituiert oder durch 1 bis 2 Substituenten aus der Gruppe Chlor, Nitro, $C_1$-$C_4$-Alkyl oder Methoxy substituiert ist;
- Naphthyl; oder
- Pyridin,

$R_5$ - $C_1$-$C_{12}$-Alkyl;
- $C_1$-$C_4$-Alkyl, welches durch eine $C_1$-$C_4$-Alkoxycarbonylgruppe substituiert ist;
- Phenyl, welches unsubstituiert oder durch ein Chloratom substituiert ist,

$R_6$ - Wasserstoff;
- $C_1$-$C_{18}$-Alkyl;
- $C_2$-$C_8$-Alkyl, welches durch eine Aminogruppe substituiert ist;
- $C_2$-$C_6$-Alkyl, welches durch eine Hydroxygruppe substituiert ist;
- $C_2$-$C_4$-Alkyl, welches durch ein Chlor- oder Bromatom, eine $C_1$-$C_4$-Alkylaminogruppe, eine Di-($C_1$-$C_4$-Alkyl)-aminogruppe, eine $C_1$-$C_4$-Hydroxyalkylaminogruppe, eine Di-($C_1$-$C_3$-Hydroxyalkyl)-aminogruppe, eine $C_1$-$C_4$-Alkoxygruppe oder eine $C_1$-$C_4$-Alkoxycarbonylgruppe substituiert ist;
- $C_1$-$C_4$-Alkyl, welches durch eine Cyanogruppe oder einen Phenylrest, welcher unsubstituiert oder durch Chlor substituiert ist, einen Furanyl-, Tetrahydrofuranyl-, Piperdino- oder Morpholinorest substituiert ist;
- $C_1$-$C_3$-Alkoxy;
- $C_3$-$C_4$-Alkenyl;
- Cyclohexyl, welches unsubstituiert oder durch eine Methylgruppe substituiert ist;
- Phenyl, welches unsubstituiert oder durch 1 oder 2 Substituenten aus der Gruppe Chlor, Nitro, Cyano, Methyl, Aethyl, Methoxy und Trifluormethyl substituiert ist, und

$R_7$ - Wasserstoff;
- $C_1$-$C_6$-Alkyl;
- $C_1$-$C_4$-Alkyl, welches durch eine Hydroxygruppe oder eine Cyanogruppe substituiert ist;
- $C_2$-$C_4$-Alkoxyalkyl;
- $C_3$-$C_4$-Alkenyl;
- Cyclohexyl; oder
- Phenyl, oder

$R_6$ und $R_7$ gemeinsam mit dem Stickstoffatom, an das sie gebunden sind, einen unsubstituierten oder durch 1 oder 2 Methylgruppen substituierten Piperidino-, Tetrahydropyrimidino-, Morpholino- oder Imidazolylrest bedeuten, oder X und Y zusammen einen Tetrahydrofuran-2-on-3-yl-Ring bilden; zum Schützen von Kulturhirse, Reis, Mais und Getreidearten gegen kulturpflanzenschädigende Wirkungen von Herbiziden.

**2.** Verwendung nach Anspruch 1, dadurch gekennzeichnet, dass in der Verbindung der Formel I Y für $-COOR_4$ steht.

**3.** Verwendung nach Anspruch 1, dadurch gekennzeichnet, dass in der Verbindung der Formel I Y für $-COSR_5$ steht.

**4.** Verwendung nach Anspruch 1, dadurch gekennzeichnet, dass in der Verbindung der Formel I Y für $-CONR_6R_7$ steht.

**5.** Verwendung nach Anspruch 1, dadurch gekennzeichnet, dass in der Verbindung der Formel I X und Y zusammen einen Tetrahydrofuran-2-on-3-yl-Ring bilden.

**6.** Verwendung nach Anspruch 1 zum Schützen von Kulturhirse, Reis, Mais und Getreidearten gegen schädigende Wirkungen von Diphenyläthern.

**7.** Verwendung nach Anspruch 1 zum Schützen von Kulturhirse, Reis, Mais und Getreidearten gegen schädigende Wirkungen von substituierten Pyridyloxyphenoxypropionsäureestern.

**8.** Verwendung nach Anspruch 7 zum Schützen von Kulturhirse, Reis, Mais und Getreidearten gegen schädigende Wirkungen von 2-[4-(3,5-Dichlorpyridyl-2-oxy)-phenoxy]-propionsäure-2-propinylester.

**9.** Verwendung nach Anspruch 1 zum Schützen von Getreidearten.

**10.** Verbindung der Formel I

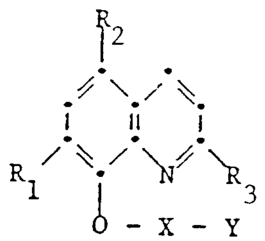

(I),

worin

$R_1$ Wasserstoff, Chlor, Jod oder Brom, $R_2$ Wasserstoff, Chlor, Jod, Brom, Nitro, Methyl oder Aethyl, und $R_3$ Wasserstoff oder Methyl bedeuten, X für eine gegebenenfalls durch eine oder zwei Methylgruppen substituierte Methylenbrücke und Y für einen Oxazolin-2-yl-Rest, $-COOR_4$, $-COSR_5$ oder $-CONR_6R_7$ steht, worin

$R_4$ - ein quaternäres Ammoniumkation, ausgewählt aus der Gruppe Trimethyl-, Triäthyl- und Tri-(2-hydroxyäthylen)-ammoniumkation;
-Alkyl mit mehr als 5 C-Atomen aber höchstens 18 C-Atomen;
- $C_1$-$C_{10}$-Alkyl, welches durch 1 oder 2 Hydroxygruppen substituiert ist;
- $C_1$-$C_4$-Alkyl, welches durch 1 bis 3 Chlor- oder Bromatome, eine Nitrogruppe, eine Cyanogruppe, eine $C_1$-$C_4$-Alkoxygruppe, eine $C_2$-$C_8$-Alkoxygruppe, welche durch 1 oder 2 Sauerstoffatome unterbrochen ist, eine Hydroxy-$C_1$-$C_4$-Alkoxygruppe, eine Hydroxy-$C_2$-$C_6$-Alkoxygruppe, welche durch 1 oder 2 Sauerstoffatome unterbrochen ist, eine $C_1$-$C_4$-Alkoxycarbonylgruppe, eine Di-($C_1$-$C_4$-Alkyl)-aminogruppe, einen Phenylrest, welcher unsubstituiert oder durch Chlor oder Methoxy substituiert ist, einen Cyclohexylrest, einen Furan-, Tetrahydrofuran-, Tetrahydropyran-, Pyridino-, Piperidino- oder Morpholinorest substituiert ist;
- $C_3$-$C_{10}$-Alkenyl;
- $C_3$-$C_4$-Alkinyl, welches unsubstituiert oder durch eine Hydroxygruppe substituiert ist;
- Cyclohexyl, welches unsubstituiert oder durch 1 oder 2 Methylgruppen substituiert ist;
- Phenyl, welches unsubstituiert oder durch 1 bis 2 Substituenten aus der Gruppe Chlor, Nitro, $C_1$-$C_4$-Alkyl oder Methoxy substituiert ist;
- Naphthyl; oder
- Pyridin,

$R_5$ - $C_1$-$C_{12}$-Alkyl;
- $C_1$-$C_4$-Alkyl, welches durch eine $C_1$-$C_4$-Alkoxycarbonylgruppe substituiert ist;
- Phenyl, welches unsubstituiert oder durch ein Chloratom substituiert ist,

$R_6$

- Alkyl mit mehr als 6 C-Atomen aber höchstens 18 C-Atomen;
- $C_2$-$C_8$-Alkyl, welches durch eine Aminogruppe substituiert ist;
- $C_2$-$C_6$-Alkyl, welches durch eine Hydroxygruppe substituiert ist;
- $C_2$-$C_4$-Alkyl, welches durch ein Chlor- oder Bromatom, eine $C_1$-$C_4$-Hydroxyalkylaminogruppe, eine Di-($C_1$-$C_3$-Hydroxylalkyl)-aminogruppe oder eine $C_1$-$C_4$-Alkoxycarbonylgruppe substituiert ist;
- $C_1$-$C_4$-Alkyl, welches durch eine Cyanogruppe oder einen Phenylrest, welcher unsubstituiert oder durch Chlor substituiert ist, einen Furanyl-, Tetrahydrofuranyl-, Piperidino- oder Morpholinorest substituiert ist;
- $C_1$-$C_3$-Alkoxy;
- Cyclohexyl, welches durch eine Methylgruppe substituiert ist;
- Phenyl, welches durch 1 oder 2 Substituenten aus der Gruppe Nitro, Cyano, Methyl, Aethyl, Methoxy und Trifluormethyl substituiert ist, und

$R_7$

- Wasserstoff;
- $C_1$-$C_6$-Alkyl;
- $C_1$-$C_4$-Allyl, welches durch eine Hydroxygruppe oder eine Cyanogruppe substituiert ist;
- $C_2$-$C_4$-Alkoxyalkyl;
- $C_3$-$C_4$-Alkenyl;
- Cyclohexyl; oder
- Phenyl, oder

$R_6$ und $R_7$ gemeinsam mit dem Stickstoffatom, an das sie gebunden sind, einen durch 1 oder 2 Methylgruppen substituierten Piperidino-, Tetrahydropyrimidino-, Morpholino- oder Imidazolylrest bedeuten, oder X und Y zusammen einen Tetrahydrofuran-2-on-3-yl-Rest bilden.

**11.** Verbindung nach Anspruch 10, dadurch gekennzeichnet, dass Y für $-COOR_4$ steht.

**12.** Verbindung nach Anspruch 10, dadurch gekennzeichnet, dass Y für $-COSR_5$ steht.

**13.** Verbindung nach Anspruch 10, dadurch gekennzeichnet, dass Y für $-CONR_6R_7$ steht.

**14.** Verbindung nach Anspruch 10, dadurch gekennzeichnet, dass X und Y zusammen einen unsubstituierten Tetrahydrofuran-2-on-3-yl-Ring bilden.

**15.** Mittel zum Schützen von Kulturhirse, Reis, Mais und Getreidearten gegen kulturpflanzenschädigende Wirkungen von Herbiziden, dadurch gekennzeichnet, dass es als aktive Komponente eine Verbindung nach Anspruch 10 enthält.

**16.** Mittel nach Anspruch 15, dadurch gekennzeichnet, dass es die aktive Komponente zusammen mit Trägermaterial und/oder inerten Zuschlagstoffen enthält.

**17.** Mittel nach Anspruch 15, dadurch gekennzeichnet, dass es als aktive Komponente eine Verbindung wie im Anspruch 10 definiert, zusammen mit einem kulturpflanzenschädigenden Herbizid enthält.

**18.** Mittel nach Anspruch 17, dadurch gekennzeichnet, dass es als Herbizid einen Diphenyläther enthält.

**19.** Mittel nach Anspruch 17, dadurch gekennzeichnet, dass es als Herbizid einen substituierten Pyridyloxyphenoxypropionsäureester enthält.

**20.** Mittel nach Anspruch 19, dadurch gekennzeichnet, dass es als Herbizid 2-[4-(3,5-Dichlorpyridyl-2-oxy)-phenoxy]-propionsäure-2-propinylester enthält.

**21.** Verfahren zur Herstellung von Verbindungen nach Anspruch 10, dadurch gekennzeichnet, dass man eine Verbindung der Formel II

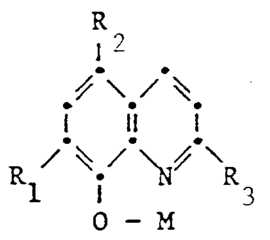

(II),

worin $R_1$, $R_2$ und $R_3$ die angegebenen Bedeutungen haben und M für Wasserstoff, ein Alkali- oder Erdalkalimetallatom steht, mit einer Verbindung der Formel III

Z - X - Y (III),

worin X und Y die angegebenen Bedeutungen haben und Z für einen abspaltbaren Rest steht, umsetzt.

**22.** Verfahren zur Herstellung von Verbindungen nach Anspruch 10, in denen Y für $-COOR_4$ steht, dadurch gekennzeichnet, dass man ein Säurehalogenid der Formel IV

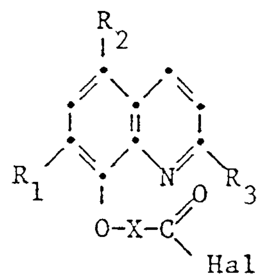

(IV),

worin $R_1$, $R_2$, $R_3$ und X die angegebenen Bedeutungen haben und Hal für ein Halogenatom steht, mit einer Verbindung der Formel V

M' - O - $R_4$ (V),

worin $R_4$ die angegebene Bedeutung hat und M' für Wasserstoff, ein Alkali- oder Erdalkalimetallatom steht, umsetzt.

**23.** Verfahren zur Herstellung von Verbindungen nach Anspruch 10, in denen Y für $-COSR_5$ steht, dadurch gekennzeichnet, dass man ein Säurehalogenid der Formel VI

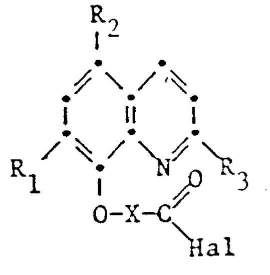

(VI),

worin $R_1$, $R_2$, $R_3$ und X die angegebenen Bedeutungen haben und Hal für ein Halogenatom steht, mit einer Verbindung der Formel VII

M'' - S - $R_5$ (VII),

worin $R_5$ die angegebene Bedeutung hat und M'' für Wasserstoff, ein Alkali- oder Erdalkalimetallatom steht, umsetzt.

**24.** Verfahren zur Herstellung von Verbindungen nach Anspruch 10, in denen Y für $-CONR_6R_7$ steht, dadurch gekennzeichnet, dass man ein Säurehalogenid der Formel VIII

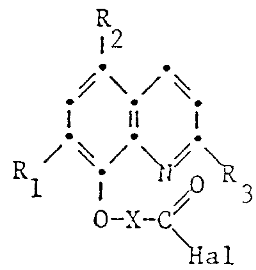

(VIII),

worin $R_1$, $R_2$, $R_3$ und X die angegebenen Bedeutungen haben und Hal für ein Halogenatom steht, mit einer Verbindung der Formel IX

$HNR_6R_7$ (IX),

worin $R_6$ und $R_7$ die angegebenen Bedeutungen haben, umsetzt.

**25.** Verfahren zur Herstellung von Verbindungen nach Anspruch 10, in denen Y für $-COOR_4$ steht, dadurch gekennzeichnet, dass man eine Verbindung der Formel X

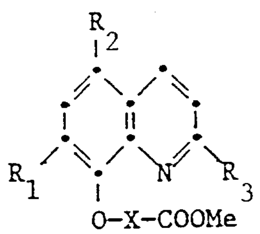

(X),

worin $R_1$, $R_2$, $R_3$ und X die angegebenen Bedeutungen haben und Me für ein Alkalimetall-, Erdalkalimetall-, Blei- oder Silberatom steht, mit einer Verbindung der Formel XI

$Hal - R_4$ (XI),

worin $R_4$ die angegebene Bedeutung hat und Hal für ein Halogenatom steht, umsetzt.

**26.** Verfahren zur Herstellung von Verbindungen nach Anspruch 10, in denen Y für $-COSR_5$ steht, dadurch gekennzeichnet, dass man eine Verbindung der Formel XII

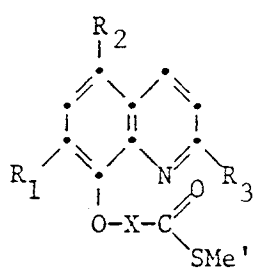

(XII),

worin $R_1$, $R_2$, $R_3$ und X die angegebenen Bedeutungen haben und Me' für ein Alkalimetall-, Erdalkalimetall-, Blei- oder Silberatom steht, mit einer Verbindung der Formel XIII

$Hal - R_5$ (XIII),

worin $R_5$ die angegebene Bedeutung hat und Hal für ein Halogenatom steht, umsetzt.

**27.** Verfahren zur Herstellung von Verbindungen nach Anspruch 10, worin Y für $-COOR_4$ steht, dadurch gekennzeichnet, dass man eine Verbindung der Formel XIV

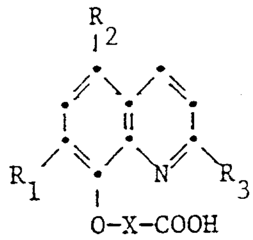

(XIV),

worin $R_1$, $R_2$, $R_3$ und X die angegebenen Bedeutungen haben, mit einer Verbindung der Formel XV

$HO - R_4$ (XV),

worin $R_4$ die angegebene Bedeutung hat, umsetzt.

**28.** Verfahren zur Herstellung von Verbindungen nach Anspruch 10, worin Y für $-COSR_5$ steht, dadurch gekennzeichnet, dass man eine Verbindung der Formel XVI

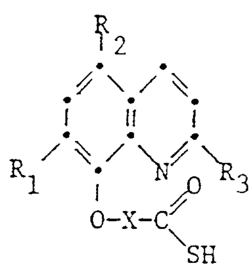

(XVI),

worin $R_1$, $R_2$, $R_3$ und X die angegebenen Bedeutungen haben, mit einer Verbindung der Formel XVII

$HO - R_5$ (XVII),

worin $R_5$ die angegebene Bedeutung hat, umsetzt.

**29.** Verfahren zur Herstellung von Verbindungen nach Anspruch 10, in denen Y für $-COOR_4$ steht, dadurch gekennzeichnet, dass man eine Verbindung der Formel Ia

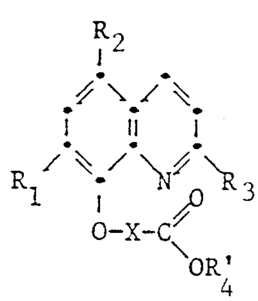

(Ia),

worin $R_1$, $R_2$, $R_3$ und X die angegebenen Bedeutungen haben und $R_4'$ die für $R_4$ angegebene Bedeutung hat, mit einer Verbindung der Formel XVIII

$HO - R_4''$ (XVIII),

worin $R_4''$ die für $R_4$ angegebene Bedeutung hat und nicht mit $R_4'$ identisch ist, umsetzt.

**30.** Verfahren zur Herstellung von Verbindungen nach Anspruch 10, in denen Y für $-COSR_5$ steht, dadurch gekennzeichnet, dass man eine Verbindung der Formel Ib

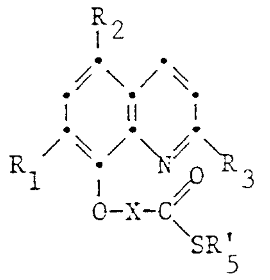

(Ib),

worin $R_1$, $R_2$, $R_3$ und X die angegebenen Bedeutungen haben und $R_5'$ die für $R_5$ angegebene Bedeutung hat, mit einer Verbindung der Formel XIX

$HSR_5''$ (XIX),

worin $R_5''$ die für $R_5$ angegebene Bedeutung hat und nicht mit $R_5'$ identisch ist, umsetzt.

**31.** Verfahren zur Herstellung von Verbindungen nach Anspruch 10, worin Y für $-CONR_6R_7$ steht, dadurch gekennzeichnet, dass man eine Verbindung der Formel XX

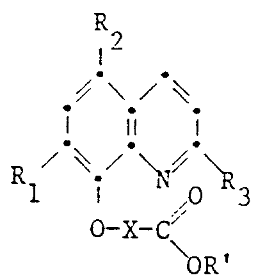

(XX),

worin $R_1$, $R_2$, $R_3$ und X die angegebenen Bedeutungen haben und R' für einen aliphatischen, acyclischen Kohlenwasserstoffrest steht, mit einer Verbindung der Formel XXI

$HNR_6R_7$ (XXI),

worin $R_6$ und $R_7$ die angegebenenen Bedeutungen haben, umsetzt.

**32.** Verfahren zur Herstellung von Verbindungen nach Anspruch 10, in denen Y für einen Oxazolin-2-yl-Rest steht, dadurch gekennzeichnet, dass man eine Verbindung der Formel XXIV

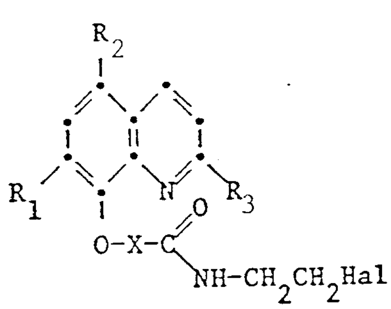

(XXIV),

worin $R_1$, $R_2$, $R_3$ und X die angegebenen Bedeutungen haben und Hal für ein Halogenatom, insbesondere ein Chlor- oder Bromatom steht, in Gegenwart eines säurebindenden Mittels cyclisiert.

**33.** Verfahren zur Herstellung von Verbindungen nach Anspruch 10, in denen Y für $-CH_2CH_2-$ steht, dadurch gekennzeichnet, dass man eine Verbindung der Formel XXII

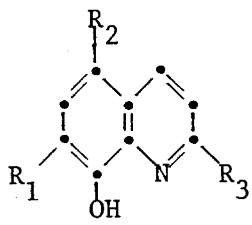

(XXII),

worin $R_1$, $R_2$ und $R_3$ die angegebenen Bedeutungen haben, mit einer Verbindung der Formel XXIII

$CH_2 = CH-Y$ (XXIII),

worin Y die angegebene Bedeutung hat, umsetzt.

**34.** Saatgut von von Kulturhirse, Reis, Mais und Getreidearten, behandelt mit einem Chinolinderivat der Formel I wie im Anspruch 1 definiert.

**Patentansprüche für folgenden Vertragsstaat : AT**

**1.** Verwendung von Chinolinderivaten der Formel I

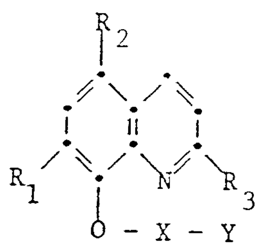

(I),

worin

$R_1$ Wasserstoff, Chlor, Jod oder Brom, $R_2$ Wasserstoff, Chlor, Jod, Brom, Nitro, Methyl oder Aethyl, und $R_3$ Wasserstoff oder Methyl bedeuten, X für eine gegebenenfalls durch eine oder zwei Methylgruppen substituierte Methylenbrücke und Y für einen Oxazolin-2-yl-Rest, $-COOR_4$, $-COSR_5$ oder $-CONR_6R_7$ steht, worin

$R_4$
- Wasserstoff, ein Alkalimetallkation oder ein quaternäres Ammoniumkation ausgewählt aus der Gruppe Ammoniumkation, Trimethyl-, Triäthyl- una Tri-(2-hydrozyäthylen)-ammoniumkation;
- $C_1$-$C_{18}$-Alkyl;
- $C_1$-$C_{10}$-Alkyl, welches durch 1 oder 2 Hydroxygruppen substituiert ist;
- $C_1$-$C_4$-Alkyl, welches durch 1 bis 3 Chlor- oder Bromatome, eine Nitrogruppe, eine Cyanogruppe, eine $C_1$-$C_4$-Alkoxygruppe, eine $C_2$-$C_8$-Alkoxygruppe, welche durch 1 oder 2 Sauerstoffatome unterbrochen ist, eine Hydroxy-$C_1$-$C_4$-Alkoxygruppe, eine Hydroxy$C_2$-$C_6$-Alkoxygruppe, welche durch 1 oder 2 Sauerstoffatome unterbrochen ist, eine $C_1$-$C_4$-Alkoxycarbonylgruppe, eine Di-($C_1$-$C_4$-Alkyl)-aminogruppe, einen Phenylrest, welcher unsubstituiert oder durch Chlor oder Methoxy substituiert ist, einen Cyclohexylrest, einen Furan-, Tetrahydrofuran-, Tetrahydropyran-, Pyridino-, Piperidino- oder Morpholinorest substituiert ist;
- $C_3$-$C_{10}$-Alkenyl;
- $C_3$-$C_4$-Alkinyl, welches unsubstituiert oder durch eine Hydroxygruppe substituiert ist;
- Cyclohexyl, welches unsubstituiert oder durch 1 oder 2 Methylgruppen substituiert ist;
- Phenyl, welches unsubstituiert oder durch 1 bis 2 Substituenten aus der Gruppe Chlor, Nitro, $C_1$-$C_4$-Alkyl oder Methoxy substituiert ist;

- Naphthyl; oder
- Pyridin,

$R_5$ - $C_1$-$C_{12}$-Alkyl;
- $C_1$-$C_4$-Alkyl, welches durch eine $C_1$-$C_4$-Alkoxycarbonylgruppe substituiert ist;
- Phenyl, welches unsubstituiert oder durch ein Chloratom substituiert ist,

$R_6$ - Wasserstoff;
- $C_1$-$C_{18}$-Alkyl;
- $C_2$-$C_8$-Alkyl, welches durch eine Aminogruppe substituiert ist;
- $C_2$-$C_6$-Alkyl, welches durch eine Hydroxygruppe substituiert ist;
- $C_2$-$C_4$-Alkyl, welches durch ein Chlor- oder Bromatom, eine $C_1$-$C_4$-Alkylaminogruppe, eine Di-($C_1$-$C_4$-Alkyl)-aminogruppe, eine $C_1$-$C_4$-Hydroxyalkylaminogruppe, eine Di-($C_1$-$C_3$-Hydroxyalkyl)-aminogruppe, eine $C_1$-$C_4$-Alkoxygruppe oder eine $C_1$-$C_4$-Alkoxycarbonylgruppe substituiert ist;
- $C_1$-$C_4$-Alkyl, welches durch eine Cyanogruppe oder einen Phenylrest, welcher unsubstituiert oder durch Chlor substituiert ist, einen Furanyl-, Tetrahydrofuranyl-, Piperidino- oder Morpholinorest substituiert ist;
- $C_1$-$C_3$-Alkoxy;
- $C_3$-$C_4$-Alkenyl;
- Cyclohexyl, welches unsubstituiert oder durch eine Methylgruppe substituiert ist;
- Phenyl, welches unsubstituiert oder durch 1 oder 2 Substituenten aus der Gruppe Chlor, Nitro, Cyano, Methyl, Aethyl, Methoxy und Trifluormethyl substituiert ist, und

$R_7$ - Wasserstoff;
- $C_1$-$C_6$-Alkyl;
- $C_1$-$C_4$-Alkyl, welches durch eine Hydroxygruppe oder eine Cyanogruppe substituiert ist;
- $C_2$-$C_4$-Alkoxyalkyl;
- $C_3$-$C_4$-Alkenyl;
- Cyclohexyl; oder
- Phenyl, oder

$R_6$ und $R_7$ gemeinsam mit dem Stickstoffatom, an das sie gebunden sind, einen unsubstituierten oder durch 1 oder 2 Methylgruppen substituierten Piperidino-, Tetrahydropyrimidino-, Morpholino- oder Imidazolylrest bedeuten, oder X und Y zusammen einen Tetrahydrofuran-2-on-3-yl-Ring bilden; zum Schützen von Kulturhirse, Reis, Mais und Getreidearten gegen kulturpflanzenschädigende Wirkungen von Herbiziden.

**2.** Verwendung nach Anspruch 1, dadurch gekennzeichnet dass in der Verbindung der Formel I Y für $-COOR_4$ steht.

**3.** Verwendung nach Anspruch 1, dadurch gekennzeichnet, dass in der Verbindung der Formel I Y für $-COSR_5$ steht.

**4.** Verwendung nach Anspruch 1, dadurch gekennzeichnet, dass in der Verbindung der Formel I Y für $-CONR_6R_7$ steht.

**5.** Verwendung nach Anspruch 1, dadurch gekennzeichnet, dass in der Verbindung der Formel I X und Y zusammen einen Tetrahydrofuran-2-on-3-yl-Ring bilden.

**6.** Verwendung nach Anspruch 1 zum Schützen von Kulturhirse, Reis, Mais und Getreidearten gegen schädigende Wirkungen von Diphenyläthern.

**7.** Verwendung nach Anspruch 1 zum Schützen von Kulturhirse, Reis, Mais und Getreidearten gegen schädigende Wirkungen von substituierten Pyridyloxyphenoxypropionsäureestern.

**8.** Verwendung nach Anspruch 7 zum Schützen von Kulturhirse, Reis, Mais und Getreidearten gegen schädigende Wirkungen von 2-[4-(3,5-Dichlorpyridyl-2-oxy)-phenoxy]-propionsäure-2-propinylester.

**9.** Verwendung nach Anspruch 1 zum Schützen von Getreidearten.

**10.** Mittel zum Schützen von Kulturhirse, Reis, Mais und Getreidearten gegen kulturpflanzenschädigende Wirkungen von Herbiziden, dadurch gekennzeichnet, dass es als aktive Komponente eine Verbindung der Formel I

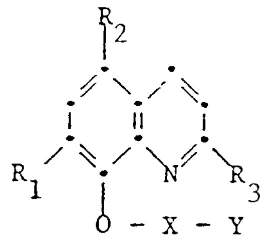

(I),

worin

$R_1$ Wasserstoff, Chlor, Jod oder Brom, $R_2$ Wasserstoff, Chlor, Jod, Brom, Nitro, Methyl oder Aethyl, und $R_3$ Wasserstoff oder Methyl bedeuten, X für eine gegebenenfalls durch eine oder zwei Methylgruppen substituierte Methylenbrücke und Y für einen Oxazolin-2-yl-Rest, $-COOR_4$, $-COSR_5$ oder $-CONR_6R_7$ steht, worin

$R_4$ - ein quaternäres Ammoniumkation, ausgewählt aus der Gruppe Trimethyl-, Triäthyl- und Tri-(2-hydroxyäthylen)-ammoniumkation;
-Alkyl mit mehr als 5 C-Atomen aber höchstens 18 C-Atomen;
- $C_1$-$C_{10}$-Alkyl, welches durch 1 oder 2 Hydroxygruppen substituiert ist;
- $C_1$-$C_4$-Alkyl, welches durch 1 bis 3 Chlor- oder Bromatome, eine Nitrogruppe, eine Cyanogruppe, eine $C_1$-$C_4$-Alkoxygruppe, eine $C_2$-$C_8$-Alkoxygruppe, welche durch 1 oder 2 Sauerstoffatome unterbrochen ist, eine Hydroxy-$C_1$-$C_4$-Alkoxygruppe, eine Hydroxy-$C_2$-$C_6$-Alkoxygruppe, welche durch 1 oder 2 Sauerstoffatome unterbrochen ist, eine $C_1$-$C_4$-Alkoxycarbonylgruppe, eine Di-($C_1$-$C_4$-Alkyl)-aminogruppe, einen Phenylrest, welcher unsubstituiert oder durch Chlor oder Methoxy substituiert ist, einen Cyclohexylrest, einen Furan-, Tetrahydrofuran-, Tetrahydropyran-, Pyridino-, Piperidino- oder Morpholinorest substituiert ist;
- $C_3$-$C_{10}$-Alkenyl;
- $C_3$-$C_4$-Alkinyl, welches unsubstituiert oder durch eine Hydroxygruppe substituiert ist;
- Cyclohexyl, welches unsubstituiert oder durch 1 oder 2 Methylgruppen substituiert ist
- Phenyl, welches unsubstituiert oder durch 1 bis 2 Substituenten aus der Gruppe Chlor, Nitro, $C_1$-$C_4$-Alkyl oder Methoxy substituiert ist;
- Naphthyl; oder
- Pyridin,

$R_5$ - $C_1$-$C_{12}$-Alkyl;
- $C_1$-$C_4$-Alkyl, welches durch eine $C_1$-$C_4$-Alkoxycarbonylgruppe substituiert ist;
- Phenyl, welches unsubstituiert oder durch ein Chloratom substituiert ist,

$R_6$
- Alkyl mit mehr als 6 C-Atomen aber höchstens 18 C-Atomen;
- $C_2$-$C_8$-Alkyl, welches durch eine Aminogruppe substituiert ist;
- $C_2$-$C_6$-Alkyl, welches durch eine Hydroxygruppe substituiert ist;
- $C_2$-$C_4$-Alkyl, welches durch ein Chlor- oder Bromatom, eine $C_1$-$C_4$-Hydroxyalkylaminogruppe, eine Di-($C_1$-$C_3$-Hydroxylalkyl)-aminogruppe oder eine $C_1$-$C_4$-Alkoxycarbonylgruppe substituiert ist;
- $C_1$-$C_4$-Alkyl, welches durch eine Cyanogruppe oder einen Phenylrest, welcher unsubstituiert oder durch Chlor substituiert ist, einen Furanyl-, Tetrahydrofuranyl-, Piperidino- oder Morpholinorest substituiert ist;
- $C_1$-$C_3$-Alkoxy;
- Cyclohexyl, welches durch eine Methylgruppe subtituiert ist;
- Phenyl, welches durch 1 oder 2 Substituenten aus der Gruppe Nitro, Cyano, Methyl, Aethyl, Methoxy und Trifluormethyl substituiert ist, und

$R_7$ - Wasserstoff;
- $C_1$-$C_6$-Alkyl;
- $C_1$-$C_4$-Alkyl, welches durch eine Hydroxygruppe oder eine Cyanogruppe substituiert ist;
- $C_2$-$C_4$-Alkoxyalkyl;

- $C_3$-$C_4$-Alkenyl;
- Cyclohexyl; oder
- Phenyl, oder

$R_6$ und $R_7$ gemeinsam mit dem Stickstoffatom, an das sie gebunden sind, einen durch 1 oder 2 Methylgruppen substituierten Piperidino-, Tetrahydropyrimidino-, Morpholino- oder Imidazolylrest bedeuten, oder X und Y zusammen einen Tetrahydrofuran-2-on-3-yl-Rest bilden.

**11.** Mittel nach Anspruch 10, dadurch gekennzeichnet, dass Y für $-COOR_4$ steht.

**12.** Mittel nach Anspruch 10, dadurch gekennzeichnet, dass Y für $-COSR_5$ steht.

**13.** Mittel nach Anspruch 10, dadurch gekennzeichnet, dass Y für $-CONR_6R_7$ steht.

**14.** Mittel nach Anspruch 10, dadurch gekennzeichnet, dass X und Y zusammen einen unsubstituierten Tetrahydrofuran-2-on-3-yl-Ring bilden.

**15.** Mittel nach Anspruch 10, dadurch gekennzeichnet, dass es die aktive Komponente zusammen mit Trägermaterial und/oder inerten Zuschlagstoffen enthält.

**16.** Mittel nach Anspruch 10, dadurch gekennzeichnet, dass es als aktive Komponente eine Verbindung wie im Anspruch 10 definiert, zusammen mit einem kulturpflanzenschädigenden Herbizid enthält.

**17.** Mittel nach Anspruch 16, dadurch gekennzeichnet, dass es als Herbizid einen Diphenyläther enthält.

**18.** Mittel nach Anspruch 16, dadurch gekennzeichnet, dass es als Herbizid einen substituierten Pyridyloxyphenoxypropionsäureester enthält.

**19.** Mittel nach Anspruch 18, dadurch gekennzeichnet, dass es als Herbizid 2-[4-(3,5-Dichlorpyridyl-2-oxy)-phenoxy]-propionsäure-2-propinylester enthält.

**20.** Verfahren zur Herstellung von Verbindungen nach Anspruch 10, dadurch gekennzeichnet, dass man eine Verbindung der Formel II

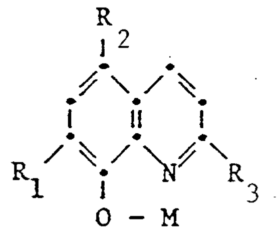

(II),

worin $R_1$, $R_2$ und $R_3$ die angegebenen Bedeutungen haben und M für Wasserstoff, ein Alkali- oder Erdalkalimetallatom steht, mit einer Verbindung der Formel III

Z - X - Y (III),

worin X und Y die angegebenen Bedeutungen haben und Z für einen abspaltbaren Rest steht, umsetzt,

**21.** Verfahren zur Herstellung von Verbindungen nach Anspruch 10, in denen Y für $-COOR_4$ steht, dadurch gekennzeichnet, dass man ein Säurehalogenid der Formel IV

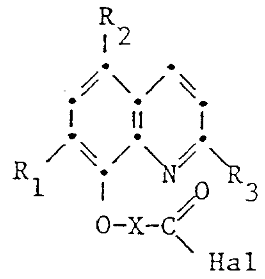

(IV),

worin $R_1$, $R_2$, $R_3$ und X die angegebenen Bedeutungen haben und Hal für ein Halogenatom steht, mit einer Verbindung der Formel V

M' - O - $R_4$ (V),

worin $R_4$ die angegebene Bedeutung hat und M' für Wasserstoff, ein Alkali- oder Erdalkalimetallatom steht, umsetzt.

**22.** Verfahren zur Herstellung von Verbindungen nach Anspruch 10, in denen Y für $-COSR_5$ steht, dadurch gekennzeichnet, dass man ein Säurehalogenid der Formel VI

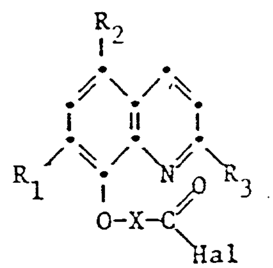

(VI),

worin $R_1$, $R_2$, $R_3$ und X die angegebenen Bedeutungen haben und Hal für ein Halogenatom steht, mit einer Verbindung der Formel VII

M" - S - $R_5$ (VII),

worin $R_5$ die angegebene Bedeutung hat und M" für Wasserstoff, ein Alkali- oder Erdalkalimetallatom steht, umsetzt.

**23.** Verfahren zur Herstellung von Verbindungen nach Anspruch 10, in denen Y für $-CONR_6R_7$ steht, dadurch gekennzeichnet, dass man ein Säurehalogenid der Formel VIII

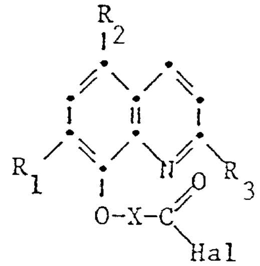

(VIII),

worin $R_1$, $R_2$, $R_3$ und X die angegebenen Bedeutungen haben und Hal für ein Halogenatom steht, mit einer Verbindung der Formel IX

$HNR_6R_7$ (IX),

worin $R_6$ und $R_7$ die angegobenen Bedeutungen haben, umsetzt.

**24.** Verfahren zur Herstellung von Verbindungen nach Anspruch 10, in denen Y für $-COOR_4$ steht, dadurch gekennzeichnet, dass man eine Verbindung der Formel X

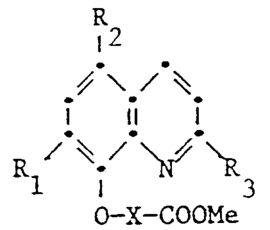

(X),

worin $R_1$, $R_2$, $R_3$ und X die angegebenen Bedeutungen haben und Me für ein Alkalimetall-, Erdalkalimetall-, Blei- oder Silberatom steht, mit einer Verbindung der Formel XI

Hal - $R_4$ (XI),

worin $R_4$ die angegebene Bedeutung hat und Hal für ein Halogenatom steht, umsetzt.

**25.** Verfahren zur Herstellung von Verbindungen nach Anspruch 10, in denen Y für $-COSR_5$ steht, dadurch gekennzeichnet, dass man eine Verbindung der Formel XII

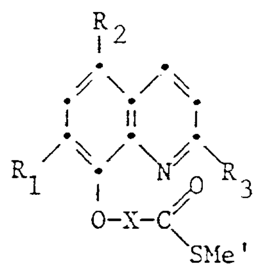

(XII),

worin $R_1$, $R_2$, $R_3$ und X die angegebenen Bedeutungen haben und Me' für ein Alkalimetall-, Erdalkalimetall-, Blei- oder Silberatom steht, mit einer Verbindung der Formel XIII

Hal - $R_5$ (XIII),

worin $R_5$ die angegebene Bedeutung hat und Hal für ein Halogenatom steht, umsetzt.

**26.** Verfahren zur Herstellung von Verbindungen nach Anspruch 10, worin Y für $-COOR_4$ steht, dadurch gekennzeichnet, dass man eine Verbindung der Formel XIV

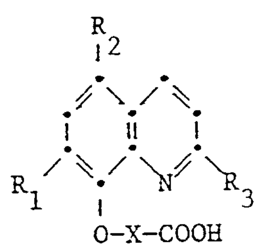

(XIV),

worin $R_1$, $R_2$, $R_3$ und X die angegebenen Bedeutungen haben, mit einer Verbindung der Formel XV

HO - $R_4$ (XV),

worin $R_4$ die angegebene Bedeutung hat, umsetzt.

**27.** Verfahren zur Herstellung von Verbindungen nach Anspruch 10, worin Y für $-COSR_5$ steht, dadurch gekennzeichnet, dass man eine Verbindung der Formel XVI

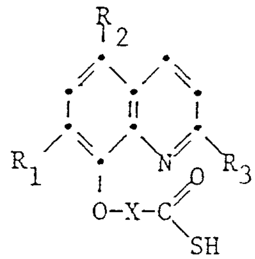

(XVI),

worin $R_1$, $R_2$, $R_3$ und X die angegebenen Bedeutungen haben, mit einer Verbindung der Formel XVII

$HO - R_5$ (XVII),

worin $R_5$ die angegebene Bedeutung hat, umsetzt.

**28.** Verfahren zur Herstellung von Verbindungen nach Anspruch 10, in denen Y für $-COOR_4$ steht, dadurch gekennzeichnet, dass man eine Verbindung der Formel Ia

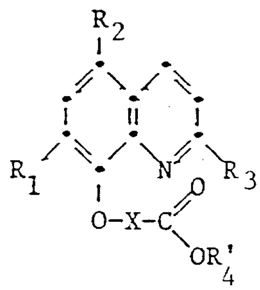

(Ia),

worin $R_1$, $R_2$, $R_3$ und X die angegebenen Bedeutungen haben und $R_4'$ die für $R_4$ angegebene Bedeutung hat, mit einer Verbindung der Formel XVIII

$HO - R_4''$ (XVIII),

worin $R_4''$ die für $R_4$ angegebene Bedeutung hat und nicht mit $R_4'$ identisch ist, umsetzt.

**29.** Verfahren zur Herstellung von Verbindungen nach Anspruch 10, in denen Y für $-COSR_5$ steht, dadurch gekennzeichnet, dass man eine Verbindung der Formel Ib

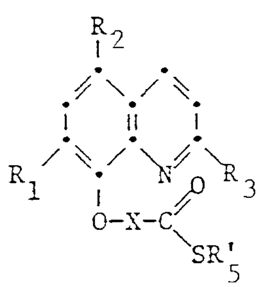

(Ib),

worin $R_1$, $R_2$, $R_3$ und X die angegebenen Bedeutungen haben und $R_5'$ die für $R_5$ angegebene

Bedeutung hat, mit einer Verbindung der Formel XIX

$HSR_5''$ (XIX),

worin $R_5''$ die für $R_5$ angegebene Bedeutung hat und nicht mit $R_5'$ identisch ist, umsetzt.

**30.** Verfahren zur Herstellung von Verbindungen nach Anspruch 10, worin Y für $-CONR_6R_7$ steht, dadurch gekennzeichnet, dass man eine Verbindung der Formel XX

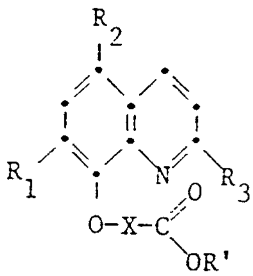

(XX),

worin $R_1$, $R_2$, $R_3$ und X die angegebenen Bedeutungen haben und R' für einen aliphatischen, acyclischen Kohlenwasserstoffrest steht, mit einer Verbindung der Formel XXI

$HNR_6R_7$ (XXI),

worin $R_6$ und $R_7$ die angegebenenen Bedeutungen haben, umsetzt.

**31.** Verfahren zur Herstellung von Verbindungen nach Anspruch 10, in denen Y für einen Oxazolin-2-yl-Rest steht, dadurch gekennzeichnet, dass man eine Verbindung der Formel XXIV

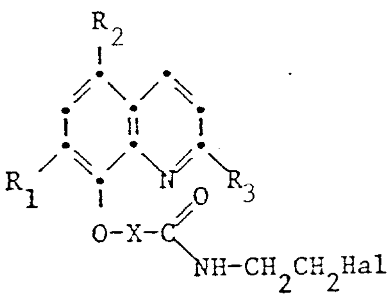

(XXIV),

worin $R_1$, $R_2$, $R_3$ und X die angegebenen Bedeutungen haben und Hal für ein Halogenatom, insbesondere ein Chlor- oder Bromatom steht, in Gegenwart eines säurebindenden Mittels cyclisiert.

**32.** Verfahren zur Herstellung von Verbindungen nach Anspruch 10, in denen Y für $-CH_2CH_2-$ steht, dadurch gekennzeichnet, dass man eine Verbindung der Formel XXII

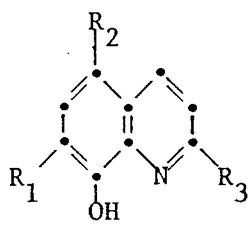

(XXII),

worin $R_1$, $R_2$ und $R_3$ die angegebenen Bedeutungen haben, mit einer Verbindung der Formel XXIII

$CH_2 = CH\text{-}Y$ (XXIII),

worin Y die angegebene Bedeutung hat, umsetzt.

**33.** Saatgut von Kulturhirse, Reis, Mais und Getreidearten, behandelt mit einem Chinolinderivat der Formel I wie im Anspruch 1 definiert.

## Claims

**Claims for the following Contracting States : BE, CH, DE, FR, IT, LI, NL, SE**

**1.** Use of a quinoline derivative of the formula I

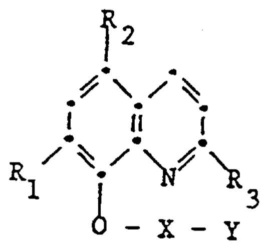

(I),

in which

$R_1$ is hydrogen, chlorine, iodine or bromine, $R_2$ is hydrogen, chlorine, iodine, bromine, nitro, methyl or ethyl, and $R_3$ is hydrogen or methyl, X is a methylene bridge which is unsubstituted or substituted by one or two methyl groups and Y is an oxazolin-2-yl radical, $-COOR_4$, $-COSR_5$ or $-CONR_6R_7$, in which

$R_4$
- hydrogen, an alkali metal cation or a quaternary ammonium cation selected from the group consisting of ammonium cation, trimethyl-, triethyl- and tri-(2-hydroxyethylene)-ammonium cation;
- $C_1$-$C_{18}$alkyl;
- $C_1$-$C_{10}$alkyl, which is substituted by 1 or 2 hydroxy groups;
- $C_1$-$C_4$alkyl, which is substituted by 1 to 3 chlorine or bromine atoms, a nitro group, a cyano group, a $C_1$-$C_4$alkoxy group, a $C_2$-$C_8$alkoxy group which is interrupted by 1 or 2 oxygen atoms, a hydroxy-$C_1$-$C_4$alkoxy group, a hydroxy-$C_2$-$C_6$alkoxy group, which is interrupted by 1 or 2 oxygen atoms, a $C_1$-$C_4$alkoxycarbonyl group, a di-($C_1$-$C_4$alkyl)-amino group, a phenyl radical which is unsubstituted or substituted by chlorine or methoxy, a cyclohexyl radical, a furan, tetrahydrofuran, tetrahydropyran, pyridino, piperidino or morpholino radical;
- $C_3$-$C_{10}$alkenyl;
- $C_3$-$C_4$alkynyl which is unsubstituted or substituted by a hydroxy group;
- cyclohexyl which is unsubstituted or substituted by 1 or 2 methyl groups;
- phenyl which is unsubstituted or substituted by 1 to 2 substituents from the group consisting of chlorine, nitro, $C_1$-$C_4$alkyl or methoxy;
- naphthyl; or
- pyridine,

$R_5$
- $C_1$-$C_{12}$alkyl;
- $C_1$-$C_4$alkyl which is substituted by a $C_1$-$C_4$alkoxycarbonyl group;
- phenyl which is unsubstituted or substituted by a chlorine atom,

$R_6$
- hydrogen;
- $C_1$-$C_{18}$alkyl;
- $C_2$-$C_8$alkyl, which is substituted by an amino group;
- $C_2$-$C_6$alkyl, which is substituted by a hydroxy group;
- $C_2$-$C_4$alkyl, which is substituted by a chlorine or bromine atom, a $C_1$-$C_4$alkylamino group, a di-($C_1$-$C_4$alkyl)-amino group, a $C_1$-$C_4$hydroxyalkylamino group, a di-($C_1$-$C_3$hydroxyalkyl)-amino group, a $C_1$-$C_4$alkoxy group or a $C_1$-$C_4$alkoxycarbonyl group;
- $C_1$-$C_4$alkyl which is substituted by a cyano group or a phenyl radical which is unsubstituted or substituted by chlorine, a furanyl, tetrahydrofuranyl, piperidino or morpholino radical;
- $C_1$-$C_3$alkoxy;
- $C_3$-$C_4$alkenyl;

- cyclohexyl which is unsubstituted or substituted by a methyl group;
- phenyl which is unsubstituted or substituted by 1 or 2 substituents from the group consisting of chlorine, nitro, cyano, methyl, ethyl, methoxy and trifluoromethyl, and

$R_7$ - hydrogen;
- $C_1$-$C_6$ alkyl;
- $C_1$-$C_4$ alkyl which is substituted by a hydroxy group or a cyano group;
- $C_2$-$C_4$ alkoxyalkyl;
- $C_3$-$C_4$ alkenyl;
- Cyclohexyl; or
- phenyl, or

$R_6$ and $R_7$ together with the nitrogen atom to which they are bonded, are a piperidino, tetrahydropyrimidino, morpholino or imidazolyl radical which is unsubstituted or substituted by 1 or 2 methyl groups, or X and Y together form a tetrahydrofuran-2-on-3-yl ring; for the protection of cultivated millet, rice, maize and cereals from harmful effects of herbicides.

**2.** Use according to claim 1, wherein, in the compound of the formula I, Y is $-COOR_4$.

**3.** Use according to claim 1, wherein, in the compound of the formula I, Y is $-COSR_5$.

**4.** Use according to claim 1, wherein, in the compound of the formula I, Y is $-CONR_6R_7$.

**5.** Use according to claim 1, wherein, in the compound of the formula I, X and Y together form a tetrahydrofuran-2-on-3-yl ring.

**6.** Use according to claim 1, for protecting cultivated millet, rice, maize and cereals from the harmful effects of diphenyl ethers.

**7.** Use according to claim 1, for protecting cultivated millet, rice, maize and cereals from the harmful effects of substituted pyridyloxyphenoxypropionic acid esters.

**8.** Use according to claim 7, for protecting cultivated millet, rice, maize and cereals from the harmful effects of 2-propynyl 2-[4-(3,5-dichloropyridyl-2-oxy)-phenoxy]-propionate.

**9.** Use according to claim 1, for protecting cereals.

**10.** A compound of the formula I

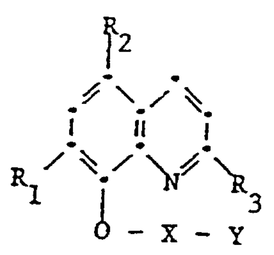

(I),

in which

$R_1$ is hydrogen, chlorine, iodine or bromine, $R_2$ is hydrogen, chlorine, iodine, bromine, nitro, methyl or ethyl, and $R_3$ is hydrogen or methyl, X is a methylene bridge which is unsubstituted or substituted by one or two methyl groups and Y is an oxazolin-2-yl radical, $-COOR_4$, $-COSR_5$ or $-CONR_6R_7$, in which

$R_4$ - a quaternary ammonium cation selected from the group consisting of trimethyl-, triethyl- and tri-(2-hydroxyethylene)-ammonium cation;
- alkyl having more than 5 carbon atoms but not more than 18 carbon atoms;
- $C_1$-$C_{10}$ alkyl, which is substituted by 1 or 2 hydroxy groups;
- $C_1$-$C_4$ alkyl, which is substituted by 1 to 3 chlorine or bromine atoms, a nitro group, a cyano group, a $C_1$-$C_4$ alkoxy group, a $C_2$-$C_8$ alkoxy group which is interrupted by 1 or 2 oxygen atoms, a hydroxy-$C_1$-$C_4$ alkoxy group, a hydroxy-$C_2$-$C_6$ alkoxy group, which is interrupted by 1

or 2 oxygen atoms, a $C_1$-$C_4$alkoxycarbonyl group, a di-($C_1$-$C_4$alkyl)-amino group, a phenyl radical which is unsubstituted or substituted by chlorine or methoxy, a cyclohexyl radical, a furan, tetrahydrofuran, tetrahydropyran, pyridino, piperidino or morpholino radical;
- $C_3$-$C_{10}$alkenyl;
- $C_3$-$C_4$alkynyl which is unsubstituted or substituted by a hydroxy group;
- cyclohexyl which is unsubstituted or substituted by 1 or 2 methyl groups;
- phenyl which is unsubstituted or substituted by 1 to 2 substituents from the group consisting of chlorine, nitro, $C_1$-$C_4$alkyl or methoxy;
- naphthyl; or
- pyridine,

$R_5$
- $C_1$-$C_{12}$alkyl;
- $C_1$-$C_4$alkyl which is substituted by a $C_1$-$C_4$alkoxycarbonyl group;
- phenyl which is unsubstituted or substituted by a chlorine atom,

$R_6$
- alkyl having more than 6 carbon atoms but not more than 18 carbon atoms;
- $C_2$-$C_8$alkyl, which is substituted by an amino group;
- $C_2$-$C_6$alkyl, which is substituted by a hydroxy group;
- $C_2$-$C_4$alkyl, which is substituted by a chlorine or bromine atom, a $C_1$-$C_4$hydroxyalkylamino group, a di- ($C_1$-$C_3$hydroxylalkyl) -amino group or a $C_1$-$C_4$alkoxycarbonyl group;
- $C_1$-$C_4$alkyl which is substituted by a cyano group or a phenyl radical which is unsubstituted or substituted by chlorine, a furanyl, tetrahydrofuranyl, piperidino or morpholino radical;
- $C_1$-$C_3$alkoxy;
- cyclohexyl which is substituted by a methyl group;
- phenyl which is substituted by 1 or 2 substituents from the group consisting of nitro, cyano, methyl, ethyl, methoxy and trifluoromethyl, and

$R_7$
- hydrogen;
- $C_1$-$C_6$alkyl;
- $C_1$-$C_4$alkyl which is substituted by a hydroxy group or a cyano group;
- $C_2$-$C_4$alkoxyalkyl;
- $C_3$-$C_4$alkenyl;
- Cyclohexyl; or
- phenyl, or

$R_6$ and $R_7$, together with the nitrogen atom to which they are bonded, are a piperidino, tetrahydropyrimidino, morpholino or imidazolyl radical which is substituted by 1 or 2 methyl groups, or X and Y together form a tetrahydrofuran-2-on-3-yl radical.

**11.** A compound according to claim 10, wherein Y is -$COOR_4$.

**12.** A compound according to claim 10, wherein Y is -$COSR_5$.

**13.** A compound according to claim 10, wherein Y is -$CONR_6R_7$.

**14.** A compound according to claim 10, wherein X and Y together are an unsubstituted tetrahydrofuran-2-on-3-yl ring.

**15.** A composition for protecting cultivated millet, rice, maize and cereals from the harmful effects of herbicides on cultivated plants, which contains, as the active component, a compound according to claim 10.

**16.** A composition according to claim 15, which contains the active component together with a carrier and/or inert adjuvants.

**17.** A composition according to claim 15, which contains, as the active component, a compound as defined in claim 10, together with a herbicide which harms cultivated plants.

**18.** A composition according to claim 17, which contains, as the herbicide, a diphenyl ether.

**19.** A composition according to claim 17, which contains, as the herbicide, a substituted pyridyloxyphenoxypropionic acid ester.

**20.** A composition according to claim 19, which contains, as the herbicide, 2-propynyl 2-[4-(3,5-dichloropyridyl-2-oxy)-phenoxy]-propionate.

**21.** A process for the preparation of a compound according to claim 10, which comprises reacting a compound of the formula II

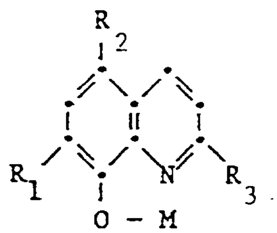

(II),

in which $R_1$, $R_2$ and $R_3$ are as defined above and M is hydrogen or an alkali metal or alkaline earth metal atom, with a compound of the formula III

Z-X-Y (III)

in which X and Y are as defined above and Z is a detachable radical.

**22.** A process for the preparation of a compound according to claim 10, in which Y is $-COOR_4$, which comprises reacting an acid halide of the formula IV

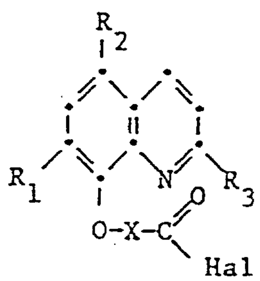

(IV),

in which $R_1$, $R_2$, $R_3$ and X are as defined above and Hal is a halogen atom, with a compound of the formula V

$M'-O-R_4$ (V)

in which $R_4$ is as defined above and M' is hydrogen or an alkali metal or alkaline earth metal atom.

**23.** A process for the preparation of a compound according to claim 10, in which Y is $-COSR_5$, which comprises reacting an acid halide of the formula VI

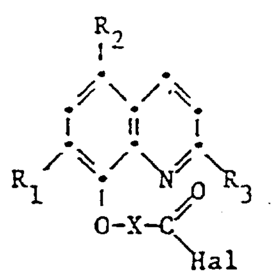

(VI),

in which $R_1$, $R_2$, $R_3$ and X are as defined above and Hal is a halogen atom, with a compound of the formula VII

M''-S-$R_5$ (VII)

in which $R_5$ is as defined above and M'' is hydrogen or an alkali metal or alkaline earth metal atom.

**24.** A process for the preparation of a compound according to claim 10, in which Y is -CONR$_6$R$_7$, which comprises reacting an acid halide of the formula VIII

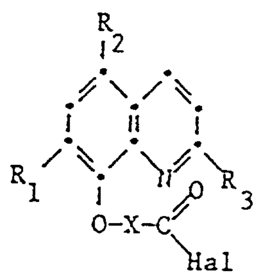

(VIII),

in which $R_1$, $R_2$, $R_3$ and X are as defined above and Hal is a halogen atom, with a compound of the formula IX

HNR$_6$R$_7$ (IX)

in which $R_6$ and $R_7$ are as defined above.

**25.** A process for the preparation of a compound according to claim 10, in which Y is -COOR$_4$, which comprises reacting a compound of the formula X

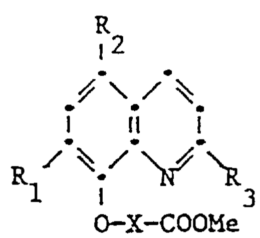

(X),

in which $R_1$, $R_2$, $R_3$ and X are as defined above and Me is an alkali metal, alkaline earth metal, lead or silver atom, with a compound of the formula XI

Hal-$R_4$ (XI)

in which $R_4$ is as defined above and Hal is a halogen atom.

**26.** A process for the preparation of a compound according to claim 10, in which Y is -COSR$_5$, which comprises reacting a compound of the formula XII

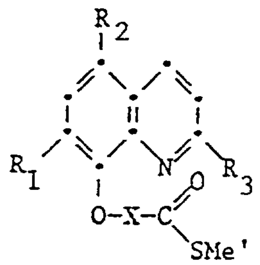

(XII),

in which $R_1$, $R_2$, $R_3$ and X are as defined above and Me' is an alkali metal, alkaline earth metal, lead or silver atom, with a compound of the formula XIII

Hal-$R_5$ (XIII)

in which $R_5$ is as defined above and Hal is a halogen atom.

**27.** A process for the preparation of a compound according to claim 10, in which Y is $-COOR_4$, which comprises reacting a compound of the formula XIV

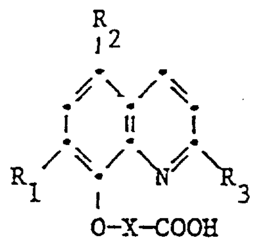

(XIV),

in which $R_1$, $R_2$, $R_3$ and X are as defined above, with a compound of the formula XV

HO-$R_4$ (XV)

in which $R_4$ is as defined above.

**28.** A process for the preparation of a compound according to claim 10, in which Y is $-COSR_5$, which comprises reacting a compound of the formula XVI

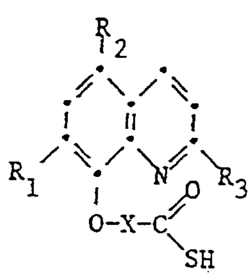

(XVI),

in which $R_1$, $R_2$, $R_3$ and X are as defined above, with a compound of the formula XVII

HO-$R_5$ (XVII)

in which $R_5$ is as defined above.

**29.** A process for the preparation of a compound according to claim 10, in which Y is $-COOR_4$, which comprises reacting a compound of the formula Ia

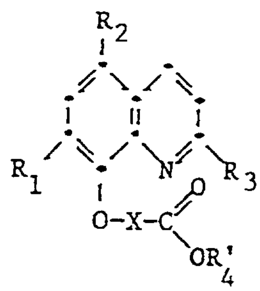

(Ia),

in which $R_1$, $R_2$, $R_3$ and X are as defined above and $R_4$ is as defined for $R_4$, with a compound of the formula XVIII

$HO-R_4''$ (XVIII)

in which $R_4''$ is as defined for $R_4$ but is not identical to $R_4'$.

**30.** A process for the preparation of a compound according to claim 10, in which Y is $-COSR_5$, which comprises reacting a compound of the formula Ib

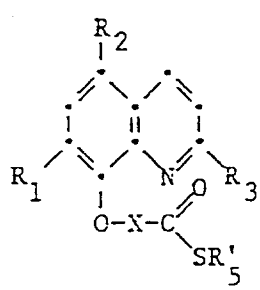

(Ib),

in which $R_1$, $R_2$, $R_3$ and X are as defined above and $R_5'$ is as defined for $R_5$, with a compound of the formula XIX

$HSR_5''$ (XIX)

in which $R_5''$ is as defined for $R_5$ but is not identical to $R_5'$.

**31.** A process for the preparation of a compound according to claim 10, in which Y is $-CONR_6R_7$, which comprises reacting a compound of the formula XX

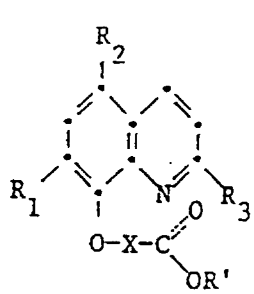

(XX),

in which $R_1$, $R_2$, $R_3$ and X are as defined above and R' is an aliphatic, acyclic hydrocarbon radical, with

a compound of the formula XXI

$HNR_6R_7$ (XXI)

in which $R_6$ and $R_7$ are as defined above.

**32.** A process for the preparation of a compound according to claim 10, in which Y is an oxazolin-2-yl radical, which comprises cyclising a compound of the formula XXIV

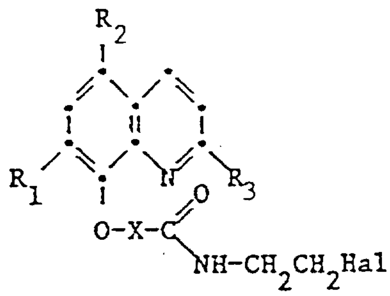

(XXIV),

in which $R_1$, $R_3$, $R_3$ and X are as defined above and Hal is a halogen atom, in particular a chlorine or bromine atom, in the presence of an acid-binding agent.

**33.** A process for the preparation of a compound according to claim 10, in which Y is $-CH_2-CH_2-$, which comprises reacting a compound of the formula XXII

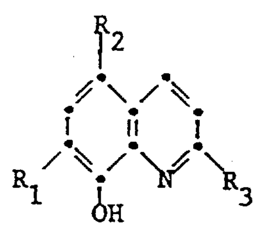

(XXII),

in which $R_1$, $R_2$ and $R_3$ are as defined above, with a compound of the formula XXIII

$CH_2=CH-Y$ (XXIII)

in which Y is as defined above.

**34.** Seed of cultivated millet, rice, maize and cereals treated with a quinoline derivative of the formula I as defined in claim 1.

**Claims for the following Contracting State : AT**

**1.** Use of a quinoline derivative of the formula I

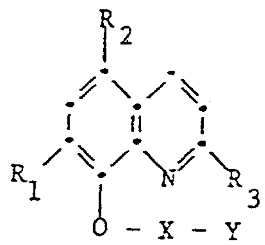

(I),

in which
$R_1$ is hydrogen, chlorine, iodine or bromine, $R_2$ is hydrogen, chlorine, iodine, bromine, nitro, methyl or ethyl, and $R_3$ is hydrogen or methyl, X is a methylene bridge which is unsubstituted or substituted by one or two methyl groups and Y is an oxazolin-2-yl radical, $-COOR_4$, $-COSR_5$ or $-CONR_6R_7$, in which

$R_4$
- hydrogen, an alkali metal cation or a quaternary ammonium cation selected from the group consisting of ammonium cation, trimethyl-, triethyl- and tri-(2-hydroxyethylene)-ammonium cation;
- $C_1$-$C_{18}$alkyl;
- $C_1$-$C_{10}$alkyl, which is substituted by 1 or 2 hydroxy groups;
- $C_1$-$C_4$alkyl, which is substituted by 1 to 3 chlorine or bromine atoms, a nitro group, a cyano group, a $C_1$-$C_4$alkoxy group, a $C_2$-$C_8$alkoxy group which is interrupted by 1 or 2 oxygen atoms, a hydroxy-$C_1$-$C_4$alkoxy group, a hydroxy-$C_2$-$C_6$alkoxy group, which is interrupted by 1 or 2 oxygen atoms, a $C_1$-$C_4$alkoxycarbonyl group, a di-($C_1$-$C_4$alkyl)-amino group, a phenyl radical which is unsubstituted or substituted by chlorine or methoxy, a cyclohexyl radical, a furan, tetrahydrofuran, tetrahydropyran, pyridino, piperidino or morpholino radical;
- $C_3$-$C_{10}$alkenyl;
- $C_3$-$C_4$alkynyl which is unsubstituted or substituted by a hydroxy group;
- cyclohexyl which is unsubstituted or substituted by 1 or 2 methyl groups;
- phenyl which is unsubstituted or substituted by 1 to 2 substituents from the group consisting of chlorine, nitro, $C_1$-$C_4$alkyl or methoxy;
- naphthyl; or
- pyridine,

$R_5$
- $C_1$-$C_{12}$alkyl;
- $C_1$-$C_4$alkyl which is substituted by a $C_1$-$C_4$alkoxycarbonyl group;
- phenyl which is unsubstituted or substituted by a chlorine atom,

$R_6$
- hydrogen;
- $C_1$-$C_{18}$alkyl;
- $C_2$-$C_8$alkyl, which is substituted by an amino group;
- $C_2$-$C_6$alkyl, which is substituted by a hydroxy group;
- $C_2$-$C_4$alkyl, which is substituted by a chlorine or bromine atom, a $C_1$-$C_4$alkylamino group, a di-($C_1$-$C_4$alkyl)-amino group, a $C_1$-$C_4$hydroxyalkylamino group, a di-($C_1$-$C_3$hydroxyalkyl)-amino group, a $C_1$-$C_4$alkoxy group or a $C_1$-$C_4$alkoxycarbonyl group;
- $C_1$-$C_4$alkyl which is substituted by a cyano group or a phenyl radical which is unsubstituted or substituted by chlorine, a furanyl, tetrahydrofuranyl, piperidino or morpholino radical;
- $C_1$-$C_3$alkoxy;
- $C_3$-$C_4$alkenyl;
- cyclohexyl which is unsubstituted or substituted by a methyl group;
- phenyl which is unsubstituted or substituted by 1 or 2 substituents from the group consisting of chlorine, nitro, cyano, methyl, ethyl, methoxy and trifluoromethyl, and

$R_7$
- hydrogen;
- $C_1$-$C_6$alkyl;
- $C_1$-$C_4$alkyl which is substituted by a hydroxy group or a cyano group;
- $C_2$-$C_4$alkoxyalkyl;

- $C_3$-$C_4$ alkenyl;
- Cyclohexyl; or
- phenyl, or

$R_6$ and $R_7$, together with the nitrogen atom to which they are bonded, are a piperidino, tetrahydropyrimidino, morpholino or imidazolyl radical which is unsubstituted or substituted by 1 or 2 methyl groups, or X and Y together form a tetrahydrofuran-2-on-3-yl ring; for the protection of cultivated millet, rice, maize and cereals from the harmful effects of herbicides on cultivated plants.

**2.** Use according to claim 1, wherein, in the compound of the formula I, Y is $-COOR_4$.

**3.** Use according to claim 1, wherein, in the compound of the formula I, Y is $-COSR_5$.

**4.** Use according to claim 1, wherein, in the compound of the formula I, Y is $-CONR_6R_7$.

**5.** Use according to claim 1, wherein, in the compound of the formula I, X and Y together form a tetrahydrofuran-2-on-3-yl ring.

**6.** Use according to claim 1, for protecting cultivated millet, rice, maize and cereals from the harmful effects of diphenyl ethers.

**7.** Use according to claim 1, for protecting cultivated millet, rice, maize and cereals from the harmful effects of substituted pyridyloxyphenoxypropionic acid esters.

**8.** Use according to claim 7, for protecting cultivated millet, rice, maize and cereals from the harmful effects of 2-propynyl 2-[4-(3,5-dichloropyridyl-2-oxy)-phenoxy]-propionate.

**9.** Use according to claim 1, for protecting cereals.

**10.** A composition for protecting cultivated millet, rice, maize and cereals from the harmful effects of herbicides on cultivated plants, which contains, as the active component, a compound of the formula I

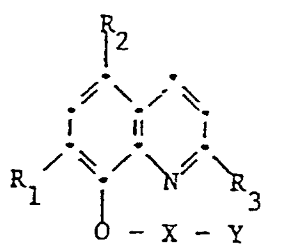

(I),

in which

$R_1$ is hydrogen, chlorine, iodine or bromine, $R_2$ is hydrogen, chlorine, iodine, bromine, nitro, methyl or ethyl, and $R_3$ is hydrogen or methyl, X is a methylene bridge which is unsubstituted or substituted by one or two methyl groups and Y is an oxazolin-2-yl radical, $-COOR_4$, $-COSR_5$ or $-CONR_6R_7$, in which

$R_4$
- a quaternary ammonium cation selected from the group consisting of trimethyl-, triethyl- and tri-(2-hydroxyethylene)-ammonium cation;
- alkyl having more than 5 carbon atoms but not more than 18 carbon atoms;
- $C_1$-$C_{10}$ alkyl, which is substituted by 1 or 2 hydroxy groups;
- $C_1$-$C_4$ alkyl, which is substituted by 1 to 3 chlorine or bromine atoms, a nitro group, a cyano group, a $C_1$-$C_4$ alkoxy group, a $C_2$-$C_8$ alkoxy group which is interrupted by 1 or 2 oxygen atoms, a hydroxy-$C_1$-$C_4$ alkoxy group, a hydroxy-$C_2$-$C_6$ alkoxy group, which is interrupted by 1 or 2 oxygen atoms, a $C_1$-$C_4$ alkoxycarbonyl group, a di-($C_1$-$C_4$ alkyl)-amino group, a phenyl radical which is unsubstituted or substituted by chlorine or methoxy, a cyclohexyl radical, a furan, tetrahydrofuran, tetrahydropyran, pyridino, piperidino or morpholino radical;
- $C_3$-$C_{10}$ alkenyl;
- $C_3$-$C_4$ alkynyl which is unsubstituted or substituted by a hydroxy group;

- cyclohexyl which is unsubstituted or substituted by 1 or 2 methyl groups;
- phenyl which is unsubstituted or substituted by 1 to 2 substituents from the group consisting of chlorine, nitro, $C_1$-$C_4$alkyl or methoxy;
- naphthyl; or
- pyridine,

$R_5$
- $C_1$-$C_{12}$alkyl;
- $C_1$-$C_4$alkyl which is substituted by a $C_1$-$C_4$alkoxycarbonyl group;
- phenyl which is unsubstituted or substituted by a chlorine atom,

$R_6$
- alkyl having more than 6 carbon atoms but not more than 18 carbon atoms;
- $C_2$-$C_8$alkyl, which is substituted by an amino group;
- $C_2$-$C_6$alkyl, which is substituted by a hydroxy group;
- $C_2$-$C_4$alkyl, which is substituted by a chlorine or bromine atom, a $C_1$-$C_4$hydroxyalkylamino group, a di-($C_1$-$C_3$hydroxylalkyl) -amino group or a $C_1$-$C_4$alkoxycarbonyl group;
- $C_1$-$C_4$alkyl which is substituted by a cyano group or a phenyl radical which is unsubstituted or substituted by chlorine, a furanyl, tetrahydrofuranyl, piperidino or morpholino radical;
- $C_1$-$C_3$alkoxy;
- cyclohexyl which is substituted by a methyl group;
- phenyl which is substituted by 1 or 2 substituents from the group consisting of nitro, cyano, methyl, ethyl, methoxy and trifluoromethyl, and

$R_7$
- hydrogen;
- $C_1$-$C_6$alkyl;
- $C_1$-$C_4$alkyl which is substituted by a hydroxy group or a cyano group;
- $C_2$-$C_4$alkoxyalkyl;
- $C_3$-$C_4$alkenyl;
- Cyclohexyl; or
- phenyl, or

$R_6$ and $R_7$, together with the nitrogen atom to which they are bonded, are a piperidino, tetrahydropyrimidino, morpholino or imidazolyl radical which is substituted by 1 or 2 methyl groups, or X and Y together form a tetrahydrofuran-2-on-3-yl radical.

**11.** A composition according to claim 10, wherein Y is $-COOR_4$.

**12.** A composition according to claim 10, wherein Y is $-COSR_5$.

**13.** A composition according to claim 10, wherein Y is $-CONR_6R_7$.

**14.** A composition according to claim 10, wherein X and Y together are an unsubstituted tetrahydrofuran-2-on-3-yl ring.

**15.** A composition according to claim 10, which contains the active component together with a carrier and/or inert adjuvants.

**16.** A composition according to claim 10, which contains, as the active component, a compound as defined in claim 10, together with a herbicide which harms cultivated plants.

**17.** A composition according to claim 16, which contains, as the herbicide, a diphenyl ether.

**18.** A composition according to claim 16, which contains, as the herbicide, a substituted pyridyloxyphenoxypropionic acid ester.

**19.** A composition according to claim 18, which contains, as the herbicide, 2-propynyl 2-[4-(3,5-dichloropyridyl-2-oxy)-phenoxy]-propionate.

**20.** A process for the preparation of a compound according to claim 10, which comprises reacting a compound of the formula II

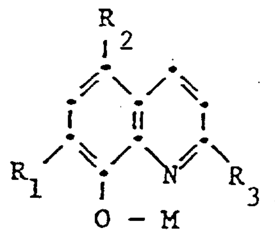

(II),

in which $R_1$, $R_2$ and $R_3$ are as defined above and M is hydrogen or an alkali metal or alkaline earth metal atom, with a compound of the formula III

Z-X-Y (III)

in which X and Y are as defined above and Z is a detachable radical.

**21.** A process for the preparation of a compound according to claim 10, in which Y is $-COOR_4$, which comprises reacting an acid halide of the formula IV

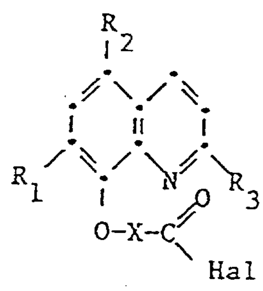

(IV),

in which $R_1$, $R_2$, $R_3$ and X are as defined above and Hal is a halogen atom-, with a compound of the formula V

$M'-O-R_4$ (V)

in which $R_4$ is as defined above and M' is hydrogen or an alkali metal or alkaline earth metal atom.

**22.** A process for the preparation of a compound according to claim 10, in which Y is $-COSR_5$, which comprises reacting an acid halide of the formula VI

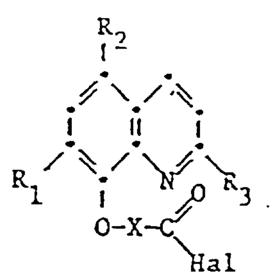

(VI),

in which $R_1$, $R_2$, $R_3$ and X are as defined above and Hal is a halogen atom, with a compound of the formula VII

$M''-S-R_5$ (VII)

in which $R_5$ is as defined above and M'' is hydrogen or an alkali metal or alkaline earth metal atom.

**23.** A process for the preparation of a compound according to claim 10, in which Y is $-CONR_6R_7$, which comprises reacting an acid halide of the formula VIII

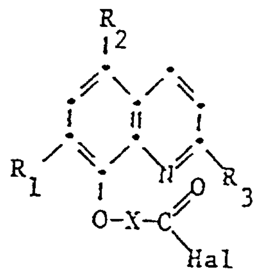

(VIII),

in which $R_1$, $R_2$, $R_3$ and X are as defined above and Hal is a halogen atom, with a compound of the formula IX

$HNR_6R_7$ (IX)

in which $R_6$ and $R_7$ are as defined above.

**24.** A process for the preparation of a compound according to claim 10, in which Y is $-COOR_4$, which comprises reacting a compound of the formula X

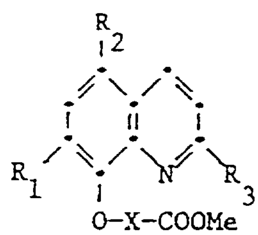

(X),

in which $R_1$, $R_2$, $R_3$ and X are as defined above, and Me is an alkali metal, alkaline earth metal, lead or silver atom, with a compound of the formula XI

Hal-$R_4$ (XI)

in which $R_4$ is as defined above and Hal is a halogen atom.

**25.** A process for the preparation of a compound according to claim 10, in which Y is $-COSR_5$, which comprises reacting a compound of the formula XII

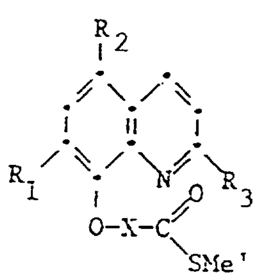

(XII),

in which $R_1$, $R_2$, $R_3$ and X are as defined above and Me' is an alkali metal, alkaline earth metal, lead or silver atom, with a compound of the formula XIII

Hal-$R_5$ (XIII)

in which $R_5$ is as defined above and Hal is a halogen atom.

**26.** A process for the preparation of a compound according to claim 10, in which Y is $-COOR_4$, which comprises reacting a compound of the formula XIV

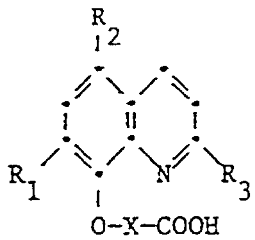

(XIV),

in which $R_1$, $R_2$, $R_3$ and X are as defined above, with a compound of the formula XV

$HO-R_4$ (XV)

in which $R_4$ is as defined above.

**27.** A process for the preparation of a compound according to claim 10, in which Y is $-COSR_5$, which comprises reacting a compound of the formula XVI

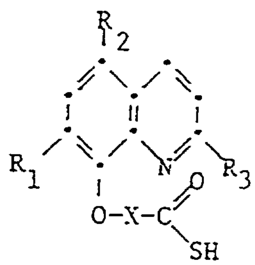

(XVI),

in which $R_1$, $R_2$, $R_3$ and X are as defined above, with a compound of the formula XVII

$HO-R_5$ (XVII)

in which $R_5$ is as defined above.

**28.** A process for the preparation of a compound according to claim 10, in which Y is $-COOR_4$, which comprises reacting a compound of the formula Ia

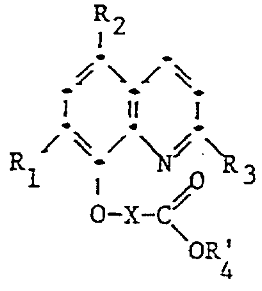

(Ia),

in which $R_1$, $R_2$, $R_3$ and X are as defined above and $R_4'$ is as defined for $R_4$, with a compound of the formula XVIII

$HO-R_4''$ (XVIII)

in which $R_4''$ is as defined for $R_4$ but is not identical to $R_4'$.

**29.** A process for the preparation of a compound according to claim 10, in which Y is $-COSR_5$, which comprises reacting a compound of the formula Ib

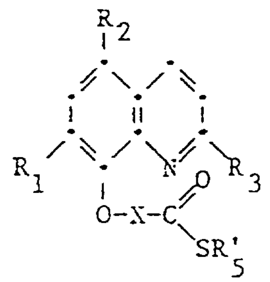

(Ib),

in which $R_1$, $R_2$, $R_3$ and X are as defined above and $R_5'$ is as defined for $R_5$, with a compound of the formula XIX

$HSR_5''$ (XIX)

in which $R_5''$ is as defined for $R_5$ but is not identical to $R_5'$.

**30.** A process for the preparation of a compound according to claim 10, in which Y is $-CONR_6R_7$, which comprises reacting a compound of the formula XX

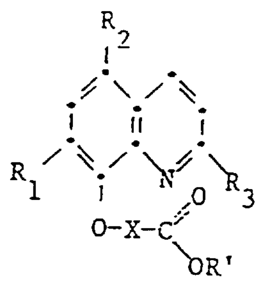

(XX),

in which $R_1$, $R_2$, $R_3$ and X are as defined above and R' is an aliphatic, acyclic hydrocarbon radical, with a compound of the formula XXI

$HNR_6R_7$ (XXI)

in which $R_6$ and $R_7$ are as defined above.

**31.** A process for the preparation of a compound according to claim 10, in which Y is an oxazolin-2-yl radical, which comprises cyclising a compound of the formula XXIV

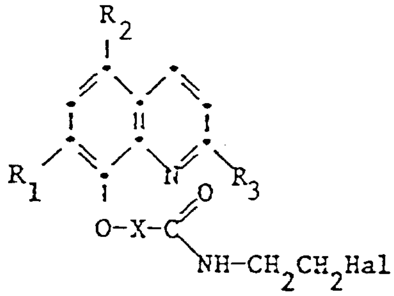

(XXIV),

in which $R_1$, $R_2$, $R_3$ and X are as defined above and Hal is a halogen atom, in particular a chlorine or bromine atom, in the presence of an acid-binding agent.

**32.** A process for the preparation of a compound according to claim 10, in which Y is $-CH_2-CH_2-$, which comprises reacting a compound of the formula XXII

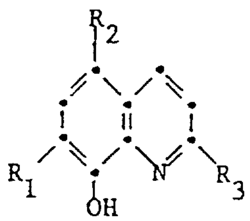

(XXII),

in which $R_1$, $R_2$ and $R_3$ are as defined above, with a compound of the formula XXIII

$CH_2 = CH-Y$ (XXIII)

in which Y is as defined above.

**33.** Seed of cultivated millet, rice, maize and cereals treated with a quinoline derivative of the formula I as defined in claim 1.

**Revendications**

**Revendications pour les Etats contractants suivants : BE, CH, DE, FR, IT, LI, NL, SE**

**1.** Utilisation de dérivés de la quinoléine de formule I

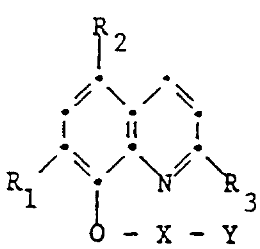

(I),

dans laquelle

$R_1$ représente l'hydrogène, le chlore, l'iode ou le brome, $R_2$ représente l'hydrogène, le chlore, l'iode, le brome, un groupe nitro, méthyle ou éthyle, et $R_3$ représente l'hydrogène ou un groupe méthyle, X représente un pont méthylène éventuellement substitué par un ou deux groupes méthyle et Y représente un groupe oxazoline-2-yle, $-COOR_4$, $-COSR_5$ ou $-CONR_6R_7$ dans lesquels

$R_4$ représente

- l'hydrogène, un cation de métal alcalin ou un cation d'ammonium quaternaire choisi parmi les cations ammonium, triméthyl-, triéthyl- et tri-(2-hydroxyéthylène)-ammonium;
- un groupe alkyle en C1-C18;
- un groupe alkyle en C1-C10 substitué par un ou deux groupes hydroxy;
- un groupe alkyle en C1-C4 substitué par un à trois atomes de chlore ou de brome, un groupe nitro, un groupe cyano, un groupe alcoxy en C1-C4, un groupe alcoxy en C2-C8 interrompu par un ou deux atomes d'oxygène, un groupe hydroxyalcoxy en C1-C4, un groupe hydroxyalcoxy en C2-C6 interrompu par un ou deux atomes d'oxygène, un groupe (alcoxy en C1-C4)-carbonyle, un groupe di-(alkyle en C1-C4)-amino, un groupe phényle non substitué ou substitué par le chlore ou des groupes méthoxy, un groupe cyclohexyle, un radical de furanne, de tétrahydrofuranne, de tétrahydropyranne, de pyridine, de pipéridine ou de morpholine;
- un groupe alcényle en C3-C10;
- un groupe alcynyle en C3-C4 non substitué ou substitué par un groupe hydroxy;
- un groupe cyclohexyle non substitué ou substitué par un ou deux groupes méthyle;
- un groupe phényle non substitué ou portant un ou deux substituants choisis parmi le chlore, les groupes nitro, alkyle en C1-C4 ou méthoxy;
- un groupe naphtyle; ou bien
- la pyridine,

$R_5$ représente
- un groupe alkyle en C1-C12;
- un groupe alkyle en C1-C4 substitué par un groupe (alcoxy en C1-C4)-carbonyle;
- un groupe phényle non substitué ou substitué par un atome de chlore;

$R_6$ représente
- l'hydrogène;
- un groupe alkyle en C1-C18;
- un groupe alkyle en C2-C8 substitué par un groupe amino;
- un groupe alkyle en C2-C6 substitué par un groupe hydroxy;
- un groupe alkyle en C2-C4 substitué par un atome de chlore ou de brome, un groupe alkylamino en C1-C4, un groupe di-(alkyle en C1-C4)-amino, un groupe hydroxyalkylamino en C1-C4, un groupe di-(hydroxyalkyle en C1-C3)-amino, un groupe alcoxy en C1-C4 ou un groupe (alcoxy en C1-C4)-carbonyle;
- un groupe alkyle en C1-C4 substitué par un groupe cyano ou par un groupe phényle non substitué ou substitué par le chlore, par un groupe furannyle, tétrahydrofurannyle, pipéridino ou morpholino;
- un groupe alcoxy en C1-C3;
- un groupe alcényle en C3-C4;
- un groupe cyclohexyle non substitué ou substitué par un groupe méthyle;
- un groupe phényle non substitué ou portant un ou deux substituants choisis parmi le chlore, les groupes nitro, cyano, méthyle, éthyle, méthoxy et trifluorométhyle; et

$R_7$ représente
- l'hydrogène;
- un groupe alkyle en C1-C6;
- un groupe alkyle en C1-C4 substitué par un groupe hydroxy ou un groupe cyano;
- un groupe alcoxyalkyle en C2-C4;
- un groupe alcényle en C3-C4;
- un groupe cyclohexyle; ou bien
- un groupe phényle, ou bien

$R_6$ et $R_7$ représentent, ensemble et avec l'atome d'azote auquel ils sont reliés, un groupe pipéridino, tétrahydropyrimidino, morpholino ou imidazolyle non substitué ou substitué par un ou deux groupes méthyle, ou bien X et Y forment ensemble un cycle tétrahydrofuranne-2-one-3-yle; pour la protection du millet cultivé, du riz, du maïs et des céréales contre les effets nocifs des herbicides sur les végétaux cultivés.

**2.** Utilisation selon revendication 1, caractérisée en ce que, dans le composé de formule I, Y représente $-COOR_4$.

**3.** Utilisation selon revendication 1, caractérisée en ce que, dans le composé de formule I, Y représente $-COSR_5$.

**4.** Utilisation selon revendication 1, caractérisée en ce que, dans le composé de formule I, Y représente $-CONR_6R_7$.

**5.** Utilisation selon revendication 1, caractérisée en ce que, dans le composé de formule I, X et Y forment ensemble un cycle tétrahydrofuranne-2-one-3-yle.

**6.** Utilisation selon revendication 1, pour la protection du millet cultivé, du riz, du maïs et des céréales contre les effets nocifs des éthers diphényliques.

**7.** Utilisation selon revendication 1, pour la protection du millet cultivé, du riz, du maïs et des céréales contre les effets nocifs des esters pyridyloxyphénoxypropioniques substitués.

**8.** Utilisation selon revendication 7, pour la protection du millet cultivé, du riz, du maïs et des céréales contre les effets nocifs du 2-[4-(3,5-dichloropyridyl-2-oxy)-phénoxy]-propionate de 2-propynyle.

**9.** Utilisation selon revendication 1 pour la protection des céréales.

**10.** Composé de formule I

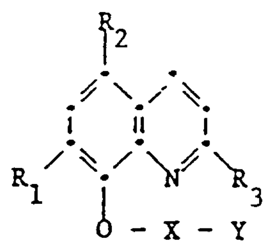

(I),

dans laquelle $R_1$ représente l'hydrogène, le chlore, l'iode ou le brome, $R_2$ représente l'hydrogène, le chlore, l'iode, le brome, un groupe nitro, méthyle ou éthyle, et $R_3$ représente l'hydrogène ou un groupe méthyle, X représente un pont méthylène éventuellement substitué par un ou deux groupes méthyle, et Y représente un groupe oxazoline-2-yle, $-COOR_4$, $-COSR_5$ ou $-CONR_6R_7$ dans lesquels

$R_4$ représente

- un cation ammonium quaternaire choisi parmi les cations triméthyl-, triéthyl- et tri-(2-hydroxyéthylène)-ammonium;
- un groupe alkyle à plus de 5 atomes de carbone mais à 18 atomes de carbone au maximum;
- un groupe alkyle en C1-C10 substitué par un ou deux groupes hydroxy;
- un groupe alkyle en C1-C4 substitué par 1 à 3 atomes de chlore ou de brome, un groupe nitro, un groupe cyano, un groupe alcoxy en C1-C4, un groupe alcoxy en C2-C8 interrompu par un ou deux atomes d'oxygène, un groupe hydoxyalcoxy en C1-C4, un groupe hydroxyalcoxy en C2-C6 interrompu par un ou deux atomes d'oxygène, un groupe (alcoxy en C1-C4)-carbonyle, un groupe di-(alkyle en C1-C4)-amino, un groupe phényle non substitué ou substitué par le chlore ou des groupes méthoxy, un groupe cyclohexyle, un radical de furanne, de tétrahydrofuranne, de tétrahydropyranne, de pyridine, de pipéridine ou de morpholine;
- un groupe alcényle en C3-C10;
- un groupe alcynyle en C3-C4 non substitué ou substitué par un groupe hydroxy;
- un groupe cyclohexyle non substitué ou substitué par un ou deux groupes méthyle;
- un groupe phényle non substitué ou portant un ou deux substituants choisis parmi le chlore, les groupes nitro, alkyle en C1-C4 ou méthoxy;
- un groupe naphtyle; ou bien
- la pyridine;

$R_5$ représente

- un groupe alkyle en C1-C12;
- un groupe alkyle en C1-C4 substitué par un groupe (alcoxy en C1-C4)-carbonyle;
- un groupe phényle non substitué ou substitué par un atome de chlore;

$R_6$ représente

- un groupe alkyle à plus de 6 atomes de carbone mais à 18 atomes de carbone au maximum;

- un groupe alkyle en C2-C8 substitué par un groupe amino;
- un groupe alkyle en C2-C6 substitué par un groupe hydroxy;
- un groupe alkyle en C2-C4 substitué par un atome de chlore ou de brome, un groupe hydroxyalkylamino en C1-C4, un groupe di-(hydroxyalkyle en C1-C3)-amino ou un groupe (alcoxy en C1-C4)-carbonyle;
- un groupe alkyle en C1-C4 substitué par un groupe cyano ou par un groupe phényle non substitué ou substitué par le chlore, ou par un radical furannyle, tétrahydrofurannyle, pipéridino ou morpholino;
- un groupe alcoxy en C1-C3;
- un groupe cyclohexyle substitué par un groupe méthyle;
- un groupe phényle portant un ou deux substituants choisis parmi les groupes nitro, cyano, méthyle, éthyle, méthoxy et trifluorométhyle, et

$R_7$ représente
- l'hydrogène;
- un groupe alkyle en C1-C6;
- un groupe alkyle en C1-C4 substitué par un groupe hydroxy ou un groupe cyano;
- un groupe alcoxyalkyle en C2-C4;
- un groupe alcényle en C3-C4;
- un groupe cyclohexyle; ou bien
- un groupe phényle; ou bien

$R_6$ et $R_7$ représentent, ensemble et avec l'atome d'azote auquel ils sont reliés, un radical pipéridino, tétrahydropyrimidino, morpholino ou imidazolyle substitué par un ou deux groupes méthyle, ou bien X et Y forment ensemble un groupe tétrahydrofuranne-2-one-3-yle.

**11.** Composé selon revendication 10, caractérisé en ce que Y représente - $COOR_4$.

**12.** Composé selon revendication 10, caractérisé en ce que Y représente $-COSR_5$.

**13.** Composé selon revendication 10, caractérisé en ce que Y représente $-CONR_6R_7$.

**14.** Composé selon revendication 10, caractérisé en ce que X et Y forment ensemble un cycle tétrahydrofuranne-2-one-3-yle non substitué.

**15.** Produit pour la protection du millet cultivé, du riz, du maïs et des céréales contre les effets nocifs des herbicides, caractérisé en ce qu'il contient en tant que composant actif un composé selon la revendication 10.

**16.** Produit selon revendication 15, caractérisé en ce qu'il contient le composant actif avec un véhicule et/ou des additifs inertes.

**17.** Produit selon revendication 15, caractérisé en ce qu'il contient en tant que composant actif un composé tel que défini dans la revendication 10, avec un herbicide nocif pour les végétaux cultivés.

**18.** Produit selon revendication 17, caractérisé en ce qu'il contient en tant qu'herbicide un éther diphénylique.

**19.** Produit selon revendication 17, caractérisé en ce qu'il contient en tant qu'herbicide, un ester pyridyloxyphénoxypropionique substitué.

**20.** Produit selon revendication 19, caractérisé en ce qu'il contient en tant qu'herbicide le 2-[4-(3,5-dichloropyridyl-2-oxy)phénoxy]-propionate de 2-propynyle.

**21.** Procédé de préparation des composés de la revendication 10, caractérisé en ce que l'on fait réagir un composé de formule II

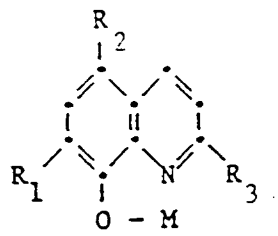

(II),

dans laquelle $R_1$, $R_2$ et $R_3$ ont les significations indiquées et M représente l'hydrogène, un atome de métal alcalin ou alcalino-terreux, avec un composé de formule III

Z - X - Y (III)

dans laquelle X et Y ont les significations indiquées et Z représente un substituant éliminable.

**22.** Procédé de préparation des composés de la revendication 10, dans lesquels Y représente $-COOR_4$, caractérisé en ce que l'on fait réagir un halogénure d'acide de formule IV

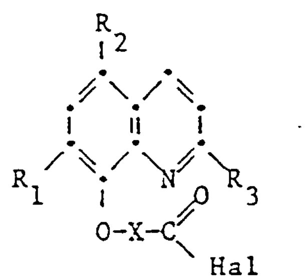

(IV),

dans laquelle $R_1$, $R_2$, $R_3$ et X ont les significations indiquées, et Hal représente un atome d'halogène, avec un composé de formule V

M' - O - $R_4$ (V)

dans laquelle $R_4$ a les significations indiquées et M' représente l'hydrogène, un atome de métal alcalin ou alcalino-terreux.

**23.** Procédé de préparation des composés de la revendication 10 dans lesquels Y représente $-COSR_5$, caractérisé en ce que l'on fait réagir un halogénure d'acide de formule VI

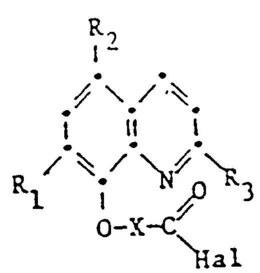

(VI),

dans laquelle $R_1$, $R_2$, $R_3$ et X ont les significations indiquées et Hal représente un atome d'halogène, avec un composé de formule VII

M'' - S - $R_5$ (VII)

dans laquelle $R_5$ a les significations indiquées et M'' représente l'hydrogène, un atome de métal alcalin

ou alcalino-terreux.

**24.** Procédé de préparation des composés de la revendication 10, dans lesquels Y représente $-CONR_6R_7$, caractérisé en ce que l'on fait réagir un halogénure d'acide de formule VIII

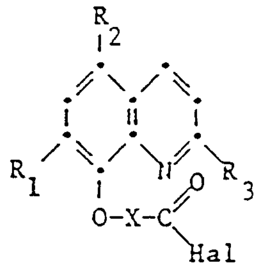

(VIII),

dans laquelle $R_1$, $R_2$, $R_3$ et X ont les significations indiquées et Hal représente un atome d'halogène, avec un composé de formule IX

$HNR_6R_7$ (IX)

dans laquelle $R_6$ et $R_7$ ont les significations indiquées.

**25.** Procédé de préparation des composés de la revendication 10 pour lesquels Y représente $-COOR_4$, caractérisé en ce que l'on fait réagir un composé de formule X

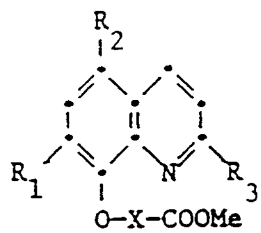

(X),

dans laquelle $R_1$, $R_2$, $R_3$ et X ont les significations indiquées et Me représente un atome de métal alcalin, de métal alcalino-terreux, de plomb ou d'argent, avec un composé de formule XI

Hal - $R_4$ (XI)

dans laquelle $R_4$ a les significations indiquées et Hal représente un atome d'halogène.

**26.** Procédé de préparation des composés de la revendication X pour lesquels Y représente $-COSR_5$, caractérisé en ce que l'on fait réagir un composé de formule XII

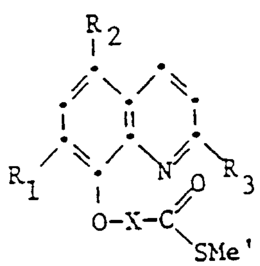

(XII),

dans laquelle $R_1$, $R_2$, $R_3$ et X ont les significations indiquées et Me' représente un atome de métal alcalin, de métal alcalino-terreux, de plomb ou d'argent, avec un composé de formule XIII

Hal - $R_5$ (XIII)

dans laquelle $R_5$ a les significations indiquées et Hal représente un atome d'halogène.

**27.** Procédé de préparation des composés de la revendication 10 pour lesquels Y représente $-COOR_4$, caractérisé en ce que l'on fait réagir un composé de formule XIV

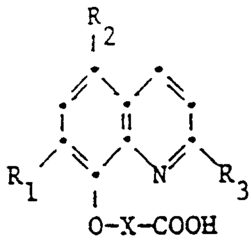

(XIV),

dans laquelle $R_1$, $R_2$, $R_3$ et X ont les significations indiquées, avec un composé de formule XV

HO - $R_4$ (XV)

dans laquelle $R_4$ a les significations indiquées.

**28.** Procédé de préparation des composés selon revendication 10 pour lesquels Y représente $-COSR_5$, caractérisé en ce que l'on fait réagir un composé de formule XVI

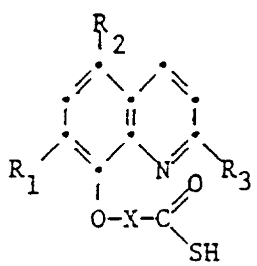

(XVI),

dans laquelle $R_1$, $R_2$, $R_3$ et X ont les significations indiquées, avec un composé de formule XVII

HO - $R_5$ (XVII)

dans laquelle $R_5$ a les significations indiquées.

**29.** Procédé de préparation des composés selon revendication 10 pour lesquels Y représente $-COOR_4$ caractérisé en ce que l'on fait réagir un composé de formule Ia

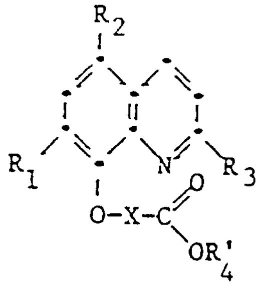

(Ia),

dans laquelle $R_1$, $R_2$, $R_3$ et X ont les significations indiquées et $R'_4$ a les significations indiquées pour

$R_4$, avec un composé de formule XVIII

HO - $R''_4$ (XVIII)

dans laquelle $R''_4$ a les significations indiquées pour $R_4$ mais ne peut être identique à $R'_4$.

**30.** Procédé de préparation des composés selon revendication 10, pour lesquels Y représente $-COSR_5$, caractérisé en ce que l'on fait réagir un composé de formule Ib

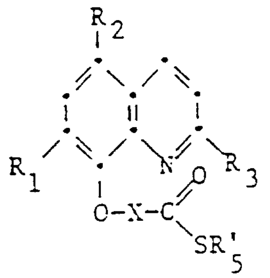

(Ib),

dans laquelle $R_1$, $R_2$, $R_3$ et X ont les significations indiquées et $R'_5$ a les significations indiquées pour $R_5$, avec un composé de formule XIX

$HSR''_5$ (XIX)

dans laquelle $R''_5$ a les significations indiquées pour $R_5$ mais ne peut être identique à $R'_5$.

**31.** Procédé de préparation des composés selon revendication 10, pour lesquels Y représente $-CONR_6R_7$, caractérisé en ce que l'on fait réagir un composé de formule XX

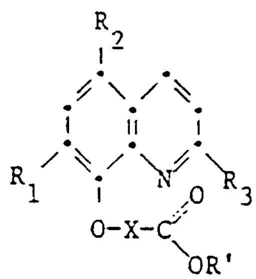

(XX),

dans laquelle $R_1$, $R_2$, $R_3$ et X ont les significations indiquées et R' représente un reste hydrocarboné aliphatique acyclique, avec un composé de formule XXI

$HNR_6R_7$ (XXI)

dans laquelle $R_6$ et $R_7$ ont les significations indiquées.

**32.** Procédé de préparation des composés selon revendication 10, pour lesquels Y représente un groupe oxazoline-2-yle, caractérisé en ce que l'on cyclise en présence d'un accepteur d'acide un composé de formule XXIV

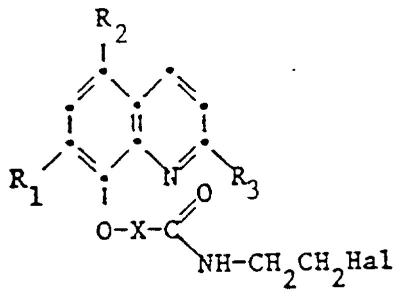

(XXIV),

dans laquelle $R_1$, $R_2$, $R_3$ et X ont les significations indiquées et Hal représente un atome d'halogène, en particulier de chlore ou de brome.

**33.** Procédé de préparation des composés selon revendication 10, pour lesquels Y représente $-CH_2CH_2-$ caractérisé en ce que l'on fait réagir un composé de formule XXII

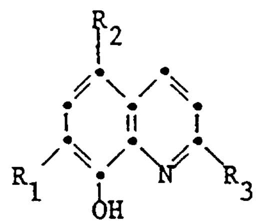

(XXII),

dans laquelle $R_1$, $R_2$ et $R_3$ ont les significations indiquées, avec un composé de formule XXIII

$CH_2 = CH - Y$ (XXIII)

dans laquelle Y a les significations indiquées.

**34.** Semences de millet cultivé, de riz, de mais et de céréales traitées par un dérivé de la quinoléine de formule I de la revendication 1.

**Revendications pour l'Etat contractant suivant : AT**

**1.** Utilisation de dérivés de la quinoléine de formule I

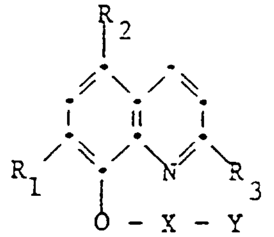

(I),

dans laquelle
$R_1$ représente l'hydrogène, le chlore, l'iode ou le brome, $R_2$ représente l'hydrogène, le chlore, l'iode, le brome, un groupe nitro, métnyle ou éthyle, et $R_3$ représente l'hydrogène ou un groupe méthyle, X représente un pont méthylène éventuellement substitué par un ou deux groupes méthyle et Y représente un groupe oxazoline-2-yle, $-COOR_4$, $-COSR_5$ ou $-CONR_6R_7$ dans lesquels
$R_4$ représente

- l'hydrogène, un cation de métal alcalin ou un cation d'ammonium quaternaire choisi parmi les cations ammonium, triméthyl-, triéthyl- et tri-(2-hydroxyéthylène)-ammonium;
- un groupe alkyle en C1-C18;

- un groupe alkyle en C1-C10 substitué par un ou deux groupes hydroxy;
- un groupe alkyle en C1-C4 substitué par un à trois atomes de chlore ou de brome, un groupe nitro, un groupe cyano, un groupe alcoxy en C1-C4, un groupe alcoxy en C2-C8 interrompu par un ou deux atomes d'oxygène, un groupe hydroxyalcoxy en C1-C4, un groupe hydroxyalcoxy en C2-C6 interrompu par un ou deux atomes d'oxygène, un groupe (alcoxy en C1-C4)-carbonyle, un groupe di-(alkyle en C1-C4)-amino, un groupe phényle non substitué ou substitué par le chlore ou des groupes méthoxy, un groupe cyclohexyle, un radical de furanne, de tétrahydrofuranne, de tétrahydropyranne, de pyridine, de pipéridine ou de morpholine;
- un groupe alcényle en C3-C10;
- un groupe alcynyle en C3-C4 non substitué ou substitué par un groupe hydroxy;
- un groupe cyclohexyle non substitué ou substitué par un ou deux groupes méthyle;
- un groupe phényle non substitué ou portant un ou deux substituants choisis parmi le chlore, les groupes nitro, alkyle en C1-C4 ou méthoxy;
- un groupe naphtyle; ou bien
- la pyridine,

$R_5$ représente
- un groupe alkyle en C1-C12;
- un groupe alkyle en C1-C4 substitué par un groupe (alcoxy en C1-C4)-carbonyle;
- un groupe phényle non substitué ou substitué par un atome de chlore;

$R_6$ représente
- l'hydrogène;
- un groupe alkyle en C1-C18;
- un groupe alkyle en C2-C8 substitué par un groupe amino;
- un groupe alkyle en C2-C6 substitué par un groupe hydroxy;
- un groupe alkyle en C2-C4 substitué par un atome de chlore ou de brome, un groupe alkylamino en C1-C4, un groupe di-(alkyle en C1-C4)-amino, un groupe hydroxyalkylamino en C1-C4, un groupe di-(hydroxyalkyle en C1-C3)-amino, un groupe alcoxy en C1-C4 ou un groupe (alcoxy en C1-C4)-carbonyle;
- un groupe alkyle en C1-C4 substitué par un groupe cyano ou par un groupe phényle non substitué ou substitué par le chlore, par un groupe furannyle, tétrahydrofurannyle, pipéridino ou morpholino;
- un groupe alcoxy en C1-C3;
- un groupe alcényle en C3-C4;
- un groupe cyclohexyle non substitué ou substitué par un groupe méthyle;
- un groupe phényle non substitué ou portant un ou deux substituants choisis parmi le chlore, les groupes nitro, cyano, méthyle, éthyle, méthoxy et trifluorométhyle; et

$R_7$ représente
- l'hydrogène;
- un groupe alkyle en C1-C6;
- un groupe alkyle en C1-C4 substitué par un groupe hydroxy ou un groupe cyano;
- un groupe alcoxyalkyle en C2-C4;
- un groupe alcényle en C3-C4;
- un groupe cyclohexyle; ou bien
- un groupe phényle, ou bien

$R_6$ et $R_7$ représentent, ensemble et avec l'atome d'azote auquel ils sont reliés, un groupe pipéridino, tétrahydropyrimidino, morpholino ou imidazolyle non substitué ou substitué par un ou deux groupes méthyle, ou bien X et Y forment ensemble un cycle tétrahydrofuranne-2-one-3-yle; pour la protection du millet cultivé, du riz, du maïs et des céréales contre les effets nocifs des herbicides sur les végétaux cultivés.

**2.** Utilisation selon revendication 1, caractérisée en ce que, dans le composé de formule I, Y représente $-COOR_4$.

**3.** Utilisation selon revendication 1, caractérisée en ce que, dans le composé de formule I, Y représente $-COSR_5$.

**4.** Utilisation selon revendication 1, caractérisée en ce que, dans le composé de formule I, Y représente $-CONR_6R_7$.

**5.** Utilisation selon revendication 1, caractérisée en ce que, dans le composé de formule I, X et Y forment ensemble un cycle tétrahydrofuranne-2-one-3-yle.

**6.** Utilisation selon revendication 1, pour la protection du millet cultivé, du riz, du mais et des céréales contre les effets nocifs des éthers diphényliques.

**7.** Utilisation selon revendication 1, pour la protection du millet cultivé, du riz, du maïs et des céréales contre les effets nocifs des esters pyridyloxyphénoxypropioniques substitués.

**8.** Utilisation selon revendication 7, pour la protection du millet cultivé, du riz, du maïs et des céréales contre les effets nocifs du 2-[4-(3,5-dichloropyridyl-2-oxy)-phénoxy]-propionate de 2-propynyle.

**9.** Utilisation selon revendication 1 pour la protection des céréales.

**10.** Produit pour protéger le millet cultivé, le riz, le maïs et les céréales contre les effets nocifs des herbicides sur les végétaux cultivés, caractérisé en ce qu'il contient, en tant que composant actif, un composé de formule I

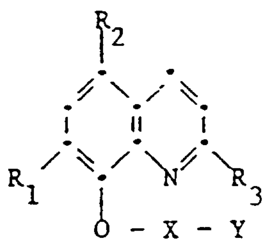

(I),

dans laquelle $R_1$ représente l'hydrogène, le chlore, l'iode ou le brome, $R_2$ représente l'hydrogène, le chlore, l'iode, le brome, un groupe nitro, méthyle ou éthyle, et $R_3$ représente l'hydrogène ou un groupe méthyle, X représente un pont méthylène éventuellement substitué par un ou deux groupes méthyle, et Y représente un groupe oxazoline-2-yle, $-COOR_4$, $-COSR_5$ ou $-CONR_6R_7$ dans lesquels

$R_4$ représente

- un cation ammonium quaternaire choisi parmi les cations triméthyl-, triéthyl- et tri-(2-hydroxyéthylène)-ammonium;
- un groupe alkyle à plus de 5 atomes de carbone mais à 18 atomes de carbone au maximum;
- un groupe alkyle en C1-C10 substitué par un ou deux groupes hydroxy;
- un groupe alkyle en C1-C4 substitué par 1 à 3 atomes de chlore ou de brome, un groupe nitro, un groupe cyano, un groupe alcoxy en C1-C4, un groupe alcoxy en C2-C8 interronpu par un ou deux atomes d'oxygène, un groupe hydroxyalcoxy en C1-C4, un groupe hydroxyalcoxy en C2-C6 interrompu par un ou deux atomes d'oxygène, un groupe (alcoxy en C1-C4)-carbonyle, un groupe di-(alkyle en C1-C4)-amino, un groupe phényle non substitué ou substitué par le chlore ou des groupes méthoxy, un groupe cyclohexyle, un radical de furanne, de tétrahydrofuranne, de tétrahydropyranne, de pyridine, de pipéridine ou de morpholine;
- un groupe alcényle en C3-C10;
- un groupe alcynyle en C3-C4 non substitué ou substitué par un groupe hydroxy;
- un groupe cyclohexyle non substitué ou substitué par un ou deux groupes méthyle;
- un groupe phényle non substitué ou portant un ou deux substituants choisis parmi le chlore, les groupes nitro, alkyle en C1-C4 ou méthoxy;
- un groupe naphtyle; ou bien
- la pyridine;

$R_5$ représente

- un groupe alkyle en C1-C12;
- un groupe alkyle en C1-C4 substitué par un groupe (alcoxy en C1-C4)-carbonyle;
- un groupe phényle non substitué ou substitué par un atome de chlore;

$R_6$ représente

- un groupe alkyle à plus de 6 atomes de carbone mais à 18 atomes de carbone au maximum;
- un groupe alkyle en C2-C8 substitué par un groupe amino;

- un groupe alkyle en C2-C6 substitué par un groupe hydroxy;
- un groupe alkyle en C2-C4 substitué par un atome de chlore ou de brome, un groupe hydroxyalkylamino en C1-C4, un groupe di-(hydroxyalkyle en C1-C3)-amino ou un groupe (alcoxy en C1-C4)-carbonyle;
- un groupe alkyle en C1-C4 substitué par un groupe cyano. ou par un groupe phényle non substitué ou substitué par le chlore, ou par un radical furannyle, tétrahydrofurannyle, pipéridino ou morpholino;
- un groupe alcoxy en C1-C3;
- un groupe cyclohexyle substitué par un groupe méthyle;
- un groupe phényle portant un ou deux substituants choisis parmi les groupes nitro, cyano, méthyle, éthyle, méthoxy et trifluorométhyle, et

$R_7$ représente
- l'hydrogène;
- un groupe alkyle en C1-C6;
- un groupe alkyle en C1-C4 substitué par un groupe hydroxy ou un groupe cyano;
- un groupe alcoxyalkyle en C2-C4;
- un groupe alcényle en C3-C4;
- un groupe cyclohexyle; ou bien
- un groupe phényle; ou bien

$R_6$ et $R_7$ représentent, ensemble et avec l'atome d'azote auquel ils sont reliés, un radical pipéridino, tétrahydropyrimidino, morpholino ou imidazolyle substitué par un ou deux groupes méthyle, ou bien X et Y forment ensemble un groupe tétrahydrofuranne-2-one-3-yle.

**11.** Produit selon revendication 10, caractérisé en ce que Y représente $-COOR_4$.

**12.** Produit selon revendication 10, caractérisé en ce que Y représente $-COSR_5$.

**13.** Produit selon revendication 10, caractérisé en ce que Y représente $-CONR_6R_7$.

**14.** Produit selon revendication 10, caractérisé en ce que X et Y forment ensemble un cycle tétrahydrofuranne-2-one-3-yle non substitué.

**15.** Produit selon revendication 10, caractérisé en ce qu'il contient le composant actif avec un véhicule et/ou des additifs inertes.

**16.** Produit selon revendication 10, caractérisé en ce qu'il contient en tant que composant actif un composé tel que défini dans la revendication 10, avec un herbicide nocif pour les végétaux cultivés.

**17.** Produit selon revendication 16, caractérisé en ce qu'il contient en tant qu'herbicide un éther diphénylique.

**18.** Produit selon revendication 16, caractérisé en ce qu'il contient en tant qu'herbicide un ester pyridyloxyphénoxypropionique substitué.

**19.** Produit selon revendication 18, caractérisé en ce qu'il contient en tant qu'herbicide le 2-[4-(3,5-dichloropyridyl-2-oxy)-phénoxy]-propionate de 2-propynyle.

**20.** Procédé de préparation des composés de la revendication 10, caractérisé en ce que l'on fait réagir un composé de formule II

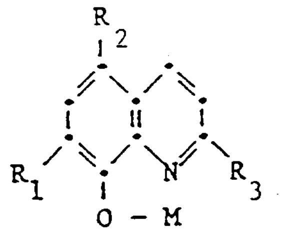

(II),

dans laquelle $R_1$, $R_2$ et $R_3$ ont les significations indiquées et M représente l'hydrogène, un atome de métal alcalin ou alcalino-terreux, avec un composé de formule III

Z - X - Y (III)

dans laquelle X et Y ont les significations indiquées et Z représente un substituant éliminable.

**21.** Procédé de préparation des composés de la revendication 10, dans lesquels Y représente $-COOR_4$, caractérisé en ce que l'on fait réagir un halogénure d'acide de formule IV

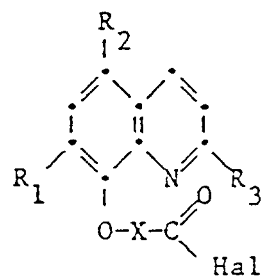

(IV),

dans laquelle $R_1$, $R_2$, $R_3$ et X ont les significations indiquées, et Hal représente un atome d'halogène, avec un composé de formule V

M' - O - $R_4$ (V)

dans laquelle $R_4$ a les significations indiquées et M' représente l'hydrogène, un atome de métal alcalin ou alcalino-terreux.

**22.** Procédé de préparation des composés de la revendication 10 dans lesquels Y représente $-COSR_5$, caractérisé en ce que l'on fait réagir un halogénure d'acide de formule VI

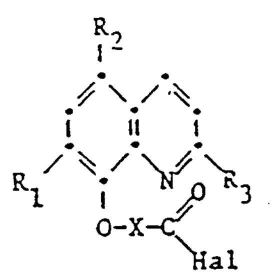

(VI),

dans laquelle $R_1$, $R_2$, $R_3$ et X ont les significations indiquées et Hal représente un atome d'halogène, avec un composé de formule VII

M'' - S - $R_5$ (VII)

dans laquelle $R_5$ a les significations indiquées et M" représente l'hydrogène, un atome de métal alcalin ou alcalino-terreux.

**23.** Procédé de préparation des composés de la revendication 10, dans lesquels Y représente $-CONR_6R_7$, caractérisé en ce que l'on fait réagir un halogénure d'acide de formule VIII

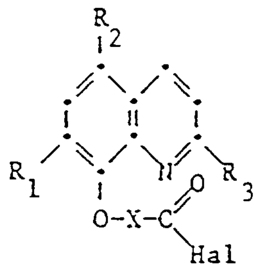

(VIII),

dans laquelle $R_1$, $R_2$, $R_3$ et X ont les significations indiquées et Hal représente un atome d'halogène, avec un composé de formule IX

$HNR_6R_7$ (IX)

dans laquelle $R_6$ et $R_7$ ont les significations indiquées.

**24.** Procédé de préparation des composés de la revendication 10 pour lesquels Y représente $-COOR_4$, caractérisé en ce que l'on fait réagir un composé de formule X

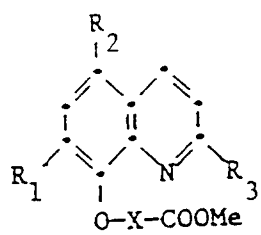

(X),

dans laquelle $R_1$, $R_2$, $R_3$ et X ont les significations indiquées et Me représente un atome de métal alcalin, de métal alcalino-terreux, de plomb ou d'argent, avec un composé de formule XI

Hal - $R_4$ (XI)

dans laquelle $R_4$ a les significations indiquées et Hal représente un atome d'halogène.

**25.** Procédé de préparation des composés de la revendication X pour lesquels Y représente $-COSR_5$, caractérisé en ce que l'on fait réagir un composé de formule XII

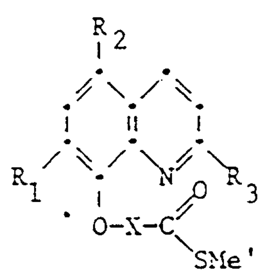

(XII),

dans laquelle $R_1$, $R_2$, $R_3$ et X ont les significations indiquées et Me' représente un atome de métal

alcalin, de métal alcalino-terreux, de plomb ou d'argent, avec un composé de formule XIII

Hal - $R_5$ (XIII)

dans laquelle $R_5$ a les significations indiquées et Hal représente un atome d'halogène.

**26.** Procédé de préparation des composés de la revendication 10 pour lesquels Y représente $-COOR_4$, caractérisé en ce que l'on fait réagir un composé de formule XIV

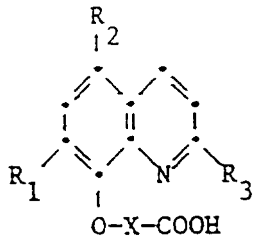

(XIV),

dans laquelle $R_1$, $R_2$, $R_3$ et X ont les significations indiquées, avec un composé de formule XV

HO - $R_4$ (XV)

dans laquelle $R_4$ a les significations indiquées.

**27.** Procédé de préparation des composés selon revendication 10 pour lesquels Y représente $-COSR_5$, caractérisé en ce que l'on fait réagir un composé de formule XVI

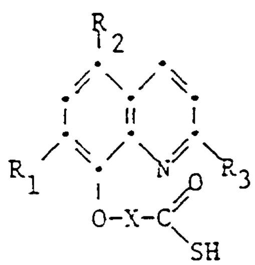

(XVI),

dans laquelle $R_1$, $R_2$, $R_3$ et X ont les significations indiquées, avec un composé de formule XVII

HO - $R_5$ (XVII)

dans laquelle $R_5$ a les significations indiquées.

**28.** Procédé de préparation des composés selon revendication 10 pour lesquels Y représente $-COOR_4$ caractérisé en ce que l'on fait réagir un composé de formule Ia

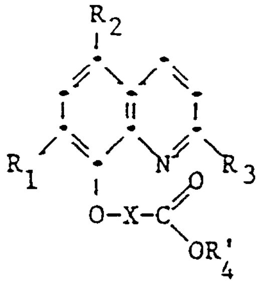

(Ia),

dans laquelle $R_1$, $R_2$, $R_3$ et X ont les significations indiquées et $R'_4$ a les significations indiquées pour $R_4$, avec un composé de formule XVIII

HO - $R''_4$ (XVIII)

dans laquelle $R''_4$ a les significations indiquées pour $R_4$ mais ne peut être identique à $R'_4$.

**29.** Procédé de préparation des composés selon revendication 10, pour lesquels Y représente $-COSR_5$, caractérisé en ce que l'on fait réagir un composé de formule Ib

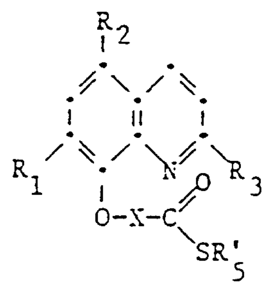

(Ib),

dans laquelle $R_1$, $R_2$, $R_3$ et X ont les significations indiquées et $R'_5$ a les significations indiquées pour $R_5$, avec un composé de formule XIX

$HSR''_5$ (XIX)

dans laquelle $R''_5$ a les significations indiquées pour $R_5$ mais ne peut être identique à $R'_5$.

**30.** Procédé de préparation des composés selon revendication 10, pour lesquels Y représente $-CONR_6R_7$, caractérisé en ce que l'on fait réagir un composé de formule XX

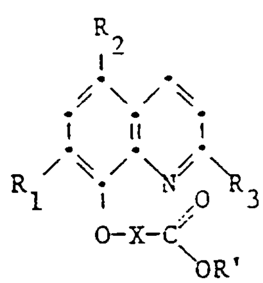

(XX),

dans laquelle $R_1$, $R_2$, $R_3$ et X ont les significations indiquées et R' représente un reste hydrocarboné aliphatique acyclique, avec un composé de formule XXI

$HNR_6R_7$ (XXI)

dans laquelle $R_6$ et $R_7$ ont les significations indiquées.

**31.** Procédé de préparation des composés selon revendication 10, pour lesquels Y représente un groupe oxazoline-2-yle, caractérisé en ce que l'on cyclise en présence d'un accepteur d'acide un composé de formule XXIV

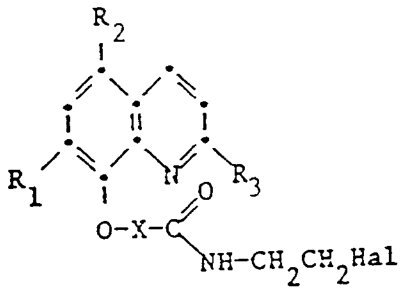

(XXIV),

dans laquelle $R_1$, $R_2$, $R_3$ et X ont les significations indiquées et Hal représente un atome d'halogène, en particulier de chlore ou de brome.

**32.** Procédé de préparation des composés selon revendication 10, pour lesquels Y représente $-CH_2CH_2-$ caractérisé en ce que l'on fait réagir un composé de formule XXII

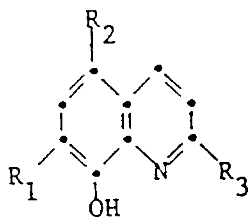

(XXII),

dans laquelle $R_1$, $R_2$ et $R_3$ ont les significations indiquées, avec un composé de formule XXIII

$CH_2 = CH - Y$ (XXIII)

dans laquelle Y a les significations indiquées.

**33.** Semences de millet cultivé, de riz, de maïs et de céréales traitées par un dérivé de la quinoléine de formule I de la revendication 1.